# EUROPEAN PATENT SPECIFICATION

(11) **EP 2 342 191 B1**
(45) Date of publication and mention of the grant of the patent: **20.03.2013**
(21) Application number: 09788067.8
(22) Date of filing: 09.09.2009
(51) Int. Cl.: C07D 401/12, C07D 403/12, C07D 403/14, C07D 401/14, A61K 31/505, C07D 405/12, A61K 31/502

(54) **AROMATIC NITROGEN-CONTAINING 6-MEMBERED RING COMPOUNDS AND THEIR USE**
AROMATISCHE, STICKSTOFFHALTIGE VERBINDUNGEN MIT 6-GLIEDRIGEM RING UND DEREN VERWENDUNG
COMPOSÉS CYCLIQUES AROMATIQUES AZOTÉS À 6 CHAÎNONS ET LEUR UTILISATION

(30) Priority: 10.09.2008 US 95765 P
(43) Date of publication of application: 13.07.2011
(73) Proprietor: Mitsubishi Tanabe Pharma Corporation, Osaka 541-8505 (JP)
(72) Inventor: MORIMOTO, Hiroshi, Osaka 541-8505 (JP); SAKAMOTO, Toshiaki, Osaka 541-8505 (JP); HIMIYAMA, Toshiyuki, Osaka 541-8505 (JP); KAWANISHI, Eiji, Osaka 541-8505 (JP); MATSUMURA, Takehiko, Osaka 541-8505 (JP)
(74) Representative: HOFFMANN EITLE
(86) International application number: PCT/JP2009/066051
(87) International publication number: WO 2010/030027

(56) References cited:
- WO-A-2007/080382
- WO-A-2008/023159
- WO-A-2008/125833
- WO-A1-2007/042806
- WO-A1-2010/027097
- US-A1- 2002 086 858
- DATABASE CA [Online] CHEMICAL ABSTRACTS SERVICE, COLUMBUS, OHIO, US; TSUJIKAWA, TERUAKI ET AL: "Heterocyclic compounds. I. Syntheses of 1,3,5-triazine derivatives and their pharmacological activities. 1" XP002558750 retrieved from STN Database accession no. 1975:564133 & YAKUGAKU ZASSHI , 95(5), 499-511 CODEN: YKKZAJ; ISSN: 0031-6903, 1975,

## Description

### TECHNICAL FIELD

The present invention relates to novel tri-substituted pyrimidine compounds having an excellent phosphodiesterase 10 (PDE10) inhibitory activity for treating or preventing a disease or condition selected from the group consisting of schizophrenia, anxiety disorder, drug addiction, a disease comprising as a symptom a deficiency in cognition, mood disorder and mood episode and useful as pharmaceuticals, and to processes for preparing such compounds and to their use.

### BACKGROUND ART

Cyclic nucleotide phosphodiesterase (hereinafter referred to as phosphodiesterase or PDE) is an enzyme that hydrolyses a phosphodiester bond in cyclic nucleotides such as cAMP (adenosine 3',5'-cyclic monophosphate) or cGMP (guanosine 3',5'-cyclic monophosphate), etc. as a substrate, to provide nucleotides such as 5'AMP (adenosine 5'-monophosphate) or 5'GMP (guanosine 5'-monophosphate), etc.

Cyclic, nucleotides such as cAMP and cGMP are involved in the regulation of many functions within a living body as second messengers of intracellular signaling. Intracellular concentrations of cAMP and cGMP, which vary in response to extracellular signals, are regulated by a balance between enzymes involved in synthesis of cAMP and cGMP (adenylate cyclase and guanylate cyclase) and PDE involved in hydrolysis of such enzymes.

For PDE of mammals, many kinds ofPDEs have been isolated and identified in mammals so far, and they have been classified into plural families in accordance with amino-acid sequence homology, biochemical properties, characterization by inhibitors and the like (Francis et al., Prog. Nucleic Acid Res., vol.65, pup.1-52, 2001).

Among such various families of PDEs of mammals, phosphodiesterase 10 (PDE10) [more specifically phosphodiesterase 10A (PDE10A)] recognizes both cAMP and cGMP as a substrate. It has been reported that PDE10 has a greater affinity for cAMP. Further, cDNAs of human, mouse and rat PDElOAs have been isolated and identified. Furthermore, the existence of PDE10 proteins has been confirmed. (Fujishige et al., J. Biol. Chem., vol.274, pp.18438-18445, 1999; Kotera et al., Biochem. Biophys. Res. Commun., vol.261, pp.551-557, 1999; Soderling et al., Proc. Natl. Acad. Sci. USA, vol.96, pp.7071-7076, 1999; and Loughley et al., Gene, vol.234, pp.109-117, 1999).

Regarding PDE10 inhibitory compounds (PDE10 inhibitors), that is, compounds having inhibitory action on the enzyme activity of PDE10, the followings have been reported:

For example, in EP1250923 (Pfizer) and WO2005/082883 (Pfizer), papaverine and various aromatic heterocyclic compounds such as quinazoline and isoquinazoline compounds are disclosed as PDE10 inhibitors.

It also has been disclosed therein that PDE10 inhibitors are useful for the treatment or prophylaxis of diseases or conditions such as:
Psychotic disorder:
   for example, schizophrenia, schizophreniform disorder,
   delusional disorder, substance-induced psychotic disorder, personality disorder of the paranoid type, personality disorder of the schizoid type, etc;
Anxiety disorder:
   for example, panic disorder, agoraphobia, specific phobia, social phobia,
   obsessive-compulsive disorder, post-traumatic stress disorder, acute stress disorder, generalized anxiety disorder, etc;
Movement disorder:
   for example, Huntington's disease, dyskinesia associated with dopamine agonist therapy, Parkinson's disease, restless leg syndrome, etc;
Drug addiction:
   for example, addiction to alcohol, amphetamine, cocaine, or opiate, etc;
Disorders comprising deficient cognition as a symptom:
   for example, dementia (including Alzheimer's disease, multi-infarct dementia, etc), delirium, amnestic disorder, post-traumatic stress disorder, mental retardation, a learning disorder, attention deficit hyperactivity disorder (ADHD), age-related cognitive decline, etc; and
Mood disorder:
   for example, major depressive disorder, dysthymic disorder, minor depressive disorder, bipolar disorder (including bipolar I disorder, bipolar II disorder), cyclothymic disorder, etc; or
Mood episode:
   for example, major depressive episode, manic or mixed mood episode, hypomanic mood episode, etc.

Further, it also has been disclosed therein that PDE10 inhibitors are useful for the treatment or prophylaxis of neurodegenerative disorders, for example, Parkinson's disease, and Hungtington's disease, etc.

In the literature of Menniti et al.[Menniti et al., Curr. Opin. Investig. Drugs., 2007, 8(1):54-59], it is disclosed that PDE10 inhibitors have potential as antipsychotic agents along with potential to improve cognitive symptoms in schizophrenia.

WO2003/000693 (Bayer) discloses imidazotriazine compounds as PDE10 inhibitors. It also discloses that PDE10 inhibitors are useful for the treatment or prophylaxis of neurodegenerative disorders, especially for Parkinson's disease.

W02003/014117 (Bayer) etc discloses various pyrroloisoquinoline compounds as PDE10 inhibitors. It also discloses that these compounds having inhibitory action on PDE10 activity show antiproliferative activity and are useful for treating cancer. Further, it discloses that those compounds are useful for treating conditions of pain and/or for lowering the temperature of the body in fever condition.

WO2005/12485 (Bayer), discloses that PDE10 inhibitors are useful for stimulating insulin release from pancreatic cells. Further, it is disclosed that PDE 10 inhibitors are useful for the treatment or prophylaxis of diabetes and diseases related thereof:
for example, type 1 or type 2 diabetes, maturity-onset diabetes of the young (MODY), latent autoimmune diabetes adult (LADA), impaired glucose tolerance (IGT), impaired fasting glucose (IGF), gestational diabetes, metabolic syndrome X, etc.

See also WO2005/120514 (Pfizer), which discloses PDE10 inhibitors that are said to be useful to decrease body weight and/or body fat in the treatment of obese patients. Further, it is disclosed therein that those PDE10 inhibitors are useful for treatment of non-insulin dependent diabetes (NIDDM), metabolic syndrome and glucose intolerance etc.

In addition, certain pyrimidine compounds are known. See for example WO2002/38551 (Roche) which discloses tri-substituted pyrimidine compounds having an activity as Neuropeptide Y receptor ligands.

WO 2008/023159 A1 and WO 2007/080382 A1 disclose morpholino pyrimidine derivatives that are used in the treatment of proliferative disease such as cancer, inflammatory diseases, obstructive airways diseases and immune diseases.

### DISCLOSURE OF THE INVENTION

The present invention provides novel compounds having an excellent PDE10 inhibitory activity, processes for preparing such compounds, use of the compounds, and pharmaceutical compositions comprising said compounds, and the like.

The present inventors have been studied and as a result, it has been found that certain aromatic nitrogen-containing 6-membered ring compounds have excellent PDE 10 inhibitory activity.

Namely, the present invention relates to an aromatic nitrogen-containing 6-membered ring compound represented by formula [I⁰]: 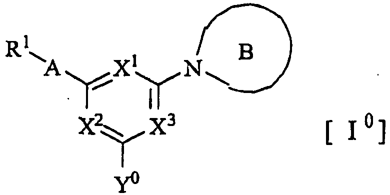 wherein:
X¹, X² and X³ each independently are N or CH, and at least two of X¹, X² and X³ are N;
A is *-CH=CH-, *-C(Alk)=CH-, *-CH₂-CH₂- or *-O-CH₂- (* is a bond with R¹);
Alk is a lower alkyl group;
Ring B is an optionally substituted nitrogen-containing aliphatic heterocyclic group;
R¹ is an optionally substituted nitrogen-containing heterocyclic group, wherein The nitrogen-containing heterocyclic group is a selected from the group consisting of quinoxalinyl, quinolyl, isoquinolyl, quinazolinyl, pyrazinyl, pyrimidinyl and the nitrogen-containing heterocyclic group may further be fused with a 5 to 6-membered aliphatic ring;
Y⁰ is a group selected from the group consisting of the following (1) to (5):
   (1) an optionally substituted phenyl or an optionally substituted aromatic monocyclic 5 to 6-membered heterocyclic group;
   (2) an optionally substituted aminocarbonyl;
   (3) an optionally substituted amino lower alkyl;
   (4) -O-R²
      wherein R² is hydrogen, an optionally substituted lower alkyl, lower cycloalkyl, aliphatic monocyclic 5 to 6-membered heterocyclic group, or 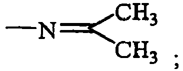
   (5) mono- or di-substituted amino;
provided that, when Y⁰ is mono- or di-substituted amino, the nitrogen-containing heterocyclic moiety of R¹ is not quinoxalinyl or quinolyl,
or a pharmaceutically acceptable salt thereof

Also, in one of the preferred embodiments of the invention, the present invention relates to an aromatic nitrogen-containing 6-membered ring compound represented by formula [I]: 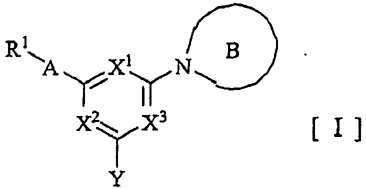 wherein:
X¹, X² and X³ each independently are N or CH, and at least two of X¹, X² and X³ are N;
A is *-CH=CH-, *-C(Alk)=CH-, *-CH₂-CH₂- or *-O-CH₂- (* is a bond with R¹);
Alk is a lower alkyl group;
Ring B is an optionally substituted nitrogen-containing aliphatic heterocyclic group;
R¹ is an optionally substituted nitrogen-containing heterocyclic group, wherein the nitrogen-containing heterocyclic group is selected from the group consisting of quinoxalinyl quinolyl, isoquinolyl, quinazolinyl, pyrazinyl, pyrimidinyl and the nitrogen-containing heterocyclic group may further be fused with a 5 to 6-membered aliphatic ring ;
Y is a group selected from the group consisting of the following (1) to (3):
   (1) an optionally substituted phenyl or an optionally substituted aromatic monocyclic 5 to 6-membered heterocyclic group;
   (2) an optionally substituted aminocarbonyl;
   (3) an optionally substituted amino lower alkyl;
or a pharmaceutically acceptable salt thereof

Also, the present invention relates to an aromatic nitrogen-containing 6-membered ring compound represented by formula [I⁰] or [I] or a pharmaceutically acceptable salt thereof for treating or preventing a disease or condition selected from the group consisting of schizophrenia, anxiety disorder, drug addiction, a disease comprising as a symptom a deficiency in cognition, mood disorder and inood episode.

Further, the present invention relates to a pharmaceutical composition comprising said compound of formula [I⁰] or [I] or a pharmaceutically acceptable salt thereof as an active ingredient, as well as to use of said compound for the manufacture of a medicament.

Furthermore, the present invention relates to said compound of formula [I⁰] or [I] or a pharmaceutically acceptable salt thereof, and to a process for preparing said compound.

The compounds of formula [I⁰] or [I] or a pharmaceutically acceptable salt thereof according to the present invention have excellent PDE10 inhibitory activity (that is, inhibitory activity on the enzyme activity of phosphodiesterase 10).

The compounds of the present invention and a pharmaceutical composition containing thereof as an active ingredient are useful for the treatment or prophylaxis of a disease or condition which is expected to be ameliorated by inhibition of PDE10 activity (that is, inhibition on the enzyme activity of phosphodiesterase 10) [for example, schizophrenia, anxiety disorder, drug addiction, a disease comprising as a symptom a deficiency in cognition, mood disorder and mood episode, etc].

### DETAILED DESCRIPTION OF THE INVENTION

Geometric isomers (E isomer or Z isomer) of formula [I⁰] or [I] may be exist due to a double bond in the molecule, for example, when a compound is of formula [I⁰] or [I] wherein A is *-CH=CH- or *-C(Alk)=CH-, etc. In the present invention, both geometric isomers and a mixture thereof are encompassed within a scope of the present invention.

In the present invention, the following terms have the following meanings, unless otherwise indicated.

Lower alkyl, lower alkylthio, lower alkyl sulfonyl, and lower alkyl amino include straight or branched group having 1 to 6 carbon atom(s) (C₁₋₆), preferably 1 to 4 carbon atom(s) (C₁₋₄).

Lower cycloalkyl includes cyclic group having 3 to 8 carbon atoms (C₃₋₈), preferably 3 to 6 carbon atoms (C₃₋₆). Also included in the lower cycloalkyl are ones having 1 to 2 lower alkyl substituent(s) on their cyclic moiety.

Lower alkoxy includes ones having 1 to 6 carbon atom(s) (C₁₋₆), preferably 1 to 4 carbon atom(s) (C₁₋₄). Included in the lower alkoxy are any of lower alkyl-O-.

Lower cycloalkoxy includes ones having 4 to 9 carbon atoms (C₄₋₉), preferably 4 to 7 carbon atom(s) (C₄₋₇). Included in the lower alkoxy are any of lower cycloalkyl-O-.

Lower alkanoyl and lower alkanoylamino include ones having 2 to 7 carbon atoms (C₂₋₇), preferably 2 to 5 carbon atoms (C₂₋₅). Included in lower alkanoyl are any of lower alkyl-C(O)-.

Lower cycloalkanoyl and lower cycloalkanoylamino includes ones having 4 to 9 carbon atoms (C₄₋₉), preferably 4 to 7 carbon atoms (C₄₋₇). Included in lower cycloalkanoyl are any of lower cycloalkyl-C(O)-.

Lower akylene includes straight or branched group having 1 to 6 carbon atom(s) (C₁₋₆), preferably 1 to 4 carbon atom(s) (C₁₋₄).

Lower alkenyl and lower alkenylene include ones having 2 to 7 carbon atoms (C₂₋₇), preferably 2 to 5 carbon atoms (C₂₋₅) and at least one double bond.

Lower cycloalkenyl includes a cyclic group having 3 to 8 carbon atoms (C₃₋₈), preferably 3 to 6 carbon atoms (C₃₋₆). Also included in lower cycloalkenyl are ones having 1 to 2 lower alkyl substituent(s) on their cyclic moiety.

Halogen means fluorine, chlorine, bromine or iodine. Halo means fluoro, chloro, bromo or iodo.

Included in the optionally substituted amino groups are unsubstituted amino groups, mono- or di-substituted acyclic amino groups, and, also included are cyclic amino groups, for example, 1-pyrrolidinyl, 1-piperidyl, 1-piperazinyl, 4-morpholinyl, etc.

When a compound of formula [I⁰] or [I] is one wherein A is *-CH=CH- or *-C(Alk)=CH-, both geometric isomers (E isomer and Z isomer) may be exist and both isomers are encompassed within a scope of the present invention. Among them, the E isomer is preferred.

In the compound [I⁰] or [I], "ask" may include methyl, ethyl, propyl, butyl and the like. Among them, methyl is more preferred.

The nitrogen-containing aliphatic heterocyclic moiety in the "optionally substituted nitrogen-containing aliphatic heterocyclic group" represented by Ring B includes saturated or unsaturated, monocyclic or bicyclic aliphatic heterocycle containing one nitrogen atom and 0 or more hetero atom(s) selected from the group consisting of nitrogen, oxygen and sulfur.

The monocyclic ones in the above nitrogen-containing aliphatic heterocycle includes saturated or unsaturated 5 to 7-membered aliphatic heterocycle containing one nitrogen and 0 to 3 hetero atom(s) selected from the group consisting of nitrogen, oxygen and sulfur.

The bicyclic ones in the above nitrogen-containing aliphatic heterocycle includes aliphatic heterocycle in which two saturated or unsaturated 5 to 7-membered rings are fused, and in which are contained one nitrogen atom and 0 to 5 hetero atom(s) selected from nitrogen, oxygen and sulfur.

Specific examples include 1-pyrrolidinyl, 1-imidazolidinyl, 1-pyrazolidinyl, 1-piperidyl, 1-piperazinyl, 4-morpholinyl, 4-thiomorpholinyl, 1-perhydroazepinyl, or a monocyclic group in which a part thereof is unsaturated.

Further specific examples include a bicyclic group having a monocyclic group and a second ring (include as the second ring, for example, cyclopentane, cyclohexane, benzene, pyrrolidine, imidazolidine, pyrazolidine, oxolane, thiolane, pyrroline, imidazoline, pyrazoline, pyrrole, imidazole, pyrazole, triazole, tetrazole, furan, oxazole, isoxazole, oxadiazole, thiophene, thiazole, isothiazole, thiadiazole, piperidine, piperazine, morpholine, thiomorpholine, pyridine, pyrimidine, pyrazine, pyridazine, pyran, perhydroazepine, perhydrothiazepine, etc.) fused thereto, and a bicyclic group thereof in which a part thereof is unsaturated.

Among these rings, preferred are 1-pyrrolidinyl, 1-imidazolidinyl, 1-pyrazolidinyl, 1-piperidyl, 1-piperazinyl, 4-morpholinyl, 4-thiomorpholinyl, or a group represented by formula [i]: 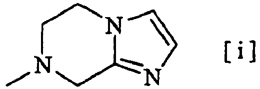 (7-5,6-dihydro-8H-imidazo[1,2-a]pyrazinyl). Particularly preferred is 1-pyrrolidinyl.

The "optionally substituted nitrogen-containing aliphatic heterocyclic group" represented by Ring B may be unsubstituted or substituted by one or more, for example, 1 to 3, substituent(s) which may be the same or different.

Examples of such substituents include:
halogen;
oxo;
hydroxy;
an optionally substituted lower alkoxy, which may have 1 to 2 substituent(s) which may be the same or different and selected from di-lower alkylamino, etc;
an optionally substituted amino, which may have 1 to 2 substituent(s) which may be the same or different and selected from the group consisting of lower alkyl, lower alkoxycarbonyl, di-lower alkylamino-lower alkylcarbonyl, di-lower alkylamino-lower alkoxycarbonyl and pyridyl-lower alkylcarbonyl, etc;
an optionally substituted lower alkyl, which may have 1 to 2 substituent(s) which may be the same or different and selected from hydroxy, etc.

The nitrogen-containing heterocyclic moiety in the "optionally substituted nitrogen-containing heterocyclic group" represented by R¹ is selected from the group consisting of quinoxalinyl, quinolyl, isoquinolyl, quinazolinyl, pyrazinyl, pyrimidinyl and the nitrogen-contraining heterocyclic group may furter be fused with a 5 to 6-membered aliphatic ring thereto.

Examples of the "5 to 6-membered aliphatic ring" fused thereto include cyclopentane, cyclohexane, pyrrolidine, imidazolidine, pyrazolidine, oxolane, thiolane, piperidine, piperazine, morpholine, thiomorpholine. Specific examples of the "group comprising the moiety fused with a 5 to 6-membered aliphatic ring" include those of the following two formulae [ii] and [iii]:

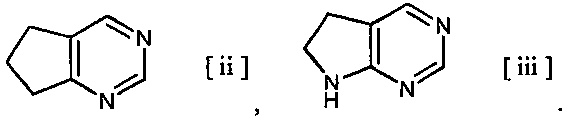

Among such nitrogen-containing heterocyclic moieties, the preferred are 2-quinoxalinyl, 2-quinolyl, 3-isoquinolyl, 2-quinazolinyl, 2-pyrazinyl, and 2-,4- or 5-pyrimidinyl. Particularly preferred are 2-quinoxalinyl, 2-quinolyl and 2-quinazolinyl.

The "optionally substituted nitrogen-containing heterocyclic group" represented by R¹ may be unsubstituted or substituted by one or more, for example, 1 to 3, substituent(s) which may be the same or different.

Examples of such substituents include:
halogen;
nitro;
hydroxy;
cyano;
an optionally substituted lower alkyl, which may have 1 to 3 substituent(s) which may be the same or different and selected from the group consisting of halogen and hydroxy; lower cycloalkyl;
lower alkoxy;
lower alkoxycarbonyl;
an optionally substituted amino group, which may have 1 to 2 substituent(s) which may be the same or different and selected from the group consisting of lower alkyl, hydroxy-lower alkyl, lower alkoxy-lower alkyl, mono- or di-lower alkylamino-lower alkyl and pyridyl-lower alkyl;
an optionally substituted phenyl, which may have 1 to 3 substituent(s) which may be the same or different and selected from lower alkoxy, etc;
an optionally substituted aromatic or aliphatic monocyclic 5 to 6-membered heterocyclic group, such as pyrrolidinyl, piperidyl, piperazinyl, morpholinyl, pyrrolyl, imidazolyl, pyridyl, and the like, each of which may have 1 to 3 substituent(s) which may be the same or different and selected from hydroxy and lower alkyl.

The heterocyclic moiety in the "optionally substituted aromatic monocyclic 5 to 6-membered heterocyclic group" represented by Y or Y⁰ includes an aromatic heterocyclic group comprising one 5 to 6-membered ring containing 1 to 4 hetero atom(s) selected from nitrogen, oxygen and sulfur.

Specific examples include pyrrolyl, imidazolyl, pyrazolyl, triazolyl, tetrazolyl, furyl, oxazolyl, isoxazolyl, oxadiazolyl, thienyl, thiazolyl, isothiazolyl, thiadiazolyl, pyridyl, pyrimidinyl, pyrazinyl and pyridazinyl.

Among them, pyridyl, such as 2- or 3-pyridyl, and furyl, such as 2-furyl are preferred.

The "optionally substituted phenyl" or "optionally substituted aromatic monocyclic 5 to 6-membered heterocyclic group" represented by Y or Y⁰ may be unsubstituted or substituted by one or more, for example, 1 to 3, substituent(s) which may be the same or different.

Examples of such substituents include:
halogen;
hydroxy;
cyano;
an optionally substituted lower alkyl, which my have 1 to 3 substituent(s) which may be the same or different and selected from halogen, etc;
an optionally substituted lower alkoxy, which may have 1 to 3 substituent(s) which may be the same or different and selected from hydroxy, lower cycloalkyl lower alkoxy, carbamoyl, mono- or di-lower alkylcarbamoyl, amino, mono- or di-lower alkylamino, an aromatic or aliphatic monocyclic 5 to 6-membered heterocyclic group (such as imidazole, pyridine, piperidiny, morpholinyl, piperazinyl, etc) optionally substituted by lower alkyl, aliphatic monocyclic 5 to 6-membered heterocyclic group-CO- (such as morpholinocarbonyl), and tetrahydropyranyl-O-;
aromatic monocyclic 5 to 6-membered heterocyclic group-O- such as pyrimidinyloxy; and
aliphatic monocyclic 5 to 6-membered heterocyclic group-O- such as piperidyloxy.

More specific examples of the "optionally substituted aminocarbonyl" represented by Y or Y⁰ include a group represented by the following formula [iv]: 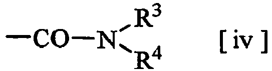 wherein:
R³ and R⁴ each independently are hydrogen, lower alkoxy, an optionally substituted amino, an optionally substituted lower alkyl, an optionally substituted lower cycloalkyl, an optionally substituted phenyl or an optionally substituted aromatic or aliphatic monocyclic 5 to 6-membered heterocyclic group, or
R³ and R⁴, together with the nitrogen atom to which they are attached, form an optionally substituted nitrogen-containing aliphatic monocyclic 5 to 6-membered heterocyclic group.

The "optionally substituted amino" represented by R³ or R⁴ may be unsubstituted or substituted by 1 to 2 substituent(s) which may be the same or different. Examples of such substituents include lower alkyl, etc.

The "optionally substituted lower alkyl" represented by R³ or R⁴ may be unsubstituted or substituted by 1 to 3 substituent(s) which may be the same or different. Examples of such substituents include hydroxy, lower alkoxy, halo-lower alkoxy, lower alkoxycarbonyl, mono- or di-lower alkylamino, pyridylamino, aliphatic monocyclic 5 to 6-membered heterocyclic group (such as oxolanyl, tetrahydropyranyl, morpholinyl) and aromatic monocyclic 5 to 6-membered heterocyclic group (such as pyridyl).

The "optionally substituted lower cycloalkyl" represented by R³ or R⁴ may be unsubstituted or substituted by 1 to 3 substituent(s) which may be the same or different. Examples of such substituents include hydroxy, lower alkoxy and aromatic monocyclic 5 to 6-membered heterocyclic group-O- (such as pyridyloxy).

The "optionally substituted phenyl" represented by R³ or R⁴ may be unsubstituted or substituted by 1 to 3 substituent(s) which may be the same or different. Examples of such substituents include lower alkoxy.

The "optionally substituted aromatic or aliphatic monocyclic 5 to 6-membered heterocyclic group" represented by R³ or R⁴ may be unsubstituted or substituted by 1 to 3 substituent(s) which may be the same or different. Examples of the heterocyclic moiety therein include pyrrolidinyl, piperidiny, morpholinyl, oxolanyl, thiolanyl, tetrahydropyranyl and pyridyl. Examples of the substituents include oxo group, lower alkyl and lower alkoxy-lower alkyl.

The "optionally substituted nitrogen-containing aliphatic monocyclic 5 to 6-membered heterocyclic group", formed by R³ and R⁴ together with the nitrogen atom to which they are attached, may be unsubstituted or substituted by 1 to 2 substituent(s) which may be the same or different. Examples of the heterocyclic moiety include piperazin-1-yl, thiomorpholin-4-yl, pyrrolidin-1-yl, piperidin-1-yl, morpholin-4-yl and 1,4-oxazepan-4-yl. Examples of the substituent include oxo group, lower alkyl, lower alkoxy and lower alkoxy-lower alkyl.

More specific examples of the "optionally substituted amino lower alkyl" represented by Y or Y⁰ include a group represented by the following formula [vi]: 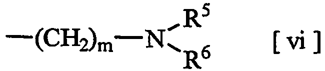 wherein:
m is 1, 2, 3 or 4; and
R⁵ and R⁶ each independently are hydrogen, an optionally substituted lower alkyl, lower cycloalkyl, or an optionally substituted aliphatic monocyclic 5 to 6-membered heterocyclic group, or
R⁵ and R⁶, together with the nitrogen atom to which they are attached, form a nitrogen-containing aliphatic monocyclic 5 to 6-membered heterocyclic group.

The "optionally substituted lower alkyl" represented by R⁵ or R⁶ may be unsubstituted or substituted by 1 to 3 substituent(s) which may be the same or different. Examples of such substituents include hydroxy and lower alkoxy.

The "optionally substituted aliphatic monocyclic 5 to 6-membered heterocyclic group" represented by R⁵ or R⁶ may be unsubstituted or substituted by 1 to 3 substituent(s) which may be the same or different. Examples of the heterocyclic moiety include tetrahydropyranyl, oxolanyl and thiolanyl. Examples of the substituent include oxo group.

Examples of the "nitrogen-containing aliphatic monocyclic 5 to 6-membered heterocyclic group" formed by R⁵ and R⁶ together with the nitrogen atom to which they are attached, include morpholin-4-yl.

The "optionally substituted lower alkyl" represented by R² in "-O-R²" represented by Y⁰, may be unsubstituted or substituted by 1 to 3 substituent(s) which may be the same or different. Examples of such substituents include lower alkoxy, lower cycloalkyl, mono- or di-lower alkylamino, aliphatic monocyclic 5 to 6-membered heterocyclic group-CO- (such as morpholinocarbonyl), an optionally substituted aliphatic monocyclic 5 to 6-membered heterocyclic group (such as pyrrolidinyl, morpholinyl, oxolanyl and tetrahydropyranyl, each of which may be unsubstituted or may have 1 to 3 substituent(s) which may be the same or different and selected from oxo group and lower alkyl).

Examples of the "aliphatic monocyclic 5 to 6-membered heterocyclic group" represented by R² include oxolanyl such as 2- or 3-oxolanyl, and, tetrahydropyranyl such as 4-tetrahydropyranyl.

The "mono- or di-substituted amino" group represented by Y⁰includes an acyclic amino group substituted by 1 or 2 substituent(s) which may be the same or different. Examples of the substituent include:
an optionally substituted lower alkyl group, which may have 1 to 3 substituent(s) which may be the same or different and selected from the group consisting of hydroxy, lower alkyl and lower alkoxy, etc;
an optionally substituted lower cycloalkyl, which may have 1 to 3 substituent(s) which may be the same or different and selected from the group consisting ofhydroxy, lower alkyl, lower alkoxy, hydroxy-lower alkyl and lower alkoxy-lower alkyl, etc; and an optionally substituted 4 to 7-membered (preferably 5 to 6-membered) aliphatic monocyclic heterocyclic group such as oxolanyl, tetrahydropyranyl and thiolanyl, each of which may have 1 to 3 substituent(s) which may be the same or different and selected from the group consisting of oxo and lower alkyl, etc.

The di-substituted amino group represented by Y° includes an optionally substituted cyclic amino. Examples of the cyclic amino include 1-pyrrolidinyl, 1-piperidyl, 1-piperazinyl and 4-morpholinyl. The cyclic amino may be substituted on its ring moiety by 1 to 3 substituent(s) which may be the same or different and selected from the group consisting of oxo, hydroxy, lower alkyl and lower alkoxy, etc.

One aspect of the present invention includes those compounds of formula [I] wherein "A" is *-CH=CH- or *-C(Alk)=CH-. In this embodiment of the invention, the E isomeric form of the double bond in "A" is preferred.

Another aspect of the present invention includes those compounds wherein the nitrogen-containing heterocyclic moiety of the optionally substituted nitrogen-containing heterocyclic group represented by R¹ is 2-quinoxalinyl, 2-quinolyl and 2-quinazolinyl .

Another aspect of the invention includes those compounds wherein Y is an optionally substituted aminocarbonyl.

Another preferable aspect of the invention includes those compounds wherein A is *-CH=CH-, *-C(Alk)=CH- or *-CH₂-CH₂-.

Another aspect of the invention includes those compounds wherein A is *-CH=CH-.

Another aspect of the invention includes those compounds wherein X¹ and X² is independently N, X³ is CH, and A is *-CH=CH-.

Another aspect of the invention includes those compounds wherein A is *-O-CH₂-.

Another aspect of the invention includes a free form of each compound as disclosed in the Examples or a pharmaceutically acceptable salt thereof (such as hydrochloride, sulfate, nitrate, phosphate, hydrobromate, acetate, fumarate, oxalate, citrate, methanesulfonate, benzenesulfonate, p-toluenesulfonate or maleate thereof).

Another aspect of the invention includes a compound selected from
2-{(E)-2-[4-(5,6-dihydroimidazo[1,2-a]pyrazin-7(8H)-yl)-6-(3,4-dimethoxyp henyl)pyrimidin-2-yl]vinyl}-N,N-dimethylquinazolin-4-amine;
2-{(E)-2-[4-(dimethylamino)quinazolin-2-yl]vinyl}-N-piperidin-1-yl-6-pyrrol idin-1-ylpyrimidine-4-carboxamide;
2-[(E)-2-(3-methylquinoxalin-2-yl)vinyl]-6-pyrrolidin-1-yl-N-(tetrahydro-2H-pyran-4-yl)pyrimidine-4-carboxamide;
N-cyclopropyl-2-[(E)-2-(3-methylquinoxalin-2-yl)vinyl]-6-pyrrolidin-1-yl-N-(tetrahydro-2H-pyran-4-yl)pyrimidine-4-carboxamide;
2-[(E)-2-(3-methylquinoxalin-2-yl)vinyl]-N-piperidin-1-yl-6-pyrrolidin-1-ylp yrimidine-4-carboxamide;
N-({2-[(E)-2-(3-methylquinoxalin-2-yl)vinyl]-6-pyrrolidin-1-ylpyrimidin-4-y 1}methyl)tetrahydro-2H-pyran-4-amine;
2- {(E)-2-[4-(cyclohexyloxy)-6-pyrrolidin-1-ylpyrimidin-2-yl]vinyl}-N,N-dim ethylquinazolin-4-amine;
acetone
O-(2- {(E)-2-[4-(dimethylamino)quinazolin-2-yl]vinyl} -6-pyrrolidin-1 -ylpyrimidin-4-yl) oxime;
(5R)-5-([(2-{(E)-2-[4-(dimethylamino)quinazolin-2-yl]vinyl)-6-pyrrolidin-1-ylpyrimidin-4-yl)oxy]methyl}pyrrolidin-2-one;
N,N,5,6-tetramethyl-2- {(E)-2-[4-pyrrolidin-1-yl-6-(tetrahydro-2H-pyran-4-yl oxy)pyrimidin-2-yl]vinyl}pyrimidin-4-amine; and
6-[(E)-2-(3-methylquinoxalin-2-yl)vinyl]-2-pyrrolidin-1-yl-N-(tetrahydro-2H-pyran-4-yl)pyrimidine-4-carboxamide;
or a pharmaceutically acceptable salt thereof (such as hydrochloride, sulfate, nitrate, phosphate, hydrobromate, acetate, fumarate, oxalate, citrate, methanesulfonate, benzenesulfonate, p-toluenesulfonate or maleate thereof).

The compounds of formula [I⁰] or [I] of the present invention may be a free form (free base or free acid) or a pharmaceutically acceptable salt thereof Examples of the pharmaceutically acceptable salts include inorganic acid salts such as the hydrochloride, sulfate, nitrate, phosphate or hydrobromate, and organic acid salts such as the acetate, fumarate, oxalate, citrate, methanesulfonate, benzenesulfonate, p-toluenesulfonate or maleate, and the like. Further, when the compounds of the present invention contain substituent(s) such as carboxyl group, the pharmaceutically acceptable salts thereof may include salts with bases such as alkali metal salts such as sodium salts and potassium salts or alkaline earth metal salts such as calcium salts.

The compounds of formula [I⁰] or [I] or a salt thereof encompass any of intramolecular salts, adducts, solvates or hydrates thereof.

The compounds of formula [I] can be prepared by a number of methods such as, but not limited to, the following:
Scheme A, Scheme A', Scheme A", Scheme B, Scheme C or Scheme D.

The compounds of formula [I⁰] can also be prepared in the same manner as set our for preparing the compound of formula [I] but using the appropriate corresponding starting materials and reactants, solvents, etc.

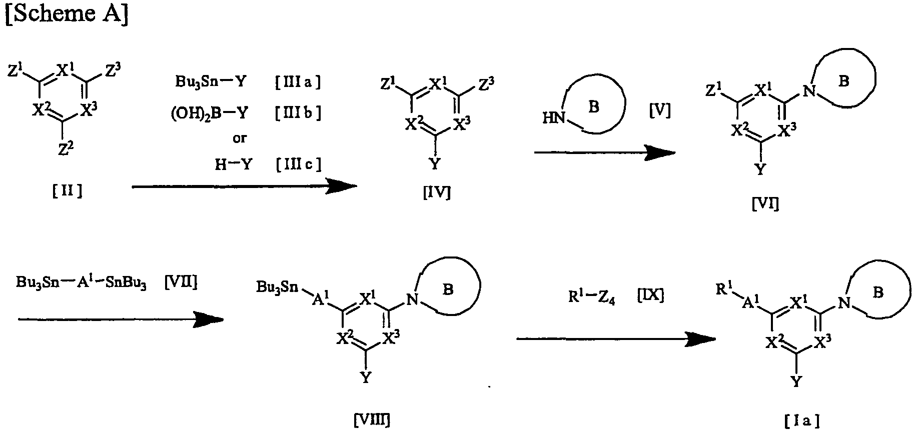

Compounds of formula [I] wherein A is *-CH=CH- or *-C(Alk)=CH-, represented by formula [Ia]:
wherein A¹ is *-CH=CH- or *-C(Alk)=CH-
(* is a bond with R¹), and the other symbols have the same meaning as defmed above,
can be prepared by the following manners.

First, a compound of formula [II]:
wherein Z¹, Z² and Z³ independently are a reactive residue, and the other symbols have the same meaning as defined above,
is reacted with a compound of formula [IIIa], [IIIb] or [IIIc]:
wherein the symbols have the same meaning as defined above, to provide a compound of formula [IV]:
wherein the symbols have the same meaning as defined above.

A compound of formula [IV] is reacted with a compound of formula [V]:
wherein the symbols have the same meaning as defined above,
   or a salt thereof to provide a compound of formula [VI]:
wherein the symbols have the same meaning as defined above.

A compound of formula [VI] is reacted with a compound of formula [VII]:
wherein the symbols have the same meaning as defined above, to provide a compound of formula [VIII]:
wherein the symbols have the same meaning as defined above.

A compound of formula [VIII] is reacted with a compound of formula [IX]:
wherein Z⁴ is a reactive residue, and the symbols have the same meaning as defined above,
to provide a compound of formula [Ia] which is optionally converted to a pharmaceutically acceptable salt thereof.

The reactive residues Z¹, Z², Z³ and Z⁴ suitably employed in the reaction include those conventionally used such as halogen, lower alkylsulfonyloxy group and arylsulfonyloxy group. Preferably the group is halogen.

Preferred salts of the compound of formula [V] are, for example, a salt formed with an inorganic acid such as hydrochloric acid and sulfuric acid, or a salt formed with inorganic base such as alkali metal base and alkali earth metal base.

The reactions in Scheme A can be carried out as described below.

The reaction of a compound of formula [II] with a compound of formula [IIIa] can be carried out in a suitable solvent in the presence of a catalyst.

Such catalyst may include palladium catalyst such as dichlorobis(triphenylphosphine)palladium, palladium acetate, palladium chloride, tetrakis(triphenylphosphine)palladium, bis(tri-t-butylphosphine)palladium, and the like.

This reaction suitably proceeds at 0 °C to 200 °C, particularly at room temperature to 110 °C.

The solvent may be any one which does not have a negative impact on the reaction. Examples include acetonitrile, methanol, ethanol, isopropyl alcohol, n-propyl alcohol, acetone, N,N-dimethylformamide, dimethylsulfoxide, tetrahydrofuran, diethyl ether, dioxane, ethyl acetate, toluene, methylene chloride, dichloroethane, chloroform, N,N-dimethylacetamide, 1,3-dimethyl-2-imidazolidinone, 1-methyl-2-pyrrolidinone, 1,2-dimethoxyethane, xylene or a combination thereof.

The reaction of a compound of formula [II] with a compound of formula [IIIb] can be carried out in a suitable solvent in the presence of a catalyst, and in the presence or absence of a base.

Such catalyst may include palladium catalyst such as dichlorobis(triphenylphosphine)palladium, palladium acetate, palladium chloride, tetrakis(triphenylphosphine)palladium, and the like.

Such base may include an organic base such as triethylamine, diisopropylethylamine, N-methylmorpholine, pyridine and dimethylaniline, an alkali metal carbonate such as sodium carbonate and potassium carbonate.

This reaction suitably proceeds at 0 °C to 200 °C, particularly at room temperature to 110 °C.

The solvent may be any one which does not have a negative impact on the reaction. Examples include the same solvents as those used in the reaction of a compound of formula [II] with a compound of formula [IIIa].

The reaction of a compound of formula [II] with a compound of formula [IIIc] can be carried out in a suitable solvent in the presence of a base or a catalyst.

Such base may be an inorganic base such as an alkali metal hydride such as sodium hydride, an alkali metal carbonate such as sodium carbonate and potassium carbonate, an alkali metal amide such as sodium amide and lithium amide, an alkali metal alkoxide such as sodium methoxide, an alkali metal such as sodium, an alkali metal hydroxide such as sodium hydroxide and potassium hydroxide, an alkyl alkali metal such as n-butyllithium, or the like. Or it may be an organic base such as triethylamine, diisopropylethylamine, morpholine, N-methylmorpholine, pyridine, dimethylaminopyridine, or the like.

Such catalyst may be palladium catalyst such as dichlorobis(triphenylphosphine)palladium, palladium acetate, palladium chloride, tetrakis(triphenylphosphine)palladium, bis(tri-t-butylphosphine)palladium, or the like; or copper iodide.

Further, for facilitating the reaction one may add phosphorus compounds such as triphenylphosphine, 2,2'-bis(diphenylphosphino)-1,1"-binaphthyl, or the like.

This reaction suitably proceeds at 0 °C to 200 °C, particularly at room temperature to 110 °C.

The solvent may be any one which does not have a negative impact on the reaction. Examples include acetonitrile, methanol, ethanol, isopropyl alcohol, n-propyl alcohol, acetone, N,N-dimethylformamide, dimethylsulfoxide, tetrahydrofuran, diethyl ether, dioxane, ethyl acetate, toluene, methylene chloride, dichloroethane, chloroform, N,N-dimethylacetamide, 1,3-dimethyl-2-imidazolidinone, 1-methyl-2-pyrrolidinone, 1,2-dimethoxyethane, xylene, N-methylpyrrolidone or a combination thereof

The reaction of a compound of formula [IV] with a compound of formula [V] or a salt thereof can be carried out in a suitable solvent in the presence or absence of a base. Such base may be an organic base such as triethylamine, diisopropylethylamine, N-methylmorpholine, pyridine, dimethylaniline, or the like. Or it may be an inorganic base such as an alkali metal hydride such as sodium hydride, an alkali metal carbonate such as sodium carbonate and potassium carbonate, an alkali metal amide such as sodium amide and lithium amide, an alkali metal such as sodium, an alkali metal hydroxide such as sodium hydroxide and potassium hydroxide, or the like.

The present reaction suitably proceeds at -78°C to 200°C, particularly at 0°C to 100°C.

The solvent may be any one which does not have a negative impact on the reaction. Examples include the same solvents as those used in the reaction of a compound of formula[II] with a compound of formula [IIIa].

The reaction of a compound of formula [VI] with a compound of formula [VII] can be carried out in a suitable solvent in the presence of a catalyst.

Such catalyst may include palladium catalyst such as dichlorobis(triphenylphosphine)palladium, palladium acetate, palladium chloride, tetrakis(triphenylphosphine)palladium, and the like.

Further, for facilitating the reaction, one may add phosphorus compounds such as triphenylphosphine, or copper iodide, or the like.

The present reaction suitably proceeds at 0°C to 200°C, particularly at 50°C to 110°C.

The solvent may be any one which does not have a negative impact on the reaction. Examples include the same solvents as those used in the reaction of a compound of formula [II] with a compound of formula [IIIa].

The reaction of a compound of formula [VIII] with a compound of formula [IX] can be carried out in the same manner as described above for reacting a compound of formula [VI] with a compound of formula [VII].

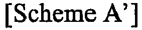

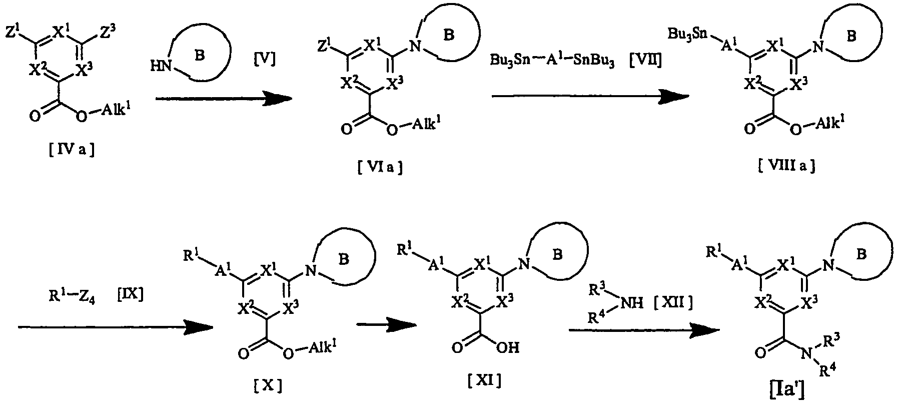

Compounds of formula [Ia] wherein Y is an optionally substituted aminocarbonyl of formula: 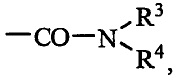 that is, compounds represented by formula [Ia']:
wherein the symbols have the same meaning as defined above, can be prepared as follows.

Instead of a compound of formula [IV] in the above Scheme A, a compound of formula [IVa]:
wherein Alk¹ is lower alkyl group, and the other symbols have the same meaning as defined above,
is reacted with a compound of formula [V] or a salt thereof to provide a compound of formula [VIa].

A compound of formula [VIa] is reacted with a compound of formula [VII] to provide a compound of formula [VIIIa].

A compound of formula [VIIIa] is reacted with a compound of formula [IX] to provide a compound of formula [X]:
wherein the symbols have the same meaning as defined above.

The reactions in the above each step can be carried out in the same manner as the reactions in the each step in Scheme A.

Then, a compound of formula [X]:
wherein the symbols have the same meaning as defined above, is hydrolyzed to provide a compound of formula [XI]:
wherein the symbols have the same meaning as defined above.

A compound of formula [XI] is reacted with a compound of formula [XII]:
wherein the symbols have the same meaning as defined above,
or a salt thereof to provide a compound of formula [Ia'] which is optionally converted to a pharmaceutically acceptable salt thereof.

A compound of formula [X] can be hydrolyzed in the presence of a base in a solvent. Such base may include an alkali metal hydroxide such as sodium hydroxide, potassium hydroxide and lithium hydroxide, an alkali metal carbonate such as sodium carbonate and potassium carbonate, and the like.

This reaction suitably proceeds at 0 to 80°C, particularly at 5 to 60°C.

The solvent may include water, as well as mixtures of water with methanol, ethanol, tetrahydrofuran, dioxane, N,N-dimethylformamide, dimethylsulfoxide, and the like.

The reaction of a compound of formula [XI] with a compound of formula [XII] can be carried out in the presence of a condensing agent in a suitable solvent.

Such condensing agent may include O-benzotriazol-1-yl-N,N,N',N'-tetramethyluronium hexafluorophosphate; DCC (dicyclohexylcarbodiimide); EDC (1-ethyl-3-(3-dimethylaminopropyl)carbodiimide); chloroformates such as ethyl chloroformate and isobutyl chloroformate; carbonyldiimidazole; and the like.

Further, for facilitating the reaction bases such as sodium carbonate, sodium hydrogen carbonate, triethyamine, pyridine, 4-dimethylaminopyridine and diisopropylethylamine, and, additives such as 1-hydroxybenzotriazole and 1-hydroxysuccinimide may be added to the above condensing agent.

This reaction suitably proceeds at 0 to 120°C, particularly at room temperature to 80°C.

The solvent may be any one which does not have a negative impact on the reaction. Examples include acetonitrile, acetone, dimethylformamide, tetrahydrofuran, ether, dioxane, ethyl acetate, toluene, methylene chloride, dichloroethane, chloroform, or a combination thereof.

Preferred salts of the compound of formula [XII] are, for example, a salt formed with an inorganic acid such as hydrochloric acid and sulfuric acid, or a salt formed with inorganic base such as alkali metal base and alkali earth metal base.

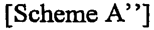

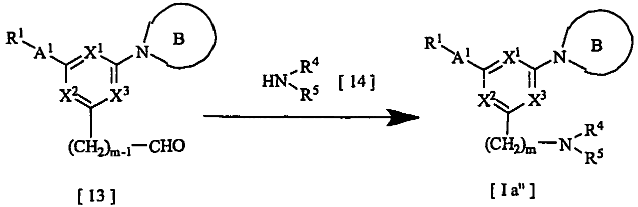

Compounds of formula [Ia] wherein Y is an optionally substituted amino lower alkyl represented by formula: 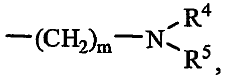 that is, compounds represented by formula [Ia"]:
wherein the symbols have the same meaning as defined above, can be prepared as follows.

A compound of formula [13]:
wherein the symbols have the same meaning as defined above, is reacted with a compound of formula [14]:
wherein the symbols have the same meaning as defined above,
or a salt thereof to provide a compound of formula [Ia"] which is optionally converted to a pharmaceutically acceptable salt thereof.

The reaction of a compound of formula [13] with a compound of formula [14] or a salt thereof can be carried out in a suitable solvent in the presence of a reducing agent.

Such reducing agent may include triacetoxysodium borohydride, sodium borohydride, cyanosodium borohydride, and the like.

The reaction suitably proceeds at 0 to 60°C, particularly at 20 to 40°C.

The solvent may be any one which does not have a negative impact on the reaction. Examples include dichloromethane, acetonitrile, tetrahydrofuran, diethyl ether, dioxane, ethyl acetate, toluene, methylene chloride, dichloroethane, chloroform, 1,2-dimethoxyethane, xylene or a combination thereof.

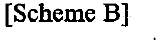

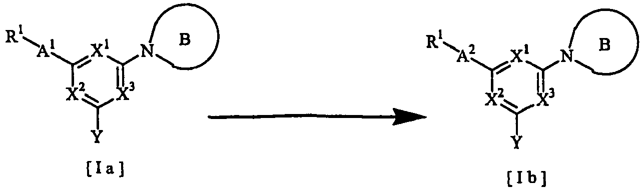

Compounds of formula [I] wherein A is *-CH₂-CH₂-, represented by formula [Ib]:
wherein A is *-CH₂-CH₂- (* is a bond with R¹), and the other symbols have the same meaning as defined above,
can be prepared as follows.

A compound of formula [Ia] wherein A¹ is -CH=CH- can be reduced (hydrogenated) to provide a compound of formula [Ib] which is optionally converted to a pharmaceutically acceptable salt thereof.

The reduction (hydrogenation) reaction in Scheme B can be carried out by catalytic reduction process in a suitable solvent in the presence of a catalyst.

Such catalyst may include platinum oxide, Raney nickel, palladium carbon and the like.

This reaction suitably proceeds at 0 °C to 100 °C, particularly at room temperature to 50 °C.

The solvent may be any one which does not have a negative impact on the reaction. Examples include the same solvents as those used in the reaction of a compound of formula [II] with a compound of formula [IIIa].

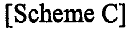

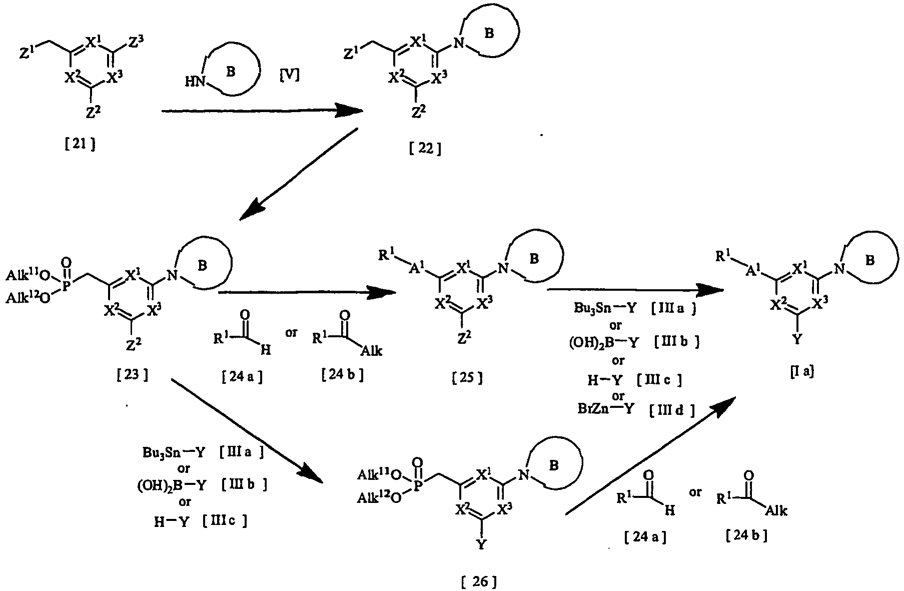

Compounds of formula [Ia] can also be prepared as follows.

A compound of formula [21]:
wherein the symbols have the same meaning as defined above, is reacted with a compound of formula [V]:
wherein the symbols have the same meaning as defined above, or a salt thereof to provide a compound of formula [22]:
wherein the symbols have the same meaning as defmed above.

A compound of formula [22] is reacted with phosphite esters such as diethyl phosphite, dimethyl phosphite, and the like to provide a compound of formula [23]:
wherein Alk¹¹ and Alk¹² each independently are alkyl group, and the other symbols have the same meaning as defined above.

A compound of formula [23] is reacted with a compound of formula [24a] or [24b]:
wherein the symbols have the same meaning as defined above, to provide a compound of formula [25]:
wherein the symbols have the same meaning as defined above.

A compound of formula [25] is reacted with a compound of formula [IIIa], [IIIb], [IIIc] or [IIId]:
wherein the symbols have the same meaning as defined above,
to provide a compound of formula [Ia] which is optionally converted to a pharmaceutically acceptable salt thereof

Alternatively, a compound of formula [23] is reacted with a compound of formula [IIIa], [IIIb] or [IIIc] to provide a compound of formula [26]:
wherein the symbols have the same meaning as defined above.

Then, a compound of formula [26] is reacted with a compound of formula [24a] or [24b] to provide a compound of formula [Ia] which is optionally converted to a pharmaceutically acceptable salt thereof.

The reactions in Scheme C can be carried out as described below.

The reaction of a compound of formula [21] with a compound of formula [V] or a salt thereof can be carried out in the same manner as described above in Scheme A for reacting a compound of formula [IV] with a compound of formula [V] or a salt thereof.

The reaction of a compound of formula [22] with phosphite esters can be carried out in a suitable solvent in the presence or absence of a base.

Such base may include an inorganic base such as an alkali metal hydride such as sodium hydride, an alkali metal carbonate such as sodium carbonate and potassium carbonate, an alkali metal amide such as sodium amide and lithium amide, an alkali metal alkoxide such as sodium methoxide, an alkali metal such as sodium, an alkali metal hydroxide such as sodium hydroxide and potassium hydroxide, and the like, or an organic base such as triethylamine, diisopropylethylamine, morpholine, N-methylmorpholine, pyridine, piperidine, dimethylaniline, dimethylaminopyridine and the like.

The present reaction suitably proceeds at -20 °C to 50 °C, particularly at 0 °C to room temperature.

The solvent may be any one which does not have a negative impact on the reaction. Examples include acetonitrile, methanol, ethanol, isopropyl alcohol, n-propyl alcohol, N,N-dimethylformamide, dimethylsulfoxide, tetrahydrofuran, diethyl ether, dioxane, ethyl acetate, toluene, methylene chloride, dichloroethane, chloroform, N,N-dimethylacetamide, 1,3-dimethyl-2-imidazolidine, 1-methyl-2-pyrrolidinone, 1,2-dimethoxyethane, xylene, or a combination thereof.

The reaction of a compound of formula [23] with a compound of formula [24a] or [24b] can be carried out in a suitable solvent in the presence or absence of a base.

Such base may include the same bases as those used in the reaction of a compound of formula [22] with phosphite esters.

The present reaction suitably proceeds at -20 °C to 50 °C, particularly at 0 °C to room temperature.

The solvent may be any one which does not have a negative impact on the reaction. Examples include the same solvents as those used in the reaction of a compound of formula [22] with phosphite esters.

The reaction of a compound of formula [25] with a compound of formula [IIIa], [IIIb], [IIIc] or [IIId] can be carried out in the same manner as described above in Scheme A for reacting a compound of formula [II] with a compound of formula [IIIa], [IIIb] or [IIIc].

The reaction of a compound of formula [23] with a compound of formula [IIIa], [IIIb], [IIIc] or [IIId] can be carried out in the same manner as described above in Scheme A for reacting a compound of formula [II] with a compound of formula [IIIa], [IIIb] or [IIIc].

The reaction of a compound of formula [26] with a compound of formula [24a] or [24b] can be carried out in the same manner as described above for reacting a compound of formula [23] with a compound of formula [24a] or [24b].

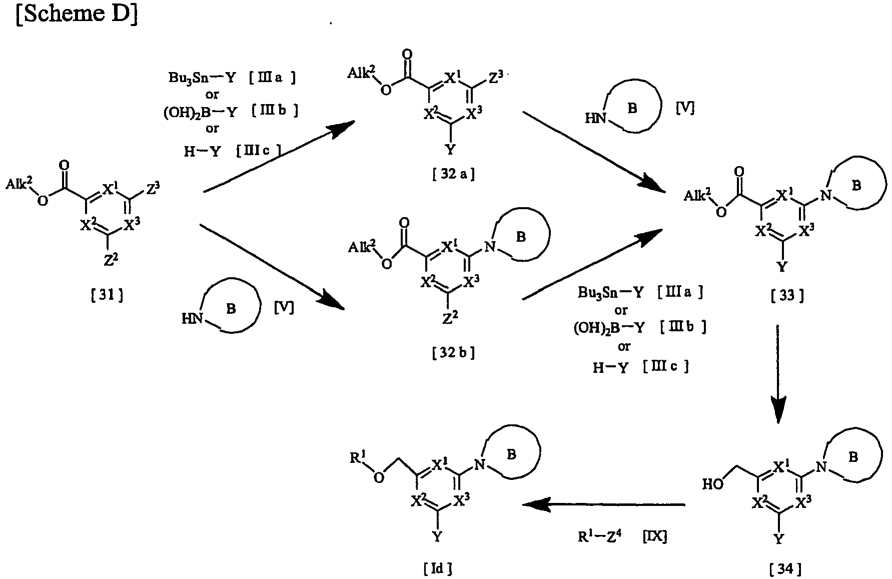

Compounds of formula [I] wherein A is *-O-CH₂-, represented by formula [Id]:
wherein the symbols have the same meaning as defined above, can be prepared as follows.

A compound represented by formula [31]:
wherein Alk² is a lower alkyl group, and the other symbols have the same meaning as defined above,
   is reacted with a compound of formula [IIIa], [IIIb] or [IIIc] to provide a compound of formula [32a]:
wherein the symbols have the same meaning as defmed above.

A compound of formula [32a] is reacted with a compound of formula [V] or a salt thereof to provide a compound of formula [33].

Alternatively, a compound of formula [31] is reacted with a compound of formula [V] or a salt thereof to provide a compound of formula [32b]:
wherein the symbols have the same meaning as defined above.

A compound of formula [32b] is reacted with a compound of formula [IIIa], [IIIb] or [IIIc] to provide a compound of formula [33].

Then, a compound of formula [33] is reduced to provide a compound of formula [34]:
wherein the symbols have the same meaning as defined above.

A compound of formula [34] is reacted with a compound of formula [IX] to provide a compound of formula [Id] which is optionally converted to a pharmaceutically acceptable salt thereof.

The reactions in Scheme D can be carried out as described below.

The reaction of a compound of formula [31] with a compound of formula [IIIa], [IIIb] or [IIIc] can be carried out in the same manner as described above in Scheme A for reacting a compound of formula [II] with a compound of formula [IIIa], [IIIb] or [IIIc].

The reaction of a compound of formula [32a] with a compound of formula [V] or a salt thereof can be carried out in the same manner as described above in Scheme A for reacting a compound of formula [IV] with a compound of formula [V] or a salt thereof.

The reaction of a compound of formula [31] with a compound of formula [V] or a salt thereof can also be carried out in the same manner as described above in Scheme A for reacting a compound of formula [IV] with a compound of formula [V] or a salt thereof.

The reaction of a compound of formula [32b] with a compound of formula [IIIa], [IIIb] or [IIIc] can be carried out in the same manner as described above in Scheme A for reacting a compound of formula [II] with a compound of formula [IIIa], [IIIb] or [IIIc].

A compound of formula [33] can be reduced using a reducing agent such as sodium borohydride, lithium borohydride, lithium aluminium hydride or the like, in a suitable solvent.

This reaction suitably proceeds at -20 °C to 60 °C, particularly at 0 °C to room temperature.

The solvent may be any one which does not have a negative impact on the reaction. Examples include diethyl ether, tetrahydrofuran, dioxane, and the like.

The reaction of a compound of formula [34] with a compound of formula [IX] can be carried out in a suitable solvent in the presence of a base or a catalyst.

Such base may include an inorganic base such as an alkali metal hydride such as sodium hydride, an alkali metal carbonate such as sodium carbonate and potassium carbonate, an alkali metal amide such as sodium amide and lithium amide, an alkali metal alkoxide such as sodium methoxide, an alkali metal such as sodium, an alkali metal hydroxide such as sodium hydroxide and potassium hydroxide, an alkyl alkali metal such as n-butyllithium, and the like. Or, one can use an organic base such as triethylamine, diisopropylethylamine, morpholine, N-methylmorpholine, pyridine, dimethylaminopyridine, and the like.

Such catalyst may include palladium catalyst such as dichlorobis(triphenylphosphine)palladium, palladium acetate, palladium chloride, tetrakis(triphenylphosphine)palladium, bis(tri-t-butylphosphine)palladium, and the like; or copper iodide.

Further, for facilitating the reaction, one may add phosphorus compounds such as triphenylphosphine and 2,2'-bis(diphenylphosphino)-1,1'-binaphthyl, or the like.

The present reaction suitably proceeds at 0 °C to 200 °C, particularly at room temperature to 110 °C.

The solvent may be any one which does not have a negative impact on the reaction. Examples include acetonitrile, methanol, ethanol, isopropyl alcohol, n-propyl alcohol, acetone, N,N-dimethylformamide, dimethylsulfoxide, tetrahydrofuran, diethyl ether, dioxane, ethyl acetate, toluene, methylene chloride, dichloroethane, chloroform, N,N-dimethylacetamide, 1,3-dimethyl-2-imidazolidine, 1-methyl-2-pyrrolidinone, 1,2-dimethoxyethane, xylene, N-methylpyrrolidone or a combination thereof.

Raw material compounds in the above preparation schemes (Scheme A, SchemeA', Scheme A", Scheme B, Scheme C and Scheme D) can be prepared by procedures known in the art and/or recited in Reference Examples described hereinafter.

Also, compounds of formula [I] or [I⁰] prepared by the above preparation schemes (Scheme A, Scheme A', Scheme A", Scheme B, Scheme C and Scheme D) can be allowed to structural conversion into the other compounds of formula [I] or [I⁰] by the procedures recited in Examples described hereinafter and/or known in the art, or a combination thereof.

The compounds of the present invention or raw material compounds thereof can be isolated and purified as the free form (free base or free acid) or as the salt thereof. The salt can be prepared by salt formation treatments usually employed. For instance, the salt formation treatment can be carried out by adding an acid or a base or the solution thereof to the solution or suspension of the compound of the present invention. Preferable acid is a pharmaceutically acceptable salt, which includes hydrochloric acid, sulfuric acid, nitric acid, phosphoric acid, hydrobromic acid, acetic acid, fumaric acid, oxalic acid, citric acid, methanesulfonic acid, benzenesulfonic acid, p-toluenesulfonic acid and maleic acid. Preferable base is a pharmaceutically acceptable salt, which includes alkali metal salts such as sodium salts and potassium salts; and alkaline earth metal salts such as calcium salts. A solvent of the solution or suspension of the compound of the present invention may be any solvent which does not have a negative impact on the salt formation treatment. Examples include water; alcohol such as methanol, ethanol, and propanol; ester such as ethyl acetate; ether such as diethyl ether, dioxane, and tetrahydrofuran; dichrormethane; and chloroform, or a combination thereof.

The isolation and purification can be carried out by usual chemical procedures such as extraction, concentration, crystallization, filtration, recrystallization and various chromatography.

The compounds of formula [I⁰] or [I] or a pharmaceutically acceptable salt thereof according to the present invention possess excellent PDE 10 inhibitory activity, that is, inhibitory activity on the enzyme activity of phosphodiesterase 10 (PDE10, more specifically PDE10A), in mammals. The compounds of formula [I⁰] or [I] or a pharmaceutically acceptable salt thereof according to the present invention are also highly selective for PDE10.

Also, the compounds of formula [I⁰] or [I] or a pharmaceutically acceptable salt thereof in the present invention exhibit various pharmacological efficacies through their PDE10 inhibitory activity. Accordingly, a pharmaceutical composition comprising the compounds of formula [I⁰] or [I] or a pharmaceutically acceptable salt thereof as an active ingredient can be used to inhibit PDE 10 activity. Further, said pharmaceutical composition can be used for the treatment or prophylaxis of diseases or conditions which are expected to be ameliorated by inhibition of PDE10 activity.

As a disease or condition which is expected to be ameliorated by inhibition of PDE10 activity, there may be mentioned, for example:
Psychotic disorder such as schizophrenia:
   for example, schizophrenia, schizophreniform disorder, delusional disorder,
   substance-induced psychotic disorder, personality disorder of the paranoid type or
   schizoid type, etc ;
Anxiety disorder:
   for example, panic disorder, agoraphobia, specific phobia, social phobia,
   obsessive-compulsive disorder, post-traumatic stress disorder, acute stress disorder,
   generalized anxiety disorder, etc ;
Drug addiction:
   for example, addiction to alcohol, amphetamine, cocaine, or opiate, etc ;
Disorders comprising deficient cognition as a symptom:
   for example, dementia (including Alzheimer's disease, multi-infarct dementia, etc), delirium, amnestic disorder, post-traumatic stress disorder, mental retardation, a learning disorder, attention deficit hyperactivity disorder (ADHD), age-related cognitive decline, etc ; and
Mood disorder:
   for example major depressive disorder, dysthymic disorder, minor depressive disorder, bipolar disorder (including bipolar I disorder, bipolar II disorder),
   cyclothymic disorder, etc ; or
Mood episode:
   for example, major depressive episode, manic or mixed mood episode, hypomanic mood episode, etc.

Of these diseases and conditions, one may wish to focus on treating the following diseases by using the compounds of the invention:
Schizophrenia:
Anxiety disorder:
   for example, panic disorder, social phobia, obsessive-compulsive disorder,
   post-traumatic stress disorder and generalized anxiety disorder;
Drug addiction:
Disorders comprising deficient cognition as a symptom:
   for example, dementia (including Alzheimer's disease, etc.), learning disorder,
   attention deficit hyperactivity disorder (ADHD) and age-related cognitive decline;
   and
Mood disorder:
   for example, major depressive disorder, dysthymic disorder, minor depressive disorder and bipolar disorder.

Of these diseases and conditions, one may wish to focus particularly on treating the following diseases by using the compounds of the invention:
Schizophrenia:
Anxiety disorder:
   for example, panic disorder, social phobia, obsessive-compulsive disorder,
   post-traumatic stress disorder and generalized anxiety disorder; and
Mood disorder:
   for example, major depressive disorder, dysthymic disorder, minor depressive disorder and bipolar disorder.

One may wish to focus more particularly on treating schizophrenia by using the compounds of the invention.

In addition, the compounds of the invention may be used to treat a disease or condition which is expected to be ameliorated by inhibition of PDE10 activity, including for example;
movement disorder or neurodegenerative disorder
including dyskinesia associated with dopamine agonist therapy;
Huntington's disease;
Parkinson's disease; and
restless leg syndrome.

In addition, the compounds of the invention may be used to treat a disease or condition which is expected to be ameliorated by inhibition of PDE10 activity, including for example, cancer.

In addition, the compounds of the invention may be used to treat a disease or condition which is expected to be ameliorated by inhibition of PDE10 activity, including for example;
type 1 or type 2 diabetes (or non-insulin dependent diabetes (NIDDM));
impaired glucose tolerance(IGT);
impaired fasting glucose(IGF);
metabolic syndrome; and
metabolism related disorders including excess of body weight or excess of body fat in obese patient.

Also within the scope of this invention is a a compound of formula [I⁰] or [I] or a pharmaceutically acceptable salt thereof for treating or preventing a disease or condition selected from the group consisting of schizophrenia, anxiety disorder, drug addiction, a disease comprising as a symptom a deficiency in cognition, mood disorder and mood episode.

Also, use of a compound of formula [I⁰] or [I] or a pharmaceutically acceptable salt thereof for the manufacture of a medicament are also encompassed within a scope of the present invention.

Inhibitory action on PDE10 and pharmacological effects of the compounds of the present invention can be confirmed by known methods and equivalent methods thereto.

For example, measurements of PDE10 inhibitory activities can be carried out by the method described below in Experimental Example 1 or by methods disclosed in literature. See for example, Fujishige et al., Eur. J. Biochem., vol.266, pp.1118-1127, 1999, and Mukai et al., Br.J.Pharmacol., vol.111, pp.389-390, 1994.

Further, selectivity of the compounds described herein for PDE10 may be evaluated by using the methods disclosed in the literature. See for example, Kotera et al., Biochem. Pharmacol., vol.60, pp.1333-1341, 2000; Sasaki et al., Biochem. Biophys. Res. Commun., vol.271, pp.575-583, 2000; Yuasa et al., Journal of Biological Chemistry, vol.275, pp.31469-31479, 2000; Gamanuma et al., Cellular Signaling, vol. 15, pp.565-574, 2003.

Pharmacological effects on the symptoms of schizophrenia can be detected by the following in vivo test systems using mouse or rat.
- MK-801-induced locomotor activity:
   [O'Neil and Shaw, Psychopharmacology, 1999, 145:237-250].
- Apomorphine-induced locomotor activity:
   [Geyer et al., Pharmacol.Biochem.Behav., 1987, 28:393-399; Ellenbroek, Pharmacol.Ther., 1993, 57:1-78].
- Conditioned avoidance response:
   [Moor et al., J. Pharmacol. Exp. Ther., 1992, 262:545-551].

Pharmacological effects to improve the deficient cognition in schizophrenia etc can be detected by the following in vivo test systems using mouse or rat.
- MK-801-induced Isolation rearing Prepulse inhibition (PPI) deficit:
   [Mansbach and Geyer, Neuropsychopharmacology, 1989, 2:299-308; Bakshi et al., J.Pharmacol.Exp.Ther., 1994, 271:787-794; Bubenikova et al., Pharmacol.Biochem.Behav., 2005, 80:591-596].
- Isolation rearing-induced Prepulse inhibition (PPI) deficit:
   [Cilia et al., Psychopharmacology, 2001, 156:327-337].
- MK-801-induced deficit in Novel object recognition task (NOR):
   [Karasawa et al., Behav.Brain.Res., 2008, 186:78-83].

The compounds of formula [I⁰] or [I] or a pharmaceutically acceptable salt thereof can be formulated into a conventional pharmaceutical preparation such as a tablet, granule, capsule, powder, solution, suspension, emulsion, inhalent, injectibles and drops, etc, by mixing the compound(s) with an inert pharmaceutically acceptable carrier suitable for each administration route.

Examples of such carriers include any conventional pharmaceutically acceptable materials, such as binders (gum Alabicum, gelatin, sorbitol, polyvinylpyrrolidone, etc), excipients (lactose, sucrose, corn starch, sorbitol, etc) lubricants (magnesium stearate, talc, polyethyleneglycol, etc), disintegrators (potato starch, etc) and the like.

In case of injectibles and drops, the compounds of the present invention can be mixed with distilled water for injection, physiological saline, aqueous glucose solution and the like.

The administration route of the compounds of formula [I⁰] or [I] or a pharmaceutically acceptable salt thereof is not limited to particular route. They can be administered orally or parenterally (for example, through intravenous, intramuscular, subcutaneous, transdermal, transnasal, transmucosal or enteral route).

Further, in case of treating a central nervous system (CNS) disease, the drug can be directly or indirectly introduced into the brain, by bypassing the blood-brain barrier (BBB). Examples of those methods include intracerebroventricular (i.c.v.) administration, and an administration method accompanying intravenous injection of hypertonic solution which enables temporary opening of the BBB (osmotic opening).

When a compound of formula [I⁰] or [I] or a pharmaceutically acceptable salt thereof is used for medical use, the dosage of the compound may be determined in accordance with the potency or property of that compound, to establish a dosage range which is effective enough for achieving the desired pharmacological efficacy. The dosage may vary depending on the administration route, age, bodyweight, and condition of the patient. A usual dosage range will be, for example, a range of 0.001 to 300 mg/kg per day.

The compound of the present invention for treating or preventing a disease or condition selected from the group consisting of schizophrenia, anxiety disorder, drug addiction, a disease comprising as a symptom a deficiency in cognition, mood disorder and mood episode is applied to a human.

However, it may also be applied to mammals other than a human.

Hereinafter, the present invention is illustrated in more detail by the following Examples. The examples are given to illustrate the invention, but should not be construed to limit it. Reference is made to the claims for determining what is reserved to the inventors.

### EXAMPLES

### Experimental Example 1: Measurement of PDE10 inhibitory activity

(1) The enzyme PDE10 (PDE10A) was isolated and prepared from bovine corpus striatum, according to the methods described in references Fujishige et al., Eur. J. Biochem., vol.266, pp. 1118-1127, 1999. The enzyme solution obtained was used for a PDE assay.

The PDE assay was performed according to the method described in Kotera et al. (Kotera et al., Biochem.Pharmacol., vol.60, pp.1333-1341, 2000), by the radiolabeled nucleotide method.

Specifically, the measurements of the inhibitory activities were carried out in one of the following methods.

(Method-A) The enzymatic reaction was carried out in 500 µl of assay buffer [50 mM Tris-HCl, pH8.0, 5mM MgCl₂, 4mM 2-mercaptoethanol, 0.33mg/ml bovine serum albumin] containing as a substrate about 4.8 nM [³H]-cAMP +0.25 µM unlabeled cAMP (available from Amersham Biosciences) or about 9.6 nM [³H]-cAMP.

The reaction was carried out for 30 minutes while keeping the temperature at 37°C. It was stopped by boiling the reaction mixture for 1.5 minutes, and further added thereto was 100 µl of snake venom (Crotalus atrox snake venom 1mg/ml) and the temperature was kept at 37°C for 30 minutes. Then 500 µl of methanol was added, and the reaction mixture was applied to a Dowex column (1x8 200-400). Subsequently, scintillation cocktail was added to each aliquot of the eluant, and the radioactivity was measured in a scintillation counter. Thus, PDE activity taking cAMP as a substrate (an activity to hydrolyze cAMP) was measured.

In the measurements of the inhibitory activities of compounds, a test compound was added to the above-mentioned reaction mixture, in various concentrations, and PDE activity was measured in the presence or absence of the test compound. From the measurements, its PDE 10 (PDE10A) inhibitory activity was determined.

(Method-B) The test compounds were dissolved in dimethyl sulfoxide (DMSO). 2 µL of the compound solution was added to 96 well plate, and the reaction mixture (20 µL of PDE enzyme solution in 50 mM Tris-HCl, pH 8.0, 40 µL of the assay buffer (50 mM Tris-HCl, pH 8.0, 2 mM MgCl2, 0.07 % 2-mercaptoethanol, and 0.825 mg/mL bovine serum albumin), and 20 µL of 1 mg/mL snake venom) was added to the 96 well plate. The enzyme reaction was started by adding and mixing with substrate solution of 20 µL containing approximate 35 nM [5',8-3H]cAMP in 50 mM Tris-HCl, pH 8.0. The final concentration of cAMP in the reaction mixtures was 7 nM. The reaction mixtures were incubated at room temperature for 90 min under dark conditions. After incubation, the reaction was stopped by adding 100 µL of methanol and resultant solutions were applied to filter plate containing Dowex (1×8 200-400) and centrifuged. 50 µL of the eluate together with wash eluate with additional 100 µL methanol was collected in another plate and the radioactivity was measured with 250 µL of scintillant.
(2) The compounds in the Examples below were tested for PDE inhibition using (Method-A) or (Method-B) described above.

They showed an IC₅₀ value of 100 nM or less. The IC₅₀ values of some preferred compounds are given in the following table.

| Example No. | IC50 (nM) | Method | | Example No. | IC50(nM) | Method |
|---|---|---|---|---|---|---|
| 1.008 | 0.44 | A | | 1.260 | 2.5 | B |
| 1.010 | 46 | A | | 1.268 | 0.7 | B |
| 1.013 | 4.2 | A | | 2.035 | 0.89 | B |
| 1.055 | 9.7 | A | | 2.039 | 0.84 | B |
| 1.057 | 29 | A | | 2.048 | 0.21 | B |
| 1.071 | 31 | A | | 2.053 | 0.64 | B |
| 1.159 | 0.60 | B | | 3.017 | 35 | A |
| 1.208 | 0.62 | B | | 3.029 | 38 | A |
| 1.211 | 0.76 | B | | 3.034 | 24 | A |
| 1.220 | 0.49 | B | | 4.001 | 0.86 | B |
| 1.226 | 20 | B | | 5.006 | 3.3 | B |
| 1.253 | 1.4 | B | | 5.012 | 2.3 | B |

### Example 1.001

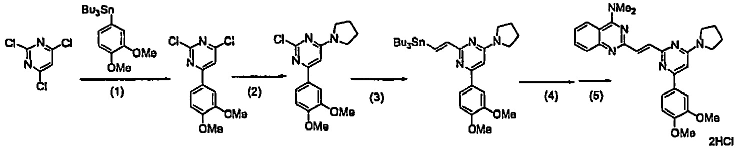

(1) A mixture of 2,4,6-trichloropyrimidine (50 g), tributyl[(3,4-dimethoxy)phenyl]stannane (60 g) and dichlorobis(triphenylphosphine)palladium (II) (4.9 g) in toluene (500 mL) was stirred at 60°C overnight. After being cooled to room temperature, the mixture was purified by silica gel column chromatography (hexane:ethyl acetate =30:1 →10:1) to give 2,4-dichloro-6-(3,4-dimethoxy)phenyl pyrimidine (23.8 g) as yellow powder (yield: 60%). The position isomer, 4,6-dichloro-2-(3,4-dimethoxy)phenyl pyrimidine (3.72 g) was also obtained as a yellow powder (yield: 9%).
   APCI-MS m/z 285 (M+H)+
(2) To a mixture of 2,4-dichloro-6-(3,4-dimethoxy)phenyl pyrimidine (3.00 g) and triethyamine (2.93 mL) in N,N-dimethylformamide (42 mL) was added pyrrolidine (0.97 mL) under ice-cooled conditions. After being stirred at room temperature for 2 hours, to the reaction solution was added ice water, and then the mixture was extracted with ethyl acetate. The organic layer was washed with water and saturated brine, dried over anhydrous sodium sulfate, and concentrated in vacuo. The residue was purified by silica gel column chromatography (hexane:ethyl acetate=1:1) to give 2-chloro-4-(3,4-dimethoxy)phenyl-6-(pyrrolidin-1-yl)pyrimidine (3.10 g) as a colorless powder (yield: 92%).
   APCI-MS m/z 320 (M+H)+
(3) A mixture of (E)-1,2-bis(tributylstannyl)ethylene (11.71 g) prepared according to the method recited in the literature (Stille et al., Organic Synthesis, 1988, vol.67, pp.86-97), 2-chloro-4-(3,4-dimethoxy)phenyl-6-(pyrrolidin-1-yl)pyrimidine (3.09 g), dichlorobis(triphenylphosphine)palladium (II) (343 mg), triphenylphosphine (507 mg) and copper bromide (I) (277 mg) in toluene (60 mL) was refluxed for 2 hours. After being cooled to room temperature, the reaction solution was purified by silica gel column chromatography (hexane:ethyl acetate=9:1 → 5:1) to give 4-(3,4-dimethoxy)phenyl-6-(1-pyrrolidinyl)-2-((*E*)-2-(tributylstannyl)ethenyl)pyrimidine (2.48 g) as a slightly yellow oil (yield: 43%).
   APCI-MS m/z 598 (M+H)+
(4) A mixture of 2-chloro-N,N-dimethylquinazolin-4-amine (the compound obtained in Reference Example 1 as described hereinafter) (62 mg), 4-(3,4-dimethoxy)phenyl-6-(1-pyrrolidinyl)-2-((E)-2-(tributylstannyl)ethenyl)pyrimidin e (150 mg), dichlorobis(triphenylphosphine)palladium (II) (10 mg), triphenylphosphine (13 mg), and copper bromide (I) (7 mg) in toluene (3 mL) was refluxed for 2.5 hours. After being cooled to room temperature, the reaction solution was purified by silica gel column chromatography (chloroform:methanol=100:0 → 95:5) to give 2- {(E)-2-[4-(3,4-dimethoxyphenyl)-6-pyrrolidin-1-yl-pyrimidin-2-yl]vinyl}-N,N-dimet hylquinazolin-4-amine (62 mg) as a yellow powder (yield: 51%).
   APCI-MS m/z 483 (M+H)+
(5) 2-{(E)-2-[4-(3,4-dimethoxyphenyl)-6-pyrrolidin-1-yl-pyrimidin-2-yl]vinyl}-N,N-dimethylquinazolin-4-amine (70 mg) was dissolved in methanol (1 mL), and 4N hydrochloric acid-ethyl acetate (0.3 mL) was added thereto. The mixture was diluted with diethyl ether and the precipitated powder was collected by filtration to give 2- {(E)-2-[4-(3,4-dimethoxyphenyl)-6-pyrrolidin-1-yl-pyrimidin-2-yl]vinyl} -N,N-dimethylquinazolin-4-amine dihydrochloride (Example 1.001 listed in Table 1 as described hereinafter) (55 mg) as a yellow powder (yield: 72%).

### Examples 1.002 to 1.072

The compounds of Examples 1.002 to 1.072 listed in Table 1 as described hereinafter were obtained in the similar manner as described in Example 1.001 using the corresponding raw materials.

### Examples 1.073 to 1.074

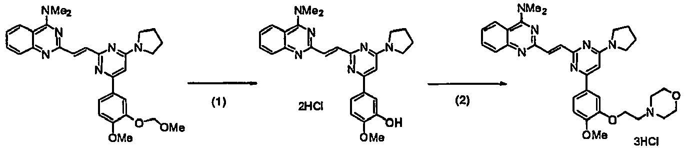

(1) A mixture of 2-((*E*)-2- {4-[4-methoxy-3-(methoxymethoxy)phenyl]-6-pyrrolidin-1-yl-pyrimidin-2-yl} vinyl)-N,N-dimethylquinazolin-4-amine (the free form of the compound of Example 1.060) (6.67 g) in 15% hydrochloric acid (6.5 mL)-methanol (100 mL) was refluxed for 1 hour. After being cooled to room temperature, the reaction solution was diluted with diethyl ether, and the precipitated powder was collected by filtration to give 5-(2-{(*E*)-2-[4-(dimethylamino)quinazolin-2-yl]vinyl}-6-pyrrolidin-1-yl pyrimidin-4-yl)-2-methoxyphenol·dihydrochloride (Example 1.073 listed in Table 1 as described hereinafter) (5.18 g) as a slightly yellow powder (yield: 74%).
(2) A mixture of 5-(2-{(*E*)-2-[4-(dimethylamino)quinazolin-2-yl]vinyl}-6-pyrrolidin-1-yl pyrimidin-4-yl)-2-methoxyphenol·dihydrochloride (2.0 g), 4-(2-chloroethyl)morpholine·hydrochloride (1.0 g), and cesium carbonate (6.0 g) in N,N-dimethylformamide (30 mL) was stirred at 80°C overnight. The reaction mixture was poured into ice water, and the precipitated powder was collected by filtration, washed with water, and dried. The resulting crude product was purified by silica gel column chromatography (chloroform:methanol=100:0 → 90:10) to give 2-((*E*)-2-{4-[4-methoxy-3-(2-morpholin-4-ylethoxy)phenyl]-6-pyrrolidin-1-yl-pyrimidi n-2-yl}vinyl)-N,N-dimethylquinazolin-4-amine (free form) (1.67 g) as a yellow powder.(yield: 78%) This was subjected to salt formation to give the trihydrochloride compound (Example 1.074 listed in Table 1 as described hereinafter).

### Examples 1.075 to 1.102

The compounds of Examples 1.075 to 1.102 listed in Table 1 as described hereinafter were obtained in the similar manner as described in Example 1.073 or 1.074 using the corresponding raw materials.

### Examples 1.103 to 1.104

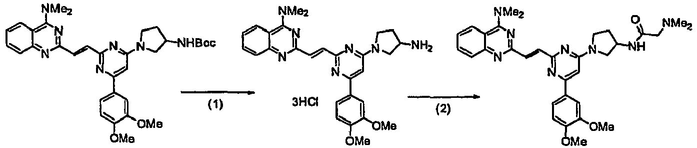

(1) *Tert*-butyl [1-(6-(3,4-dimethoxyphenyl)-2-{(*E*)-2-[4-(dimethylamino)quinazolin-2-yl]vinyl}pyrimi din-4-yl)pyrrolidin-3-yl] carbamate (the compound of the above Example 1.005) (1.61 g) was treated in the same manner as described in Example 1.073 (1) to give 2-{(*E*)-2-[4-(3-aminopyrrolidin-1 -yl)-6-(3,4-dimethoxyphenyl)pyrimidin-2-yl]vinyl}-N, N-dimethylquinazolin-4-amine-trihydrochloride (Example 1.103 listed in Table 1 as described hereinafter) (1.59 g) as a colorless powder (yield: 99%).
(2) To a mixture of 2-{(*E*)-2-[4-(3-aminopyrrolidm-l-yl)-6-(3,4-dimethoxyphenyl)pyrimidin-2-yl]vinyl}-N, N-dimethylquinazolin-4-amine(150 mg) in N,N-dimethylformamide (3 mL) were added sequentially N,N-dimethylglycine•hydrochloride (55 mg), 1-hydroxybenzotriazole (50 mg), triethyamine (0.1 mL), followed by 1-ethyl-3-[3-(dimethylamino)propyl]carbodiimide-hydrochloride (70 mg), and the mixture was stirred at room temperature for 15 hours. To the reaction mixture was added an aqueous sodium bicarbonate, and the mixture was extracted with ethyl acetate. The organic layer was washed with water and saturated brine, dried over anhydrous sodium sulfate, and concentrated in vacuo. The residue was purified by silica gel column chromatography (chloroform:methanol=100:0 → 60:40) to give N¹-[1-(6-(3,4-dimethoxyphenyl)-2-{(*E*)-2-[4-(dimethylamino)quinazolin-2-yl]vinyl}pyr imidin-4-yl)pyrrolidin-3-yl]-N²,N²-dimethylglycine amide (Example 1.104 listed in Table 1 as described hereinafter) (99 mg) as a yellow powder (yield: 56%).

### Example 1.105

The compound of Example 1.105 listed in Table 1 as described hereinafter was obtained in the same manner as described in Example 1.104 using the corresponding raw materials.

### Example 1.106

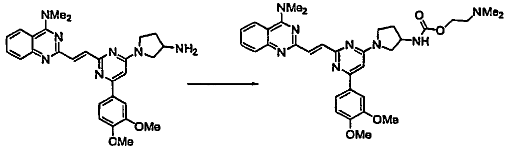

A mixture of 2-{(E)-2-[4-(3-aminopyrrolidin-1-yl)-6-(3,4-dimethoxyphenyl)pyrimidin-2-yl]vinyl}-N, N-dimethylquinazolin-4-amine trihydrochloride (the compound of Example 1.103 (1)) (150 mg), 2-(dimethylamino)ethyl chloride·hydrochloride (70 mg), and potassium carbonate (92 mg) in N,N-dimethylformamide (3 mL) was stirred at 70°C for 5 hours. The reaction mixture was poured into ice water, and extracted with ethyl acetate. The organic layer was washed with water and saturated brine, dried over anhydrous sodium sulfate, and concentrated in vacuo. The residue was purified by silica gel column chromatography (chloroform:methanol=100:0 → 90:10) to give 2-(dimethylaminoethyl) [1-(6-(3,4-dimethoxyphenyl)-2-{(*E*)-2-[4-(dimethylamino)quinazolin-2-yl]vinyl} pyrimidin-4-yl)pyrrolidin-3-yl] carbamate (Example 1.106 listed in Table 1 as described hereinafter) (51 mg) as a yellow powder (yield: 28%).

### Example 1.107

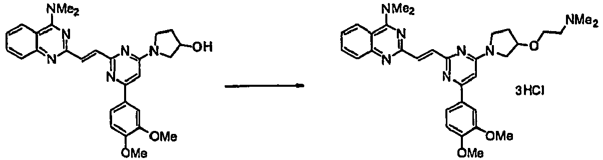

To a solution of 1-(6-(3,4-dimethoxyphenyl)-2-{(*E*)-2-[4-(diinethylamino)quinazolin-2-yl]vinyl}pyrimid in-4-yl)pyrrolidin-3-ol (the compound of Example 1.007) (100 mg) in N,N-dimethylformamide (5 mL) was added 60% sodium hydride (24 mg). After being stirred at room temperature for 10 minutes, 2-(dimethylamino)ethyl chloride hydrochloride (43 mg) was added and stirred at room temperature for 2 hours. Ice water was poured into the reaction mixture, and the mixture was extracted with chloroform. The organic layer was dried over anhydrous sodium sulfate, and concentrated in vacuo. The residue was purified by silica gel column chromatography (chloroform:methanol=100:0 → 80:20) to give 2-[(*E*)-2-[4-(3,4-dimethoxyphenyl)-6- {3-[2-(dimethylamino)ethoxy]pyrrolidin-1-yl} pyrimidin-2-yl]vinyl]-N,N-dimethylquinazolin-4-amine (free form) (21 mg) as a yellow powder (yield: 14%). This was subjected to salt formation to give the trihydrochloride compound (Example 1.107 listed in Table 1 as described hereinafter).

### Example 1.108

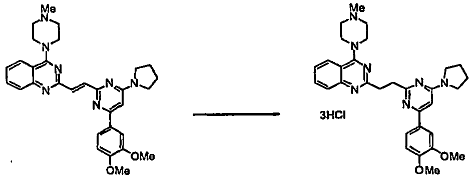

A mixture of 2-{(*E*)-2-[4-(3,4-dimethoxyphenyl)-6-pyrrolidin-1 -yl-pyrimidin-2-yl] vinyl} -4-(4-methy lpiperazin-1-yl)quinazoline (the compound of Example 1.031) (100 mg) and platinum oxide (10 mg) in methanol (4 mL)-tetrahydrofuran (4 mL) was stirred under hydrogen atmosphere (1 atm) at room temperature overnight. The catalyst was removed by filtration, and the filtrate was concentrated in vacuo. The resulting residue was purified by silica gel column chromatography (chloroform: methanol= 100:0 → 90:10). Subsequently, the treatment was carried out in the same manner as described in Example 1 to give 2-{2-[4-(3,4-dimethoxyphenyl)-6-pyrrolidin-1-yl-pyrimidin-2-yl]ethyl}-4-(4-methylpip erazin-1-yl)quinazoline·trihydrochloride (Example 1.108 listed in Table 1 as described hereinafter) (67 mg) as a colorless powder (yield: 56%).

### Example 1.109

The compound of Example 1.109 listed in Table 2 as described hereinafter was obtained in the same manner as described in Example 1.001 (2) to (5) using 4,6-dichloro-2-(3,4-dimethoxy)phenyl pyrimidine obtained in Example 1.001 (1).

### Example 1.110

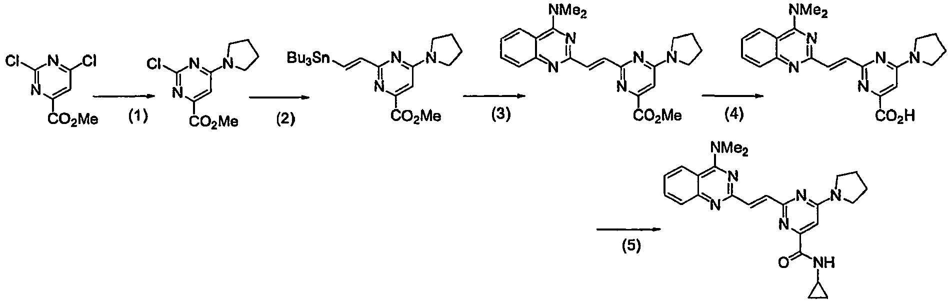

(1) Methyl 2,6-dichloro pyrimidin-4-carboxylate (2 g) prepared according to the method recitied in J. Org. Chem., 1961, vol.26, pp.2755-2763 was treated in the same manner as described in Example 1.001 (2) to give methyl 2-chloro-6-pyrrolidin-1-yl-pyrimidin-4-carboxyliate (1.95 g, yield: 84%) and methyl 6-chloro-2-pyrrolidin-1-yl-pyrimidin-4-carboxylate (148 mg, yield: 6%) as a pale yellow powder.
   APCI-MS m/z 242 (M+H)+
(2) Methyl 2-chloro-6-pyrrolidin-1-yl-pyrimidin-4-carboxylate (1.94 g) was treated in the same manner as described in Example 1.001 (3) to give methyl 6-pyrrolidin-1-yl-2-[(E)-2-tributylstannyl-vinyl]-pyrimidin-4-carboxylate (1.99 g) as a pale yellow oil (yield: 47%).
(3) Methyl 6-pyrrolidin-1-yl-2-[(E)-2-tributylstannyl-vinyl]-pyrimidin-4-carboxylate (1.99 g) was treated in the same manner as described in Example 1.001 (4) to give methyl 2-[(E)-2-(4-dimethylamino-quinazolin-2-yl)-vinyl]-6-pyrrolidin-1-yl-pyrimidin-4-carbo xylate (815 mg) as a yellow powder (yield: 53%).
   APCI-MS m/z 405 (M+H)+
(4) A mixture of methyl 2-[(E)-2-(4-dimethylamino-quinazolin-2-yl)-vinyl]-6-pyrrolidin-1-yl-pyrimidin-4-carbo xylate (773 mg), 2N-aqueous sodium hydroxide solution (2.87 mL), methanol (8 mL), and tetrahydrofuran (2 mL) was stirred at at room temperature for 3 hours. To the reaction solution was added 6N-aqueous hydrochloric acid solution (0.96 mL), and the mixture was extracted with chloroform. The organic layer was washed with water and saturated brine, dried over anhydrous sodium sulfate, and concentrated in vacuo. The residue was suspended in diisopropy ether, and the precipitated precipitate was collected by filtration to give 2-[(E)-2-(4-dimethylamino-quinazolin-2-yl)-vinyl]-6-pyrrolidin-1-yl-pyrimidin-4-carbo xylic acid (540 mg) as a yellow powder (yield: 72%).
   ESI-MS m/z 389 (M-H)-
(5) A mixture of 2-[(E)-2-(4-dimethylamino-quinazoli.n-2-yl)-vinyl]-6-pyrrolidin-1-yl-pyrimidin-4-carbo xylic acid (90 mg), cyclopropylamine (25 mg), 1-ethyl-3-[3-(dimethylamino)propyl]carbodiimide hydrochloride (53 mg), 1-hydroxybenzotriazole (37 mg), and N,N-dimethylformamide (3 mL) was stirred at room temperature overnight. To the reaction solution was added an aqueous sodium bicarbonate, and the mixture was extracted with ethyl acetate. The organic layer was washed with water and saturated brine, dried over anhydrous sodium sulfate, and concentrated in vacuo. The residue was suspended in diisopropy ether, and the precipitate was collected by filtration to give 2-[(E)-2-(4-dimethylamino-quinazolin-2-yl)-vinyl]-6-pyrrolidin-1-yl-pyrimidin-4-carbo xylic acid cyclopropyl amide (Example 1.110 listed in Table 3 as described hereinafter) (72 mg) as a pale yellow powder (yield: 73%).
The compound above is applied to salt formulation treatment to obtain salt forms, that is, hydrochloride, phosphate, hydrobromate, fumarate, citrate, methanesulfonate, benzenesulfonate, p-toluenesulfonate or maleate.

### Examples 1.111 to 1.146

The compounds Examples 1.111 to 1.146 listed in Table 3 as described hereinafter were obtained in the similar manner as described in Example 1.110 using the corresponding raw materials.

### Examples 1.147 to 1.262

The compounds of Examples 1.147 to 1.262 listed in Table 3 as described hereinafter were obtained in the similar manner as described in Example 1.110 using the corresponding raw materials.

### Example 1.263

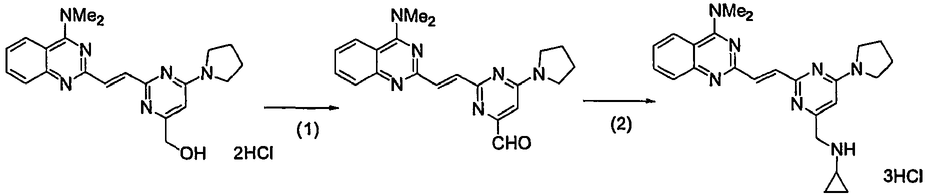

(1) A mixture of {2-[(E)-2-(4-dimethylammo-quinazolin-2-yl)-vinyl]-6-pyrrolidin-1-yl-pyrimidin-4-yl}-methanol·dihydrochloride (the compound of Reference Example 26) (260 mg), {1,1,1-tris(acetoxy)-1,1-dihydro-1,2-benziodoxol-3-(1H)-one} (308 mg), and dichloromethane (5 mL) was stirred at room temperature for 2 hours. To the reaction solution was added aqueous sodium thiosulfate solution, followed by an aqueous sodium bicarbonate, and the mixture was stirred at room temperature for 30 minutes, and extracted with chloroform. The organic layer was washed with water and saturated brine, dried over anhydrous sodium sulfate, and concentrated in vacuo. The residue was purified by silica gel column chromatography (chloroform:methanol=40:1) to give 2-[(E)-2-(4-dimethylamino-quinazolin-2-yl)-vinyl]-6-pyrrolidin-1-yl-pyrimidin-4-carbal dehyde (257 mg) as a yellow powder. (yield: 99%)
   APCI-MS m/z 375 (M+H)+
(2) To a mixture of 2-[(E)-2-(4-dimethylamino-quinazo lin-2-yl)-vinyl]-6-pyrrolidin-1-yl-pyrimidin-4-carbal dehyde (80 mg), cyclopropylamine (24 mg), and dichloromethane (2 mL) was added sodium triacetoxy borohydride (50 mg), and the mixture was stirred at room temperature for 2 hours. To the reaction solution was added an aqueous sodium bicarbonate, and the mixture was extracted with chloroform. The organic layer was washed with water and saturated brine, dried over anhydrous sodium sulfate, and concentrated in vacuo. The residue was purified by silica gel column chromatography (chloroform:methanol=85:15). The product was dissolved in chloroform (0.5 ml), and 4N-hydrochloric acid-ethyl acetate solution (0.5 mL) and diethyl ether (1 mL) were added. The precipitated powder was collected by filtration to give {2-[(E)-2-(4-cyclopropylaminomethyl-6-pyrrolidin-1-yl-pyrimidin-2-yl)-vinyl]-quinazo lin-4-yl}-dimethyl-amine·trihydrochloride (Example 1.263 listed in Table 3 as described hereinafter) (53 mg) as a yellow powder. (yield: 47%)

### Examples 1.264 to 1.265

The compounds of Examples 1.264 to 1.265 listed in Table 3 as described hereinafter were obtained in the similar manner as described in the above Example 1.263 using the corresponding raw materials.

### Examples 1.266 to 1.273

The compounds of Examples 1.266 to 1.273 listed in Table 3 as described hereinafter were obtained in the similar manner as described in Example 1.263 using the corresponding raw materials.

### Example 2.001

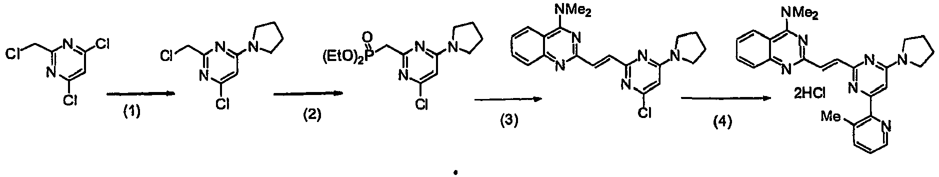

(1) 4,6-dichloro-2-(chloromethyl) pyrimidine (17.5 g) prepared according to the method recited in J. Chem. Soc. C, 1968, vol.17, pp.2188-98 was treated in the same manner as described in Example 1.001 (2) to give 4-chloro-2-(chloromethyl)-6-pyrrolidin-1-yl pyrimidine (19.6 g) as a brown solid. (yield: 95%)
   APCI-MS m/z 232/234 (M+H)+
(2) To a solution of diethyl phosphite (15 mL) in N,N-dimethylformamide (200 mL) was added 60% sodium hydride (4.1 g), and the mixture was stirred at room temperature for 30 minutes. Thereto was added a solution of 4-chloro-2-(chloromethyl)-6-pyrrolidin-1-yl pyrimidine (19 g) in N,N-dimethylformamide (50 mL), and the mixture was stirred at room temperature overnight. To the reaction solution was added ice water, and the mixture was extracted with ethyl acetate. The organic layer was washed with water and saturated brine, dried over anhydrous sodium sulfate, and concentrated in vacuo. The residue was suspended in hexane, and the precipitate was collected by filtration to give [(4-chloro-6-pyrrolidin-1-yl-pyrimidin-2-yl)methyl]diethyl phosphonate (26.9 g) as a yellow powder. (yield: 91 %)
   APCI-MS m/z 334 (M+H)+
(3) To a solution of [(4-chloro-6-pyrrolidin-1-yl-pyrimidin-2-yl)methyl]diethyl phosphonate(100 mg) and 4-(dimethylamino)quinazolin-2-carbaldehyde (the compound obtained in Reference Example 6 (5)) (90 mg) in N,N-dimethylformamide (3 mL) was added 60% sodium hydride (16 mg) under ice-cooled conditions, and the mixture was stirred at room temperature overnight. The reaction mixture was poured into ice water, and the precipitate was collected by filtration, washed with water, and then dried. The resulting crude product was purified by silica gel column chromatography (chloroform:methanol=100:0 → 90:10) to give 2-[(E)-2-(4-chloro-6-pyrrolidin-1-yl-pyrimidin-2-yl)vinyl]-N,N-dimethylquinazolin-4-a mine(92 mg) as a yellow powder. (yield: 81%)
(4) A mixture of 2-[(*E*)-2-(4-chloro-6-pyrrolidin-1-yl-pyrimidin-2-yl)vinyl]-N,N-dimethylquinazolin-4-a mine (100 mg), bis(tributylphosphine)palladium (3 mg) and 6-methyl-2-pyridyl zinc bromide (0.8 ml, 0.5M tetrahydrofuran solution) in N-methylpyrrolidone (1.0 mL) was stirred at 100°C for 1.5 hours. The reaction mixture was poured into an aqueous sodium bicarbonate, and the precipitate was collected by filtration, washed with water, and then dried. The resulting crude product was purified by silica gel column chromatography (chloroform:methanol=100:0 → 90:10) to give N,N-dimethyl-2-{(*E*)-2-[4-(3-methylpyridine-2-yl)-6-pyrrolidin-1-yl-pyrimidin-2-yl]vin yl}quinazolin-4-amine (free form) (96 mg) as yellow powder. (yield: 95%) This was subjected to salt formation to give the dihydrochloride compound (Example 2.001 listed in Table 4 as described hereinafter).

### Examples 2.002 to 2.003

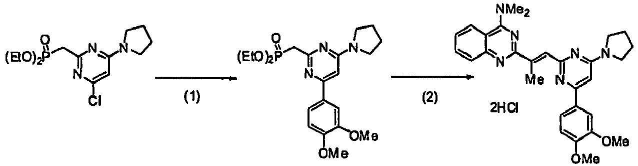

(1) A mixture of [(4-chloro-6-pyrrolidin-1-yl-pyrimidin-2-yl)methyl]diethyl phosphonate (the compound obtained in Example 2.001 (2)) (2.0 g), 3,4-dimethoxyphenyl borate (2.2 g), and dichlorobis(triphenylphosphine)palladium (II) (420 mg) in 1,2-dimethoxyethane (50 mL)-2M aquesous sodium carbonate solution (12 mL) was stirred at 90°C for 2.5 hours. After being cooled to room temperature, the reaction solution was diluted with ethyl acetate and washed with saturated brine. The organic layer was dried over anhydrous sodium sulfate, and concentrated in vacuo.
   The resulting residue was purified by silica gel column chromatography (chloroform:methanol=90:10) to give {[4-(3,4-dimethoxyphenyl)-6-pyrrolidin-1-yl-pyrimidin-2-yl]methyl}diethyl phosphonate (2.4 g) as colorless powder. (yield: 91%)
   APCI-MS m/z 436 (M+H)+
(2) {[4-(3,4-dimethoxyphenyl)-6-pyrrolidin-1-yl-pyrimidin-2-yl]methyl}diethyl phosphonate (200 mg), and 1-[4-(dimethylamino)quinazolin-2-yl]ethanone (the compound obtained in Reference Example 3 (2)) (50 mg) was treated in the same manner as described in Example 2.001 (3) to give 2- {(E)-2-[4-(3,4-dimethoxyphenyl)-6-pyrrolidin-l-yl-pyrimidin-2-yl]-1-methylvinyl}-N ,N-dimethylquinazolin-4-amine (free form) (32 mg) and 2,2'-(E)-ethen-1,2-diylbis[4-(3,4-dimethoxyphenyl)-6-pyrrolidin-1-yl pyrimidine] (free form) (15 mg) as a yellow powder, respectively. These were subjected to salt formation to give the respective dihydrochloride compounds (Example 2.002 and Example 2.003 listed in Table 4 as described hereinafter).

### Examples 2.004 to 2.005

The compounds of Examples 2.004 to 2.005 listed in Table 4 as described hereinafter were obtained in the similar manner as described in Example 2.002 using the corresponding raw materials.

### Example 2.006

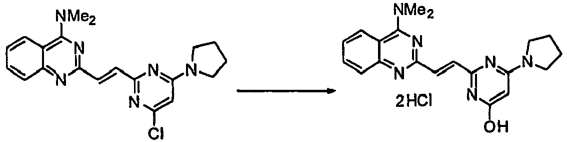

To a solution of 2-[(E)-2-(4-chloro-6-pyrrohdin-1-yl-pyrimidin-2-yl)vinyl]-N,N-dimethylquinazolin-4-a mine (the compound obtained in Example 2.001 (3)) (50 mg) in N,N-dimethylformamide (1 mL) was added 60% sodium hydride (15 mg), and the mixture was stirred at room temperature for 10 minutes. Subsequently, N,N,N'-trimethylethylenediamine (40 mg) was added, and the mixture was stirred at 80°C overnight. The reaction mixture was poured into ice water, and the precipitate was collected by filtration, washed with water, and then dried. The resulting crude product was purified by silica gel column chromatography (chloroform:methanol=100:0 → 90:10) to give 2-{(*E*)-2-[4-(dimethylamino)quinazolin-2-yl]vinyl}-6-pyrrolidin-1-yl-pyrimidin-4-ol (free form) (20 mg) as a yellow powder. (yield: 43%) This was subjected to salt formation to give the dihydrochloride compound (Example 2.006 listed in Table 4 as described hereinafter).

### Example 2.007

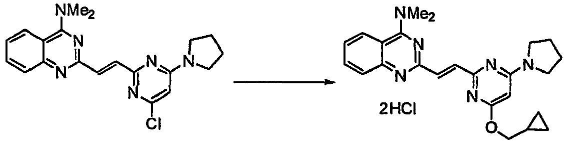

To a solution of 2-[(*E*)-2-(4-chloro-6-pyrrolidin-1-yl-pyrimidin-2-yl)vinyl]-N,N-dimethylquinazolin-4-a mine (the compound obtained in Example 2.001 (3)) (100 mg) and cyclopropylmethanol (58 mg) in N,N-dimethylformamide (3 mL) was added 60% sodium hydride (32 mg), and the mixture was stirred at 80°C overnight. The reaction mixture was poured into ice water, and the precipitate was collected by filtration, washed with water, and then dried. The resulting crude product was purified by silica gel column chromatography (chloroform:methanol=100:0 → 95:5) to give 2-{(*E*)-2-[4-(cyclopropylmethoxy)-6-pyrrolidin-1-yl-pyrimidin-2-yl]vinyl}-N,N-dimeth ylquinazolin-4-amine (free form) (73 mg) as a brown powder.(yield: 77%) This was subjected to salt formation to give the dihydrochloride compound (Example 2.007 listed in Table 4 as described hereinafter).

### Examples 2.008 to 2.031

The compounds of Examples 2.008 to 2.031 listed in Table 4 as described hereinafter were obtained in the similar manner as described in the above Example 2.007 using corresponding raw materials.

### Examples 2.032 to 2.060

The compounds of Examples 2.032 to 2.060 listed in Table 4 as described hereinafter were obtained in the similar manner as described in Example 2.007 using the corresponding raw materials.

### Example 2.061

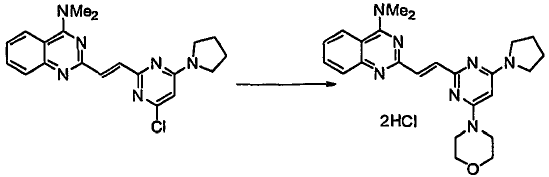

A mixture of 2-[(*E*)-2-(4-chloro-6-pyrrolidin-1-yl-pyrimidin-2-yl)vinyl]-N,N-dimethylquinazolin-4-a mine (the compound obtained in Example 2.001(3)) (90 mg) and morpholine (1.0 mL) in 1,4-dioxane (1 mL) was stirred at 120°C overnight. The reaction mixture was poured into ice water, and the precipitate was collected by filtration, washed with water, and then dried. The resulting crude product was purified by NH silica gel column chromatography (hexane:ethyl acetate=100:0 → 10:90) to give N,N-dimethyl-2-[(*E*)-2-(4-morpholin-4-yl-6-pyrrolidin-1-yl-pyrimidin-2-yl)vinyl]quina zolin-4-amine (free form) (22 mg) as a yellow powder.(yield: 18%) This was subjected to salt formation to provide the dihydrochloride compound (Example 2.061 listed in Table 4 as described hereinafter).

### Examples 2.062 to 2.063

The compounds of Examples 2.062 to 2.063 listed in Table 4 as described hereinafter were obtained in the similar manner as described in Example 2.061 using the corresponding raw materials.

### Example 3.001

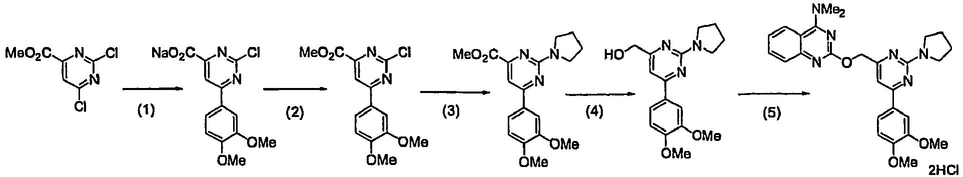

(1) A mixture of 2,6-dichloro pyrimidin-4-carboxylic acid methylester (5.91 g), 3,4-dimethoxyphenyl borate (5.46 g), dichlorobis(triphenylphosphine)palladium (II) (1.00 g), 2M-aquesous sodium carbonate solution (90 mL), and 1,2-dimethoxyethane (130 mL) was refluxed for 40 minutes. After the reaction solution was cooled to room temperature, the precipitate was collected by filtration, and washed with diisopropy ether to give sodium 2-chloro-6-(3,4-dimethoxy-phenyl)-pyridine-4-carboxyliate (12.33 g) as a colorless powder.
(2) The compound obtained in the above was suspended in N,N-dimethylformamide (75 mL), and iodomethane (7.98 mL) was added thereto, and the mixture was stirred at room temperature overnight. To the reaction solution was added ice water, and the precipitate was collected by filtration. The resulting powder was dissolved in chloroform, washed with water and saturated brine, dried over anhydrous sodium sulfate, and concentrated in vacuo. The residue was suspended in diisopropy ether, and the precipitated precipitate was collected by filtration to give methyl 2-chloro-6-(3,4-dimethoxy-phenyl)-pyridine-4-carboxylate (5.86 g) as a pale brown powder. (yield: 66%)
   APCI-MS m/z 309 (M+H)+
(3) Methyl 2-chloro-6-(3,4-dimethoxy-phenyl)-pyridine-4-carboxylate (5.79 g) was treated in the same manner as described in Example 1.001 (2) to give methyl 6-(3,4-dimethoxy-phenyl)-2-pyrrolidin-1-yl-pyrimidin-4-carboxylate (6.30 g) as a pale yellow powder. (yield: 98%)
   APCI-MS m/z 344 (M+H)+
(4) Methyl 6-(3,4-dimethoxy-phenyl)-2-pyrrolidin-1-yl-pyrimidin-4-carboxylate (3.00 g) was treated with sodium borohydride in the same manner as described in Reference Example 25 to give [6-(3,4-dimethoxy-phenyl)-2-pyrrolidin-1-yl-pyrimidin-4-yl]-methanol (2.59 g) as colorless powder. (yield: 94%)
   APCI-MS m/z 316 (M+H)+
(5) A mixture of [6-(3,4-dimethoxy-phenyl)-2-pyrrolidin-1-yl-pyrimidin-4-yl]-methanol (200 mg), 4-dimethylamino-2-chloroquinazoline (198 mg), 60% sodium hydride (38 mg), N,N-dimethylformamide (2 mL), and tetrahydrofuran (3 mL) was stirred at 60°C for 2 hours. To the reaction solution was added ice water, and the mixture was extracted with ethyl acetate. The organic layer was washed with water and saturated brine, dried over anhydrous sodium sulfate, and concentrated in vacuo. The residue was purified by silica gel column chromatography (chloroform: ethyl acetate=9:1→1:1). The product was dissolved in chloroform (0.5 ml), and 4N-hydrochloric acid-ethyl acetate solution (0.5 mL) and diethyl ether (1 mL) were added thereto. The precipitate was collected by filtration to give {2-[6-(3,4-dimethoxy-phenyl)-2-pyrrolidin-1-yl-pyrimidin-4-ylmethoxy]-quinazolin-4-yl}-dimethyl-amine·dihydrochloride (Example 3.001 listed in Table 5 as described hereinafter) (238 mg) as pale a yellow powder. (yield: 69%)

### Examples 3.002 to 3.032

The compounds of Examples 3.002 to 3.031 listed in Table 5 as described hereinafter were obtained in the similar manner as described in Example 3.001 using the corresponding raw materials.

### Example 3.032

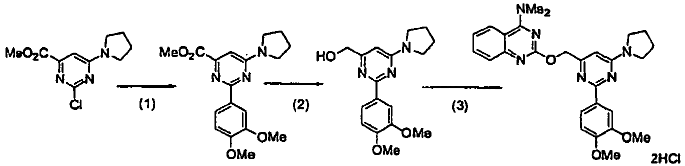

(1) Methyl 2-chloro-6-pyrrolidin-1-yl-pyrimidin-4-carboxylate (the compound obtained in Example1.110 (1)) (560 mg) was treated in the same manner as described in Example1.001 (1) to give methyl 2-(3,4-dimethoxy-phenyl)-6-pyrrolidin-1-yl-pyrimidin-4-carboxylate (591 mg) as a pale yellow powder.(yield: 74%) APCI-MS m/z 344 (M+H)+
(2) Methyl 2-(3,4-dimethoxy-phenyl)-6-pyrrolidin-1-yl-pyrimidin-4-carboxylate (580 mg) was treated with sodium borohydride in the same manner as described in Reference Example 25 to give [2-(3,4-dimethoxy-phenyl)-6-pyrrolidin-1-yl-pyrimidin-4-yl]-methanol (526 mg) as a colorless powder. (yield: 99%)
   APCI-MS m/z 316 (M+H)+
(3) [2-(3,4-dimethoxy phenyl)-6-pyrrolidin-1-yl-pyrimidin-4-yl]-methanol (100 mg) was treated in the same manner as described in Example 3.001 (5) to give {2-[2-(3,4-dimethoxy-phenyl)-6-pyrrolidin-1-yl-pyrimidin-4-ylmethoxy]-quinazolin-4-yl}-dimethyl-amine·dihydrochloride (Example 3.033 listed in Table 6 as described hereinafter) (160 mg) as a pale yellow powder. (yield: 90%)

### Examples 3.033 to 3.035

The compounds of Examples 3.033 to 3.035 listed in Table 6 as described hereinafter were obtained in the similar manner as described in Example 3.032 using the corresponding raw materials.

### Examples 4.001 to 4.002

The compounds of Examples 4.001 to 4.002 listed in Table 7 as described hereinafter were obtained from methyl 6-chloro-2-pyrrolidine-1-yl-pyrimidine-4-carboxylate in the similar manner as described in the above Example 1.110.

### Example 5.001

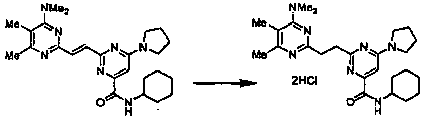

A suspension of N-cyclohexyl-2-{(E)-2-[4-(dimethylamino)-5,6-dimethylpyrimidin-2-yl]vinyl}-6-pyrrol idin-1-ylpyrimidin-4-carboxamide dihydrochloride (see Example 1.140 ; 169 mg, 0.323 mmol) and palladium on carbon (5%,170 mg) in methanol was stirred for 2 hour at room temperature under hydrogen atmosphere. The reaction mixture was filtrated and concentrated in vacuo. The residue was purified by trituration with diethyl ether to give N-cyclohexyl-2-{2-[4-(dimethylamino)-5,6-dimethylpyrimidin-2-yl]ethyl}-6-pyrrolidin -1-ylpyrimidin-4-carboxamide dihydrochloride (the compound of Example 5.001 listed in Table 8 as described hereinafter) as a colorless powder (148 mg, 87%).

### Examples 5.002 to 5.013

The compounds of Examples 5.002 to 5.013 listed in Table 8 as described hereinafter were obtained in the similar manner as described in Example 5.001 using the corresponding raw materials.

### Reference Example 1

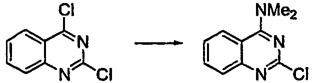

To a mixture of 2,4-dichloroquinazoline (10 g) prepared according to the method recited in J. Org. Chem., 1962, pp.957-961, and triethyamine (7.7 mL) in N,N-dimethylformamide (75 mL) was added 50% aqueous dimethylamine solution (4.98 g) under ice-cooled conditions. After being stirred at room temperature for 2 hours, to the reaction solution was added ice water, and the mixture was extracted with ethyl acetate. The organic layer was washed with water and saturated brine, dried over anhydrous sodium sulfate, and concentrated in vacuo to give 2-chloro-N,N-dimethylquinazolin-4-amine (Reference Example 1 listed in Table of Reference Example as described hereinafter) (9.32 g) as a pale brown powder. APCI-MS m/z 208/210 (M+H)+

### Reference Example 2

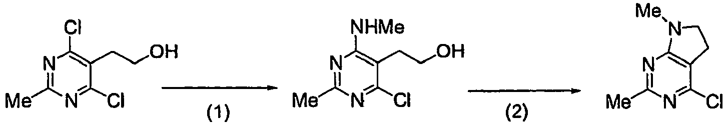

(1) A mixture of 2-(4,6-dichloro-2-methyl-pyrimidin-5-yl)-ethanol (2 g) prepared according to the method recited in WO 02/880951 A1, 40% aqueous methylamine solution (5 mL), and N,N-dimethylformamid (20 mL) was stirred at room temperature overnight. The reaction solution was poured into water, and the mixture was extracted with ethyl acetate. The organic layer was washed with water and saturated brine, dried over anhydrous sodium sulfate, and concentrated in vacuo to give 2-(4-chloro-2-methyl-6-methylamino-pyrimidin-5-yl)-ethanol (1.53 g) as a resinous compound. (yield: 79%) APCI-MS m/z 202 (M+H)+
(2) A mixture of 2-(4-chloro-2-methyl-6-methylamino-pyrimidin-5-yl)-ethanol (1.00 g), thionyl chloride (0.54 mL), and toluene (10 mL) was refluxed for 1 hour, and then concentrated in vacuo. The residue was suspended in N,N-dimethylformamide (10 mL), and potassium carbonate (2.06 g) was added, and the mixture was stirred at 100°C for 1 hour. To the reaction solution was added water, and the mixture was extracted with ethyl acetate. The organic layer was washed with water and saturated brine, dried over anhydrous sodium sulfate, and concentrated in vacuo. The residue was purified by silica gel column chromatography (hexane:ethyl acetate=1:1) to give 4-chloro-2,7-dimethyl-6,7-dihydro-5H-pyrrolo[2,3-d]pyrimidine (Reference Example 2 listed in Table of Reference Example as described hereinafter) (686 mg) as a pale yellow powder. (yield: 75%)
   APCI-MS m/z 184 (M+H)+

### Reference Example 3

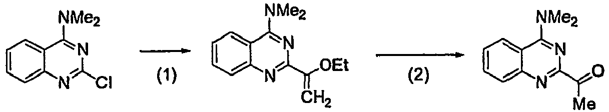

(1) 2-chloro-N,N-dimethylquinazolin-4-amine (the compound of the above Reference Example 1) (1.0 g) was treated in the same manner as described in Example 1.001 (4) to give 2-(1-ethoxyvinyl)-N,N-dimethylquinazolin-4-amine (1.0 g) as a pale yellow oil. (yield: 92%) APCI-MS m/z 244 (M+H)+
(2) 2-(1-ethoxyvinyl)-N,N-dimethylquinazolin-4-amine (1.0 g) was treated with hydrochloric acid-dioxane in the same manner as described in Example 1.150 to give 1-[4-(dimethylamino)quinazolin-2-yl]ethanone (Reference Example 3 listed in Table of Reference Example as described hereinafter) (624 mg) as a pale yellow powder. (yield: 60%)
APCI-MS m/z 216 (M+H)+

### Reference Example 4

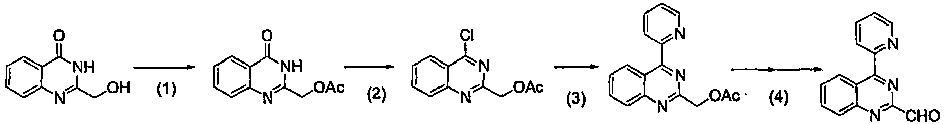

(1) A mixture of 2-(hydroxymethyl)quinazolin-4(3H)-one (100 mg) prepared according to the method recited in Tetrahedron, 1990, vol.46, pp.1295-1310, acetic anhydride (0.1 ml), and pyridine (1.0 ml) was stirred at room temperature for 5 hours. The reaction mixture was poured into ice water, and the precipitated powder was collected by filtration, washed with water, and then dried to give (4-oxo-3,4-dihydroquinazolin-2-yl)methyl acetate (82 mg) as a colorless oil. (yield: 66%) APCI-MS m/z 219 (M+NH₄)+
(2) A mixture of (4-oxo-3,4-dihydroquinazolin-2-yl)methyl acetate (76 mg), N,N-dimethylaniline (0.5 mL), and phosphorus oxychloride (3.2 g) was refluxed for 1.5 hours. After being cooled to room temperature, to the reaction solution was added water, and the mixture was extracted with ethyl acetate. The organic layer was washed with water followed by saturated brine, dried over anhydrous sodium sulfate, and concentrated in vacuo to give (4-chloroquinazolin-2-yl)methyl acetate (69 mg) as a brown powder. (yield: 84%)
   APCI-MS m/z 237 (M+NH₄)+
(3) The treatment was carried out in the same manner as described in Example 1.001 (3) using (4-chloroquinazolin-2-yl)methyl acetate (65 mg) and 2-tributylstannylpyridine to give (4-pyridine-2-ylquinazolin-2-yl)methyl acetate (68 mg) as a pale yellow oil. (yield: 89%)
   APCI-MS m/z 280 (M+H)+
(4) (4-pyridine-2-ylquinazolin-2-yl)methyl acetate (61 mg) was treated in the same manner as described in Example1.110 (4), and further treated in the same manner as described in Example 1.263 (1) to give 4-pyridin-2-ylquinazolin-2-carbaldehyde (Reference Example 4 listed in Table of Reference Example as described hereinafter) (43 mg) as a colorless powder. (yield: 80%)
   APCI-MS m/z 236 (M+H)+

### Reference Example 5

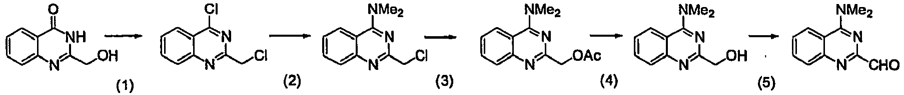

(1) 2-(hydroxymethyl)quinazolin-4(3H)-one (2.72 g) was treated in the same manner as described in Reference Example 4 (2) to give 4-chloro-2-(chloromethyl)quinazoline (3.24 g) as a pale yellow powder. (yield: 99%) APCI-MS m/z 213/215 (M+H)+
(2) 4-chloro-2-(chloromethyl)quinazoline (3.21 g) was treated in the same manner as described in Reference Example 1 to give 2-(chloromethyl)-N,N-dimethylquinazolin-4-amine(3.03 g) as a pale yellow powder. (yield: 91%)
   APCI-MS m/z 222/224 (M+H)+
(3) A mixture of 2-(chloromethyl)-N,N-dimethylquinazolin-4-amine (1.0 g) and potassium acetate (0.6 g) in N,N-dimethylformamide (1.0 mL) was stirred at 55°C for 4 hours. To the reaction solution was added ice water, and the mixture was extracted with ethyl acetate. The organic layer was washed with water and saturated brine, dried over anhydrous sodium sulfate, and concentrated in vacuo to give [4-(dimethylamino)quinazolin-2-yl]methyl acetate (1.1 g) as a pale yellow oil. (yield: 99%)
   APCI-MS m/z 246 (M+H)+
(4) [4-(dimethylamino)quinazolin-2-yl]methyl acetate (1.07 g) was treated in the same manner as described in Example 1.110 (4) to give [4-(dimethylamino)quinazolin-2-yl]methanol (0.85 g) as a pale yellow powder. (yield: 95%)
   APCI-MS m/z 204 (M+H)+
(5) [4-(dimethylamino)quinazolin-2-yl]methanol (250 mg) was treated in the same manner as described in Example 1.263 (1) to give 4-(dimethylamino)quinazolin-2-carbaldehyde (Reference Example 5 listed in Table of Reference Example as described hereinafter) (235 mg) as a colorless oil. (yield: 95%) APCI-MS m/z 202 (M+H)+

### Reference Example 6

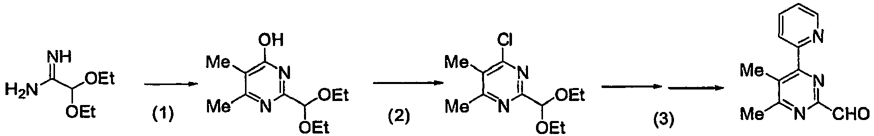

(1) To a suspension of 2,2-diethoxyacetamidinee hydrochloride (10 g) prepared according to the method recited in WO 01/21597, ethyl 2-methyl acetoacetate (23.7 g) in ethanol was added 28% sodium methoxide-methanol solution (55 mL), and the mixture was refluxed overnight. The solvent was distilled off, and the resulting residue was neutralized with aqueous citric acid solution, and then extracted with chloroform. The organic layer was washed with water and saturated brine, dried over anhydrous sodium sulfate, and concentrated in vacuo. The residue was purified by silica gel column chromatography (chloroform:ethyl acetate=9:1) to give 2-(diethoxymethyl)-5,6-dimethyl pyrimidin-4-ol (8.78 g) as a pale yellow powder. (yield: 71%)
   APCI-MS m/z 227 (M+H)+
(2) To a mixture of 2-(diethoxymethyl)-5,6-dimethyl pyrimidin-4-ol (2.2 g) in N,N-dimethylformamide (30 mL) was added thionyl chloride (1.5 mL) under ice-cooled conditions, and the mixture was stirred at room temperature for 2 hours. The reaction solution was neutralized with an aqueous sodium bicarbonate, and extracted with ethyl acetate. The organic layer was washed with water and saturated brine, dried over anhydrous sodium sulfate, and concentrated in vacuo to give 4-chloro-2-(diethoxymethyl)-5,6-dimethyl pyrimidine (1.5 g) as a yellow powder.
   (yield: 63%)
   APCI-MS m/z 245/247 (M+H)+
(3) 4-chloro-2-(diethoxymethyl)-5,6-dimethyl pyrimidine (731 mg) and 2-tributylstannylpyridine were used and treated in the same manner as described in Example 1.001 (3), and further treated in the same manner as described in Example 1.150 to give 4-(pyridin-2-yl)-5,6-dimethyl pyrimidin-2-carbaldehyde (Reference Example 6 listed in Table of Reference Example as described hereinafter) (349 mg) as a pale brown powder. (yield: 99%)
   APCI-MS m/z 214 (M+H)+

### Reference Example 7

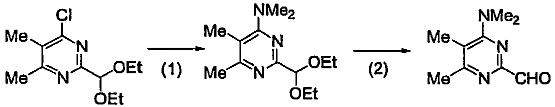

(1) 4-chloro-2-(diethoxymethyl)-5,6-dimethyl pyrimidine (750 mg) was treated in the same manner as described in Reference Example 1 to give 2-(diethoxymethyl)-N,N,5,6-tetramethyl pyrimidin-4-amine (610 mg) as a pale brown oil. (yield: 79%)
   APCI-MS m/z 254 (M+H)+
(2) 2-(diethoxymethyl)-N,N,5,6-tetramethyl pyrimidin-4-amine (600 mg) was treated in the same manner as described in Example 1.150 to give 4-(dimethylamino)5,6-dimethyl pyrimidin-2-carbaldehyde (Reference Example 7 listed in Table of Reference Example as described hereinafter) (412 mg) as a pale brown powder. (yield: 97%)
   APCI-MS m/z 180 (M+H)+

### Reference Example 8

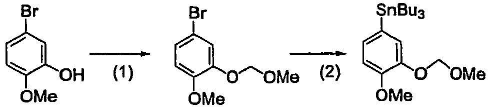

(1) To a mixture of 5-bromo-2-methoxyphenol (38.8 g) prepared according to the method recited in J. Med. Chem., 2001, vol.44, pp.2523 and diisopropylethylamine (67 mL) in methylene chloride (300 mL) was added methoxymethyl chloride (22 mL) under ice-cooled conditions. After being stirred at room temperature overnight, the reaction solution was washed with water and saturated brine. The organic layer was dried over anhydrous sodium sulfate, and concentrated in vacuo to give 4-bromo-1-methoxy 2-(methoxymethoxy)benzene (47 g) as a colorless oil. (yield: 99%)
   APCI-MS m/z 264/266 (M+NH₄)+
(2) To a solution of 4-bromo-1-methoxy 2-(methoxymethoxy)benzene (7.0 g) in tetrahydrofuran (80 mL) was added n-butyllithium (1.6M hexane solution) (18 ml) under -78°C, and the mixture was stirred at the same temperature for 30 minutes. Thereto was added tributyl tin chloride (8.0 ml), and the mixture was further stirred at the same temperature for 2 hours. To the reaction solution was added ice water, and the mixture was extracted with hexane. The organic layer was washed with water and saturated brine, dried over anhydrous sodium sulfate, and concentrated in vacuo. The residue was purified by silica gel column chromatography (hexane:ethyl acetate=30: 1) to give tributyl[4-methoxy-3-(methoxymethoxy)phenyl]stannane (Reference Example 8 listed in Table of Reference Example as described hereinafter) (9.9 g) as a colorless oil. (yield: 77%)
   ¹H-NMR (CDCl₃) δ7.22 (d, 1H, J = 1.3 Hz), 7.06 (dd, 1H, J = 7.9, 1.1 Hz), 6.91 (d, 1H, J = 7.9 Hz), 5.22 (s, 2H), 3.86 (s, 3H), 3.52 (s, 3H), 1.59-0.86 (m, 27H).

### Reference Example 9

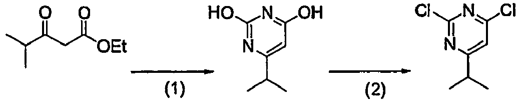

(1) A mixture of ethyl isobutylyl acetate (10.0 g), urea (5.69 g), 28% sodium methylate-methanol solution (31.5 mL), and ethanol (100 mL) was refluxed for 40 hours. After the reaction solution was cooled to room temperature, ethanol was distilled under reduced pressure. To the residue was added 10%-aqueous hydrochloric acid solution to make it acidic, and the mixture was extracted with chloroform. The organic layer was washed with water and saturated brine, dried over anhydrous sodium sulfate, and concentrated in vacuo. The residue was suspended in diisopropy ether, and the precipitate was collected by filtration to give 6-isopropyl-pyrimidin-2,4-diol (2.66 g) as a colorless powder. (yield: 23%)
   ESI-MS m/z 153 (M-H)-
(2) A mixture of 6-isopropyl-pyrimidin-2,4-diol (3.50 g), N,N-dimethylaniline (1.9mL), and phosphorus oxychloride (11.1 mL) was refluxed for 4 hours. After being cooled to room temperature, the reaction solution was added to water, and extracted with chloroform. The organic layer was washed with an aqueous sodium bicarbonate, water followed by and saturated brine, dried over anhydrous sodium sulfate, and concentrated in vacuo to give 2,4-dichloro-6-isopropyl-pyrimidine (Reference Example 9 listed in Table of Reference Example as described hereinafter) (4.15 g) as an oil.
   (yield: 96%)

### Reference Examples 10 to 12

The compounds of Reference Examples 10 to 12 listed in Table of Reference Example as described hereinafter were obtained in the same manner as described in Reference Example 1 using the corresponding raw materials.

### Reference Example 13

The compounds of Reference Example 13 listed in Table of Reference Example as described hereinafter were obtained in the same manner as described in Reference Example 7 using the corresponding raw materials.

### Reference Example 14

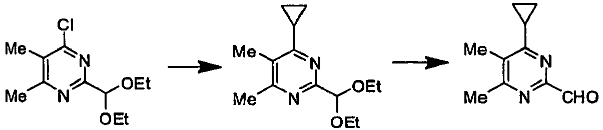

(1) To a solution of 4-chloro-2-(diethoxymethyl)-5,6-dimethylpyrimidine (see Reference Example 6 (2) ; 1.00 g, 4.09 mmol) and iron(III) acetylacetonate (72 mg, 0.204 mmol) in 1-methyl-2-pyrrolidinone (3.0 mL) and tetrahydrofuran (10 mL) was added cyclohexylmagnesium bromide (0.5M in tetrahydrofuran 24.5 mL, 12.3 mmol) at 0 °C. After being stirred for 5 min at 0 °C, the reaction mixture was poured in 1N hydrogene chloride solution, and then the mixture was extracted with ethyl acetate. The organic layer was washed with saturated brine, dried over magnesium sulfate, filtrated and concentrated in vacuo. The residue was purified by silica gel column chromatography (hexane:ethyl acetate = 4:1 1 to 2:1) to give 4-cyclohexyl-2-(diethoxymethyl)-5,6-dimethylaminopyrimidine as a pale yellow oil (911 mg, 89%). MS (APCI): m/z 251 (M+H).
(2) The preparation was carried out in the same manner as described in Reference Example 26 from 4-cyclohexyl-2-(diethoxymethyl)-5,6-dimethylaminopyrimidine (910 mg, 3.64 mmol) to give 4-cyclohexyl-5,6-dimethylaminopyrimidin-2-carbardehyde (the compound of Reference Example 14 listed in Table of Reference Example as described hereinafter) as a colorless oil (115 mg, 18%).

### Reference Example 15

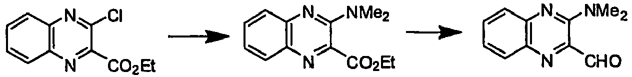

(1) To a solution of ethyl 3-chloroquinoxaline-2-carboxylate prepared by a method recited in J. Chem. Soc. 1945, 622; 12.3 g, 52.0 mmol and triethylamine (8.70 mL, 62.4 mmol) in N,N-dimethylformamide (52 mL) was added aqueous dimethylamine (50%, 6.60 mL, 62.7 mmol) at room temperature. After being stirred for 3 hour at room temperature, the reaction mixture was poured into water (500 mL), and the mixture was extracted with ethyl acetate (2000 mL). The organic layer was washed with water, dried over sodium sulfate, filtrated and concentrated in vacuo. The residue was purified by silica gel column chromatography (hexane:ethyl acetate = 4:1) to give ethyl 3-(dimethylamino)quinoxaline-2-carboxylate as a pale yellow oil (12.6 g, 99%). MS (APCI): m/z 246 (M+H).
(2) To a solution of ethyl 3-(dimethylamino)quinoxaline-2-carboxylate (6.32 g, 25.8 mmol) in tetrahydrofuran (80 mL) was added diisobutylaluminium hydride (1.01 M solution in toluene, 77.0 mL, 77.8 mmol) dropwise over 10 min at -78 °C. The reaction mixture was stirred for 1 hour at -78 °C, and then methanol (77 mL) was added and allowed to warm to room temperature. The precipitate was removed through celite with ethyl acetate (1000 mL) and diethyl ether (1000 mL). The filtrate was combined and concentrated in vacuo. The residue was purified by silica gel column chromatography (hexane:ethyl acetate = 9:1 to 1:1) to give 3-dimethylaminoqunoxaline-2-carbaldehyde (the compound of Reference Example 15 listed in Table of Reference Example as described hereinafter) as a yellow solid (4.85 g, 94%).

### Reference Example 16

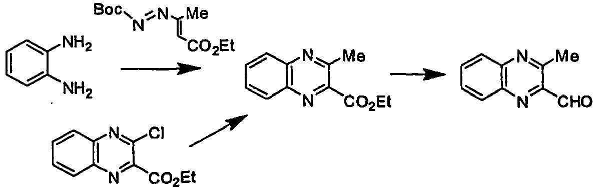

(1A) Method A: This preparation was performed in the same manner as described in Helv. Chim. Acta. 2001, 84, 2379 to give ethyl 3-methylquinoxali.ne-2-carboxylate.
(1B) Method B: A suspension of ethyl 3-chloroquinoxaline-2-carboxylate (11.5 g, 48.6 mmol) prepared by a method recited in J. Chem. Soc. 1945, 622, trimethylboroxine (6.06 g, 48.6 mmol), [1,1'-bis(diphenylphosphino)ferrocene]dichloropalladium(II) (1.98 g, 2.42 mmol), and potassium carbonate (13.4 g, 97.0 mmol) in 1,4-dioxane (162 mL) was heated for 4.5 hour at 115 °C. After being cooled to ambient temperature, the reaction mixture was filtrated through celite with ethyl acetate (500 mL). The filtrate was combined and concentrated in vacuo. The residue was purified by silica gel column chromatography (hexane:ethyl acetate = 9:1 to 2:1) followed by recrystallization from ethanol-water (1/4) to give ethyl 3-methylquinoxaline-2-carboxylate as a colorless crystals (8.36 g, 80%). mp 74-75 °C. MS (APCI): m/z 217 (M+H).
(2) To a solution of ethyl 3-methylquinoxaline-2-carboxylate (1.00 g, 4.62 mmol) in tetrahydrofuran (50 mL) was added diisobutylaluminium hydride (1.01 M solution in toluene, 13.7 mL, 13.8 mmol) dropwise at -78 °C. The reaction mixture was stirred for 1 hour at -78 °C, and then methanol was added and allowed to warm to room temperature. The precipitate was removed through celite with chloroform. The filtrate was combined and concentrated in vacuo. The residue was purified by silica gel column chromatography (hexane:ethyl acetate = 9:1 to 4:1) to 3-methylquinoxaline-2-carbaldehyde (the compound of Reference Example 16 listed in Table of Reference Example as described hereinafter) as a colorless powder (285 mg, 36%).

### Reference Examples 17 to 18

The compounds of Reference Examples 17 to 18 listed in Table as described hereinafter were obtained in the same manner as described in the above Reference Example 14 (1).

### Reference Example 19

To a solution of 2-chloroquinoline-3-carboxaldehyde (1.00 g, 5.19 mmol) in ethanol (24 mL) and dichloromethane (50 mL) was added bis(2-methxymethyl)aminosulfur trifluoride (1.26 g, 5.70 mmol) at 0 °C. After being stirred for 23 hour, the reaction mixture was poured into saturated aqueous sodium bicarbonate. The organic layer was separated and washed with saturated brine, dried over magnesium sulfate, filtrated and concentrated in vacuo. The residue was purified by silica gel column chromatography (hexane:ethyl acetate = 97:3 to 4:1) to give 2-chloro-3-(difluoromethyl)quinoline (the compound of Reference Example 19 listed in Table of Reference Example as described hereinafter) as a pale yellow powder (823 mg, 74%)..

### Reference Example 20

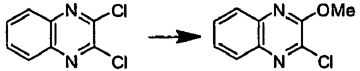

To a suspension of 2,3-dichloroquinoxaline (300 mg, 1.51 mmol) in methanol (15 mL) and N,N-dimethylformamide (1.0 mL) was added sodium methoxide (28% in methanol, 309 mg, 1.66 mmol) dropwise at 0 °C. After being stirred for 2 hour at room temperature, the reaction mixture was concentrated in vacuo. The residue was diluted with chloroform and water. The organic layer was separated with phase separator and concentrated in vacuo. The residue was purified by silica gel column chromatography (hexane to hexane:ethyl acetate =19:1) to give 2-chloro-3-methoxyquinoxaline (the compound of Reference Example 20 listed in Table of Reference Example as described hereinafter) as a colorless powder (251 mg, 86%).

### Reference Example 21

The compounds of Reference Example 21 listed in Table of Reference Example as described hereinafter were obtained in the same manner as described in the above Reference Example 20 using the corresponding raw materials.

### Reference Example 22

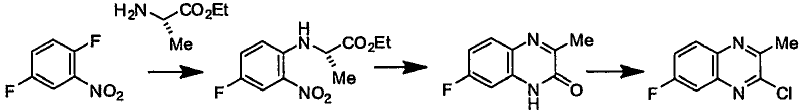

(1) A mixture of 2,5-difluoronitrobenzene (1.27 g, 8.00 mmol), L-alanine ethyl ester hydrochloride (1.84 g, 12.0 mmol), and triethylamine (2.10 mL, 15.1 mmol) in pyridine (10 mL) was heated for 17 hour at 80 °C. After being cooled to ambient temperature, the reaction mixture was diluted with ethyl acetate. The organic layer was washed with 10% aqueous copper(II) sulfate, dried over magnesium sulfate, filtrated and concentrated in vacuo. The residue was purified by silica gel column chromatography (hexane:ethyl acetate = 19:1 to 9:1) to give ethyl N-(4-fluoro-2-nitrophenyl)-L-alanine as an orange semi solid (1.52 g, 74%). MS (APCI): m/z 257 (M+H).
(2) A mixture of ethyl N-(4-fluoro-2-nitrophenyl)-L-alanine (1.58 g, 6.20 mmol) and tin(II) chloride dihydrate (5.60 g, 24.8 mmol) in ethanol (50 mL) and water (0.50 mL) was refluxed for 3.5 hour. After being cooled to ambient temperature, the reaction mixture was basified by 2N aqueous sodium hydroxide. The precipitate was removed by filtration through celite with diethyl ether and tetrahydrofuran. The filtrates were combined and concentrated in vacuo. A mixture of the residue and 2,3-dichloro-5,6-dicyano-p-benzoquinone (1.41 g, 6.20 mmol) in acetonitrile (50 mL) was stirred for 30 min at room temperature. The reaction mixture was dissolved to ethyl acetate-tetrahydrofuran (4:1). The organic layer was washed with saturated sodium bicarbonate, water and saturated brine, dried over magnesium sulfate, filtrated and concentrated in vacuo. The residue was purified by trituration with diethyl ether to give 7-fluoro-3-methylquinoxalin-2(1H)-one as a colorless needles (1.01 g, 91%). mp 258-260 °C. MS (APCI): m/z 179 (M+H).
(3) The preparation was performed in the same manner as described in Reference Example 4 (2) to give 2-chloro-7-fluoro-3-methylquinoxaline (the compound of Reference Example 22 listed in Table of Reference Example as described hereinafter).

### Reference Example 23

The compound of Reference Example 23 listed in Table of Reference Example as described hereinafter was obtained in the same manner as described in the above Reference Example 4 (2) using the corresponding raw materials.

### Reference Example 24

The compound of Reference Example 24 listed in Table of Reference Example as described hereinafter was obtained in the same manner as described in the above Example 1.100 using the corresponding raw materials.

### Reference Example 25

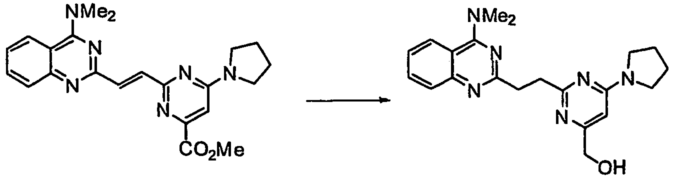

To a mixture of methyl 2-[(E)-2-(4-dimethylamino-quinazolin-2-yl)-vinyl]-6-pyrrolidin-1-yl-pyrimidin-4-carbo xylate (the compound obtained Example 1.110 (3)) (2.74 g), ethanol (25 mL), and tetrahydrofuran (50 mL) was added sodium borohydride (1.03 g) under ice-cooled conditions, and the mixture was stirred at room temperature for 14 hours. To the reaction solution was added ice water, and the mixture was extracted with chloroform. The organic layer was washed with water and saturated brine, dried over anhydrous sodium sulfate, and concentrated in vacuo. The residue was purified by NH silica gel column chromatography (chloroform:isopropanol 30:1→chloroform:methanol 20:1) to give {2-[2-(4-dimethylamino-quinazolin-2-yl)-ethyl]-6-pyrrolidin-1-yl-pyrimidin-4-yl}-meth anol (free form) (2.04 g) as a pale brown powder. (yield: 80%) This was subjected to salt formation to give the dihydrochloride compound (Reference Example 25 listed in Table of Reference Example as described hereinafter).

### Reference Example 26

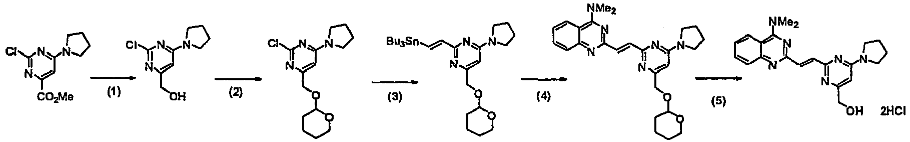

(1) Methyl 2-chloro-6-pyrrolidin-1-yl-pyrimidin-4-carboxylate (the compound obtained in Example 1.110 (1)) (4 g) was treated with sodium borohydride in the same manner as described in Reference Example 25 to give (2-chloro-6-pyrrolidin-1-yl-pyrimidin-4-yl)-methanol (2.94 g) as a colorless powder. (yield: 83%) APCI-MS m/z 216 (M+H)+
(2) A mixture of (2-chloro-6-pyrrolidin-1-yl-pyrimidin-4-yl)-methanol (1.92 g), 3,4-dihydro-2H-pyrane (832 mg), p-toluenesulfonic acid monohydrate (171 mg), and dichloromethane (20 mL) was stirred at room temperature overnight. To the reaction solution was added an aqueous sodium bicarbonate, and the mixture was extracted with chloroform. The organic layer was washed with water and saturated brine, dried over anhydrous sodium sulfate, and concentrated in vacuo. The residue was purified by silica gel column chromatography (hexane:ethyl acetate=3:1) to give 2-chloro-4-pyrrolidin-1-yl-6-(tetrahydro-pyran-2-yloxymethyl)-pyrimidine (2.56 g) as an oil.(yield: 96%)
   APCI-MS m/z 298 (M+H)+
(3) 2-chloro-4-pyrrolidin-1-yl-6-(tetrahydro-pyran-2-yloxymethyl)-pyrimidine (1.2 g) was treated in the same manner as described in Example 1.001 (3) to give 4-pyrrolidin-1-yl-6-(tetrahydro-pyran-2-yloxymethyl)-2-[(E)-2-tributylstannyl-vinyl]-py rimidine (1.79 g) as a pale brown oil.(yield: 77%)
(4) 4-pyrrolidin-1-yl-6-(tetrahydro-pyran-2-yloxymethyl)-2-[(E)-2-tributylstannyl-vinyl]-py rimidine (1.75 g) was treated in the same manner as described in the above Example 1.001 (4) to give dimethyl-(2- {(E)-2-[4-pyrrolidin-1-yl-6-(tetrahydro-pyran-2-yloxymethyl)-pyrimidin-2-yl]vinyl}-quinazolin-4-yl)-amine(395 mg) as a yellow powder. (yield: 28%)
   (5) A mixture of dimethyl-(2-{(E)-2-[4-pyrrolidin-1-yl-6-(tetrahydro-pyran-2-yloxymethyl)-pyrimidin-2-yl]vinyl}-quinazolin-4-yl)-amine (365 mg), 4N-hydrochloric acid-dioxane solution (1 mL), methanol (6 mL), and tetrahydrofuran (3 mL) was stirred at room temperature for 1 hour. To the reaction solution was added ice water, and the mixture was washed with diethyl ether. To the aqueous layer was added an aqueous sodium bicarbonate to make it alkaline, and the mixture was extracted with chloroform. The organic layer was washed with water and saturated brine, dried over anhydrous sodium sulfate, and concentrated in vacuo to give {2-[(E)-2-(4-dimethylamino-quinazolin-2-yl)-vinyl]-6-pyrrolidin-1-yl-pyrimidin-4-yl}-methanol (283 mg) as a pale yellow powder. (yield: 95%) The above product (20 mg) was dissolved in chloroform (0.5 ml), and 4N-hydrochloric acid-ethyl acetate solution (0.5 mL) and diethyl ether (1 mL) were added thereto. The precipitate was collected by filtration to give the dihydrochloride compound (Reference Example 26 listed in Table of Reference Example as described hereinafter) (23 mg) as a yellow powder. (yield: 96%)

The structural formula and physical properties, etc of the compounds of the Examples and the Reference Examples are shown in the following Tables and Tables of Reference Example.

In the tables, "MS(APCI)(m/z)" (or "MS■ APCI") means mass spectrometry (Atmospheric pressure chemical ionization mass spectrometry). The "mp" means melting point. The following abbreviations are utilized in the Examples, Reference Examples and the following tables:
"Me" means methyl group;
"Et" means ethyl group;
"Bu" means butyl group; and
"Boc" means tert-butoxycarbonyl group.

**Table 1**

| | | | | | |
|---|---|---|---|---|---|
| | 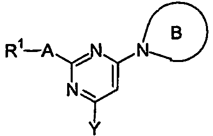 | | | | |

| Example No. | R¹-A- | 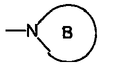 | -Y | Salt | Physical properties, etc. |
|---|---|---|---|---|---|
| 1.001 | 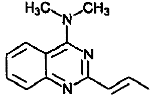 | 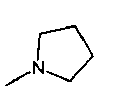 | 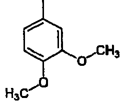 | 2H | MS■APCI(MeOH)-1: 483 [M+H]+ |
| 1.002 | 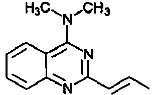 | 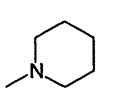 | 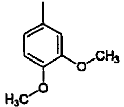 | 2HCl | MS■APCI(MeOH)-2 : 497[M+H]+ |
| 1.003 | 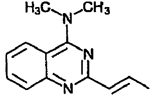 | 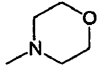 | 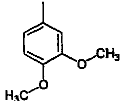 | 2HCl | MS■APCI(MeOH)-2 : 499[M+H]+ |
| 1.004 | 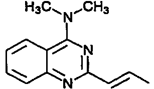 | 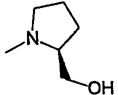 | 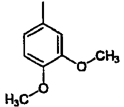 | 2HCl | MS■APCI(MeOH)-2: 513[M+H]+ |
| 1.005 | 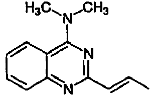 | 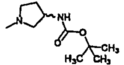 | 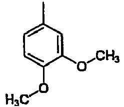 | free form | mp: 128-134°C |
| 1.006 | 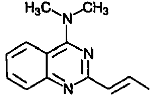 | 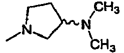 | 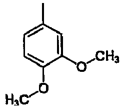 | free form | mp: 180-183°C |
| 1.007 | 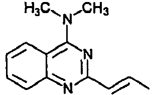 | 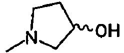 | 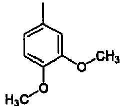 | free form | mp: 199-201 °C |
| 1.008 | 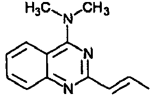 | 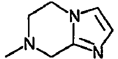 | 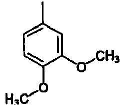 | free form | mp: 103-108°C |
| 1.009 | 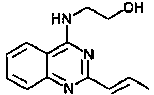 | 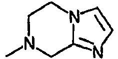 | 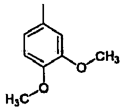 | free form | mp: 161°C |
| 1.010 | 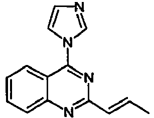 | 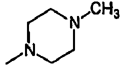 | 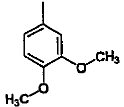 | free form | mp: 177-180°C |
| 1.011 | 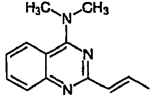 | 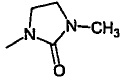 | 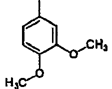 | free form | mp: 208-210°C |
| 1.012 | 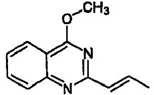 | 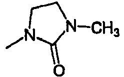 | 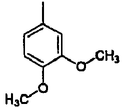 | free form | mp: 194-202°C |
| 1.013 | 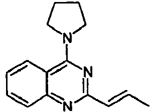 | 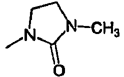 | 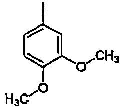 | free form | mp: 258-262°C (Degradation) |
| 1.014 | 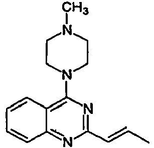 | 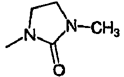 | 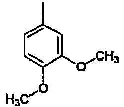 | free form | mp: 195-202°C |
| 1.015 | 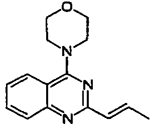 | 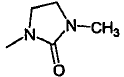 | 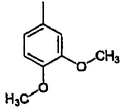 | free form | mp: 158-161°C |
| 1.016 | 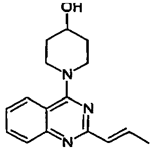 | 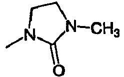 | 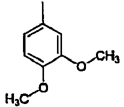 | free form | mp: 136-138°C |
| 1.017 | 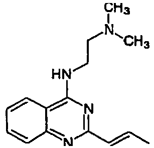 | 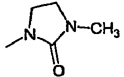 | 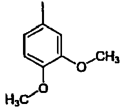 | free form | mp: 206-210 °C |
| 1.018 | 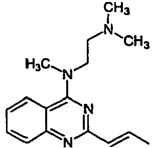 | 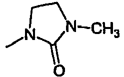 | 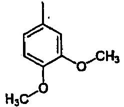 | free form | mp: 133-135°C |
| 1.019 | 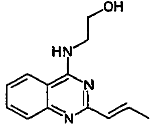 | 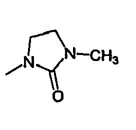 | 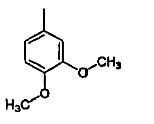 | free form | mp: 165-168°C |
| 1.020 | 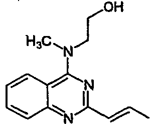 | 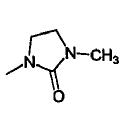 | 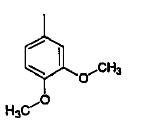 | free form | mp: 144-147°C |
| 1.021 | 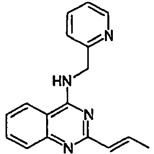 | 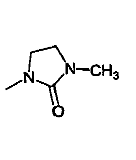 | 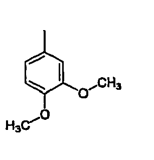 | free form | mp: 133-135°C |
| 1.022 | 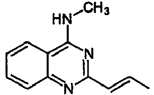 | 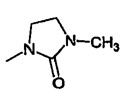 | 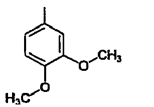 | free form | mp: 167-170°C |
| 1.023 | 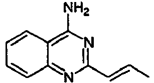 | 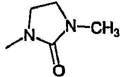 | 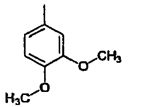 | free form | mp: 203°C |
| 1.024 | 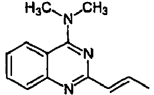 | 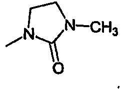 | 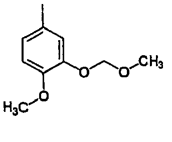 | free form | mp: 227-237°C |
| 1.025 | 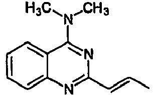 | 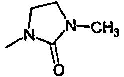 | 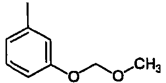 | free form | mp: 199-205°C |
| 1.026 | 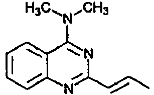 | 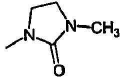 | 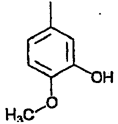 | free form | mp: 281-285°C |
| 1.027 | 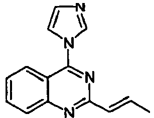 | 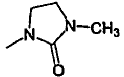 | 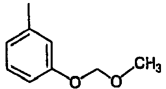 | free form | mp : 197-201 °C |
| 1.028 | 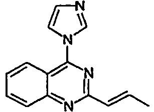 | 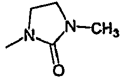 | 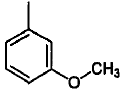 | free form | mp: 222-231 °C |
| 1.029 | 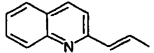 | 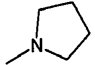 | 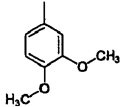 | 2HCl | MS■APCI(MeOH)-1: 439[M+H]+ |
| 1.030 | 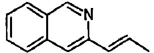 | 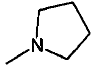 | 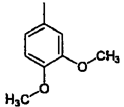 | 2HCl | MS■APCI(MeOH)-1: 439[M+H]+ |
| 1.031 | 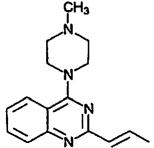 | 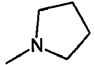 | 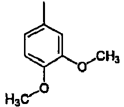 | 3HCl | MS■APCI (10mM-AcONH₄/MeOH)-1: 538[M+H]+ |
| 1.032 | 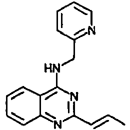 | 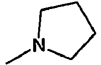 | 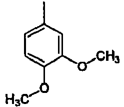 | free form | mp: 220-226°C |
| 1.033 | 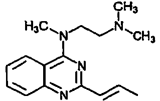 | 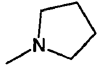 | 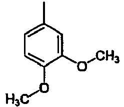 | free form | mp: 180-187°C |
| 1.034 | 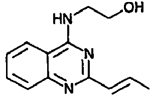 | 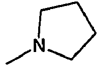 | 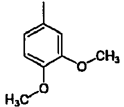 | free form | mp: 126-133°C |
| 1.035 | 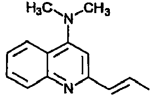 | 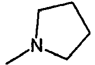 | 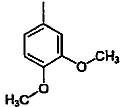 | 2HCl | MS■APCI(MeOH)-2 : 482/484 [M+H]+ |
| 1.036 | 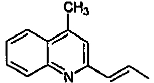 | 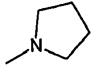 | 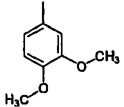 | 2HCl | MS■APCI (10mM-AcONH₄/MeOH)-1: 453 [M+H]+ |
| 1.037 | 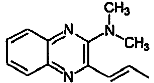 | 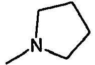 | 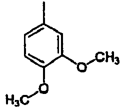 | 2HCl | MS■APCI(MeOH)-1: 483 [M+H]+ |
| 1.038 | 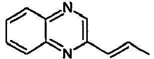 | 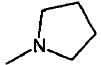 | 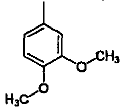 | 2HCl | MS■APCI (10mM-AcONHa/MeOH)-1: 440 [M+H]+ |
| 1.039 | 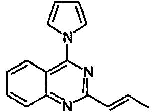 | 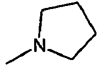 | 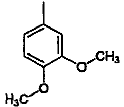 | 2HCl | MS■APCI(MeOH)-1: 505 [M+H]+ |
| 1.040 | 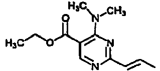 | 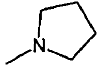 | 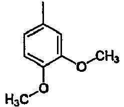 | 2HCl | MS■APCI(MeOH)-2 : 505 [M+H]+ |
| 1.041 | 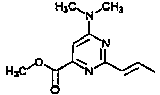 | 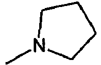 | 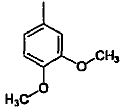 | 2HCl | MS■APCI(MeOH)-2: 491[M+H]+ |
| 1.042 | 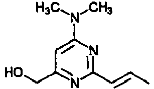 | 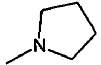 | 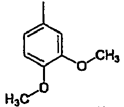 | 2HCl | MS■APCI(MeOH)-2 : 463[M+H]+ |
| 1.043 | 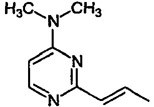 | 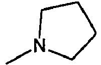 | 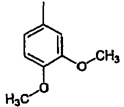 | 2HCl | MS-APCI: (10mM-AcONH₄/MeOH)-1: 433 [M+H]+ |
| 1.044 | 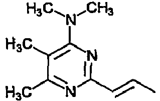 | 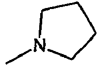 | 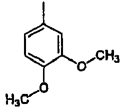 | 2HCl | MS■APCI(MeOH)-2: 461 [M+H]+ |
| 1.045 | 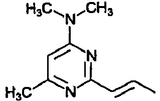 | 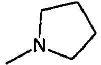 | 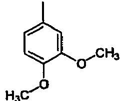 | 2HCl | MS■APCI(MeOH)-2: 447 [M+H] + |
| 1.046 | 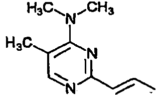 | 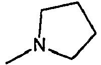 | 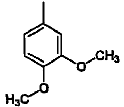 | 2HCl | MS■APCI(MeOH)-2 : 447[M+H]+ |
| 1.047 | 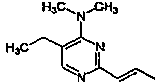 | 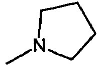 | 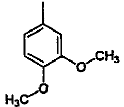 | 2HCl | MS■APCI: (10mM-AcONH₄/MeOH)-1: 461 [M+H]+ |
| 1.048 | 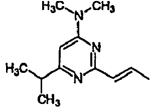 | 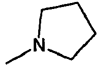 | 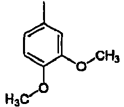 | 2HCl | MS■APCI(MeOH)-1: 475[M+H]+ |
| 1.049 | 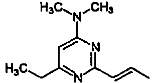 | 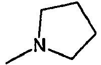 | 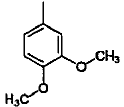 | 2HCl | MS■APCI: (10mM-AcONH₄/MeOH)-1: 461 [M+H]+ |
| 1.050 | 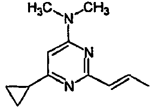 | 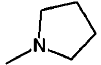 | 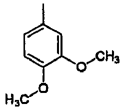 | 2HCl | MS■APCI (10mM-AcONH₄/MeOH)-1: 473[M+H]+ |
| 1.051 | 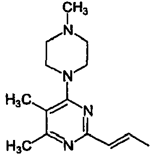 | 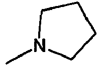 | 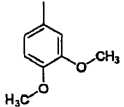 | 3HCl | MS■APCI(MeOH)-1: 516[M+H]+ |
| 1.052 | 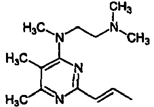 | 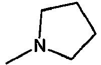 | 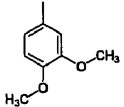 | 3HCl | MS■APCI(MeOH)-1: 518[M+H]+ |
| 1.053 | 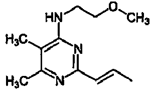 | 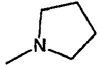 | 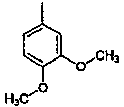 | 2HCl | MS■APCI(MeOH)-1: 491 [M+H]+ |
| 1.054 | 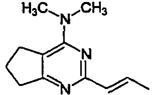 | 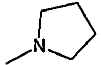 | 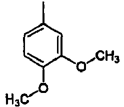 | 2HCl | MS■APCI (10mM-AcONH₄/MeOH)-1: 473 [M+H]+ |
| 1.055 | 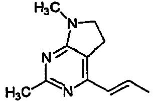 | 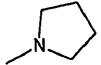 | 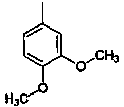 | 2HCl | MS■APCI (10mM-AcONH₄/MeOH)-1: 459 [M+H] + |
| 1.056 | 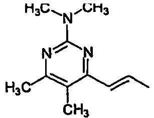 | 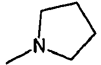 | 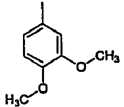 | 2HCl | MS■APCI (10mM-AcONH₄/MeOH)-1: 461 [M+H] + |
| 1.057 | 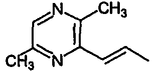 | 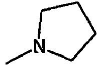 | 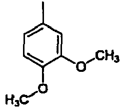 | 2HCl | MS■APCI (10mM-AcONH₄/MeOH)-1: 418[M+H] + |
| 1.058 | 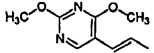 | 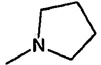 | 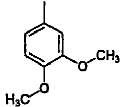 | free form | MS■APCI(MeOH)-1: 450 [M+H]+ |
| 1.059 | 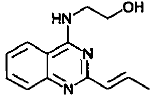 | 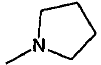 | 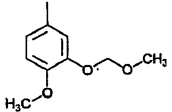 | free form | mp: 126°C |
| 1.060 | 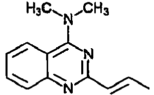 | 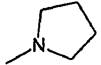 | 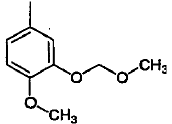 | HCl | MS■APCI(MeOH)-1 : 513[M+H]+ |
| 1.061 | 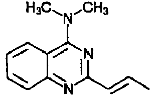 | 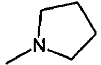 | 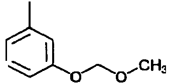 | free form | MS■APCI(MeOH): 483[M+H]+ |
| 1.062 | 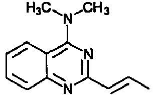 | 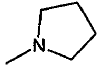 | 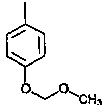 | free form | MS■APCI(MeOH): 483[M+H]+ |
| 1.063 | 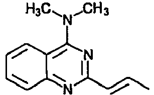 | 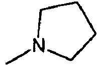 | 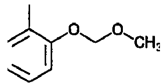 | free form | MS■APCI(MeOH): 483 [M+H] + |
| 1.064 | 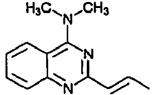 | 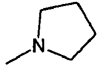 | 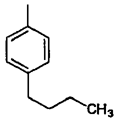 | 2HCl | MS■APCI(MeOH)-2: 479[M+H] + |
| 1.065 | 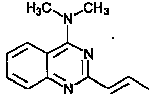 | 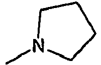 | 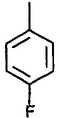 | 2HCl | MS■APCI(MeOH)-1: 441[M+H] + |
| 1.066 | 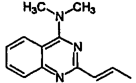 | 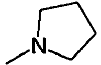 | 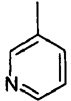 | 2HCl | MS■APCI(MeOH)-1: 424[M+H]+ |
| 1.067 | 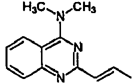 | 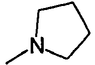 | 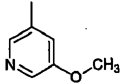 | 2HCl | MS·APCI(MeOH)-1:454 [M+H]+ |
| 1.068 | 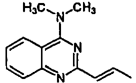 | 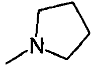 | 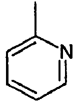 | 2HCl | MS · APCI(MeOH)-1: 424 [M+H]+ |
| 1.069 | 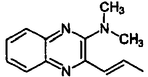 | 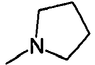 | 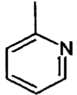 | 2HCl | MS · APCI(MeOH)-1: 424[M+H]+ |
| 1.070 | 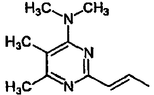 | 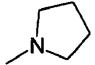 | 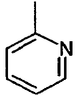 | 2HCl | MS·APCI(MeOH)-1: 402[M+H]+ |
| 1.071 | 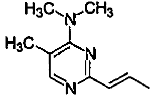 | 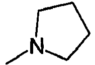 | 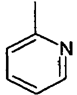 | 2HCl | MS · APCI(MeOH)-1: 388 [M+H]+ |
| 1.072 | 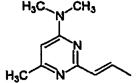 | 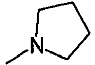 | 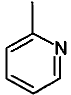 | 2HCl | MS · APCI(MeOH)-1: 388[M+H]+ |
| 1.073 | 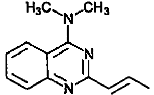 | 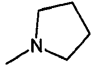 | 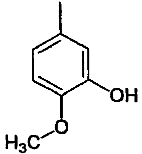 | 2HCl | MS · APCI(MeOH)-1: 469 [M+H] + |
| 1.074 | 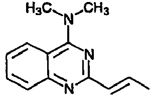 | 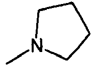 | 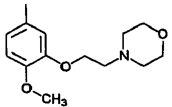 | 3HCl | MS·APCI(MeOH)-2 : 582 [M+H]+ |
| 1.075 | 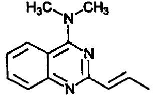 | 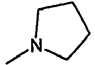 | 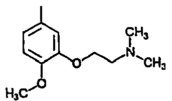 | free form | MS·APCI(MeOH)-1: 540 [M+H] + |
| 1.076 | 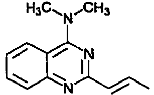 | 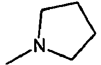 | 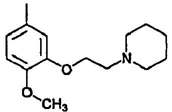 | 3HCl | MS·APCI(MeOH)-2 : 580 [M+H]+ |
| 1.077 | 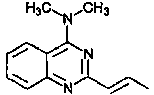 | 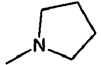 | 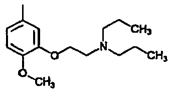 | 3HCl | MS·APCI(MeOH)-2 : 596[M+H]+ |
| 1.078 | 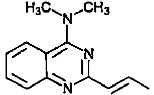 | 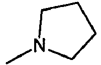 | 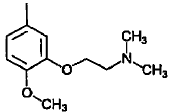 | 3HCl | MS · APCI(MeOH)-1: 540 [M+H]+ |
| 1.079 | 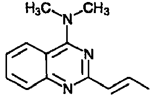 | 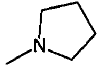 | 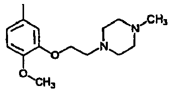 | 2HCl | MS · APCI(MeOH)-2 : 595[M+H]+ |
| 1.080 | 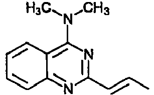 | 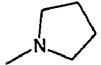 | 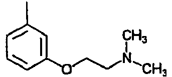 | 3HCl | MS·APCI(MeOH)-2 : 510[M+H]+ |
| 1.081 | 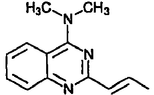 | 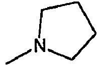 | 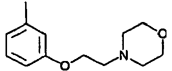 | 3HCl | MS·APCI(MeOH)-2: 552[M+H]+ |
| 1.082 | 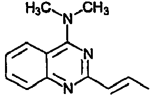 | 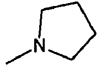 | 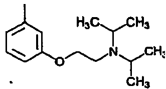 | 3HCl | MS·APCI(MeOH)-1: 566 [M+H]+ |
| 1.083 | 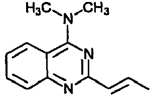 | 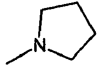 | 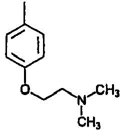 | 3HCl | MS·APCI(MeOH)-2 : 510[M+H]+ |
| 1.084 | 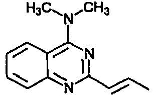 | 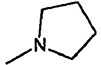 | 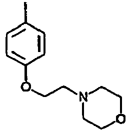 | 3HCl | MS · APCI(MeOH)-2 : 552 [M+H]+ |
| 1.085 | 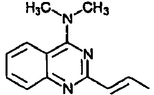 | 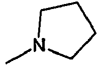 | 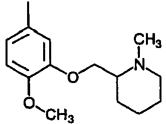 | 3HCl | MS·APCI(MeOH)-2 : 580 [M+H]+ |
| 1.086 | 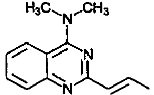 | 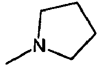 | 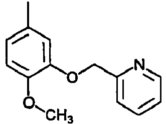 | 5/2HC l | MS·APCI(MeOH)-2 : 560[M+H]+ |
| 1.087 | 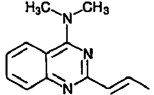 | 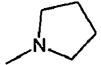 | 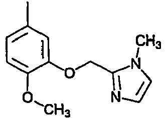 | 3HCl | MS · APCI(MeOH)-2 : 563[M+H]+ |
| 1.088 | 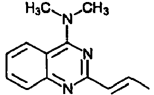 | 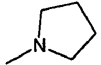 | 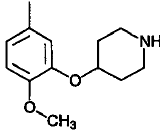 | 3HCl | MS · APCI(MeOH)-2 : 552[M+H]+ |
| 1.089 | 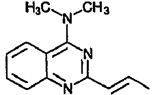 | 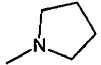 | 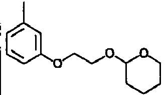 | free form | MS · APCI(MeOH)-2 : 567 [M+H]+ |
| 1.090 | 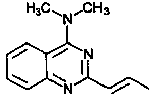 | 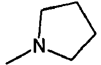 | 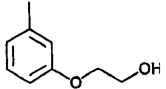 | 2HCl | MS · APCI(MeOH)-1: 483 [M+H]+ |
| 1.091 | 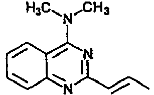 | 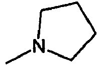 | 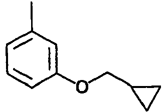 | 2HCl | MS·APCI(MeOH)-2 : 493[M+H]+ |
| 1.092 | 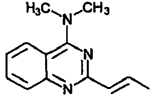 | 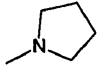 | 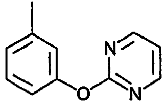 | 2HCl | MS-APCI(MeOH)-2: 517 [M+H]+ |
| 1.093 | 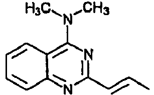 | 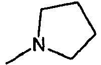 | 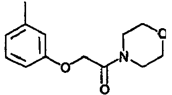 | 2HCl | MS·APCI(MeOH)-1: 566[M+H]+ |
| 1.094 | 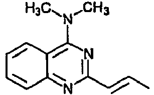 | 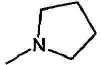 | 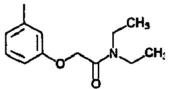 | 2HCl | MS · APCI(MeOH)-1: 552[M+H]+ |
| 1.095 | 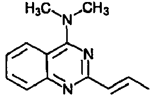 | 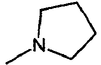 | 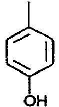 | 2HCl | MS·APCI(MeOH)-1: 439 [M+H]+ |
| 1.096 | 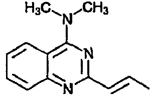 | 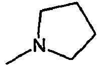 | 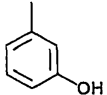 | 2HCl | MS·APCI(MeOH): 439[M+H]+ |
| 1.097 | 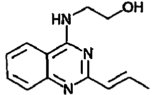 | 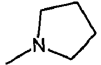 | 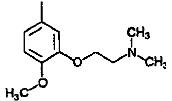 | free form | MS·APCI(MeOH): 556[M+H]+ |
| 1.098 | 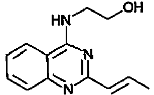 | 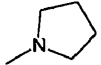 | 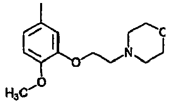 | free form | MS·APCI(MeOH): 598[M+H]+ |
| 1.099 | 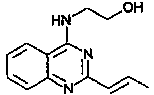 | 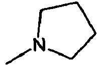 | 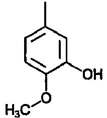 | 2HCl; 3H₂O | MS·APCI(MeOH): 485[M+H]+ |
| 1.100 | 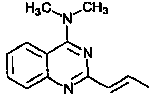 | 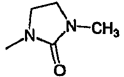 | 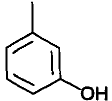 | free form | mp: 322-337°C |
| 1.101 | 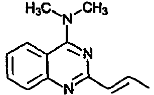 | 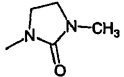 | 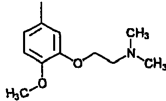 | free form | mp: 112-118°C |
| 1.102 | 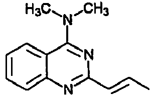 | 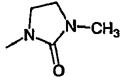 | 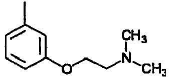 | free form | mp: 183-186°C |
| 1.103 | 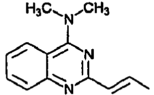 | 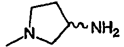 | 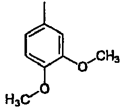 | 3HCl | mp: 251°C (Degradation) |
| 1.104 | 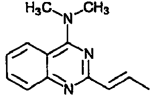 | 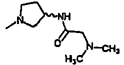 | 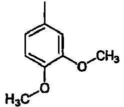 | free form | mp: 126-131°C |
| 1.105 | 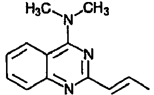 | 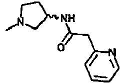 | 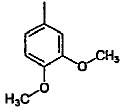 | free form | mp: 215-219°C |
| 1.106 | 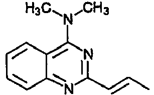 | 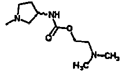 | 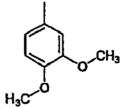 | free form | mp: 186-188°C |
| 1.107 | 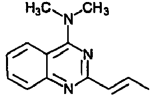 | 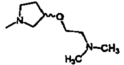 | 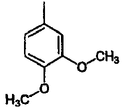 | 3HCl; 5H₂O | MS·APCI (10mM-AcONH₄/MeOH) : 570[M+H]+ |
| 1.108 | 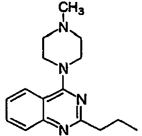 | 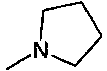 | 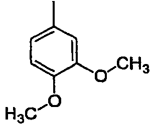 | 3HCl | MS·APCI (10mM-AcONH₄/MeOH)-1: 540[M+H]+ |

**Table 2**

| | | | | | |
|---|---|---|---|---|---|
| | 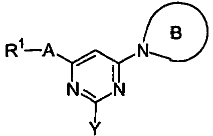 | | | | |

| Example No. | R¹-A- | 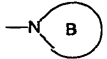 | -Y | Salt | Physical properties, etc. |
|---|---|---|---|---|---|
| 1.109 | 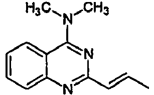 | 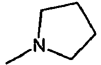 | 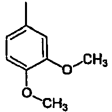 | 2HCl | MS · APCI(MeOH)-1: 483 [M+H]+ |

**Table 3**

| | | | | | |
|---|---|---|---|---|---|
| | 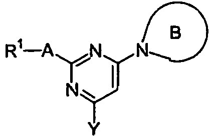 | | | | |

| Example No. | R¹-A- | 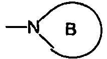 | -Y | Salt | Physical properties, etc. |
|---|---|---|---|---|---|
| 1.110 | 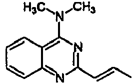 | 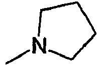 | 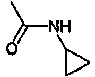 | free form | MS · APCI(MeOH)-2 : 430[M+H]+ |
| 1.111 | 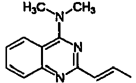 | 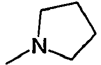 | 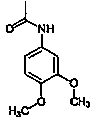 | free form | MS · APCI(MeOH) : 526[M+H]+ |
| 1.112 | 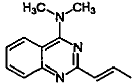 | 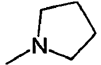 | 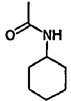 | free form | MS·APCI(MeOH): 472[M+H]+ |
| 1.113 | 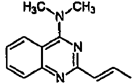 | 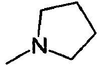 | 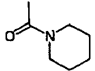 | free form | MS · APCI(MeOH) : 458[M+H]+ |
| 1.114 | 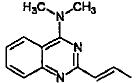 | 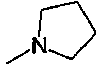 | 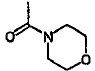 | free form | MS · APCI(MeOH) : 460[M+H]+ |
| 1.115 | 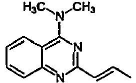 | 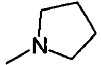 | 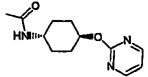 | free form | MS · APCI(MeOH)-2 : 566[M+H]+ |
| 1.116 | 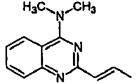 | 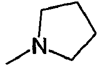 | 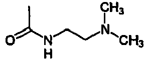 | 3HCl | MS · APCI(MeOH)-2 : 461 [M+H]+ |
| 1.117 | 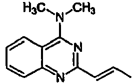 | 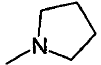 | 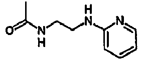 | 3HCl | MS · APCI (10mM-AcONH₄/MeOH)-1: 510[M+H]+ |
| 1.118 | 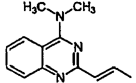 | 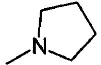 | 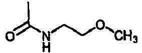 | 2HCl | MS·APCI (10mM-AcONH₄/MeOH)-1: 448[M+H]+ |
| 1.119 | 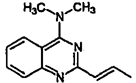 | 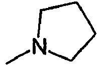 | 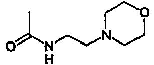 | 3HCl | MS·APCI (10mM-AcONH₄/MeOH)-1: 503[M+H]+ |
| 1.120 | 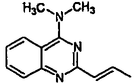 | 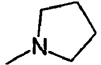 | 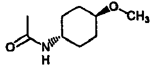 | 2HCl | MS·APCI (10mM-AcONH₄/MeOH)-1: 502[M+H]+ |
| 1.121 | 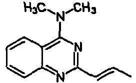 | 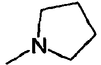 | 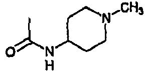 | 3HCl | MS · APCI (10mM-AcONH₄/MeOH)-1: 487[M+H]+ |
| 1.122 | 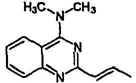 | 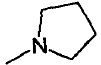 | 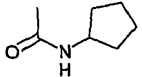 | 2HCl | MS · APCI (10mM-AcONH₄/MeOH)-1: 458[M+H]+ |
| 1.123 | 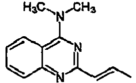 | 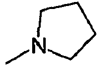 | 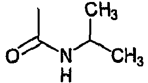 2HCl | 2HCl | MS · APCI (10mM-AcONH₄/MeOH)-1: 432[M+H]+ |
| 1.124 | 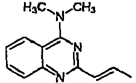 | 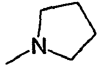 | 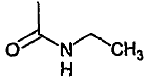 | 2HCl | MS · APCI (10mM-AcONH₄/MeOH)-1: 418 [M+H]+ |
| 1.125 | 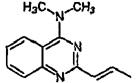 | 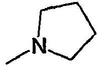 | 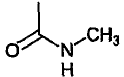 | 2HCl | MS · APCI (10mM-AcONH₄/MeOH)-1: 404[M+H]+ |
| 1.126 | 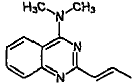 | 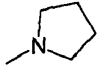 | 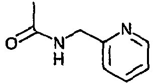 | 3HCl | MS·APCI (10mM-AcONH₄/MeOH)-1: 481[M+H]+ |
| 1.127 | 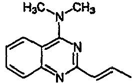 | 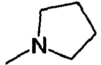 | 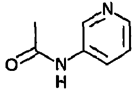 | 3HCl | MS· APCI(10mM-AcONH₄/MeO H)-1:467[M+H]+ |
| 1.128 | 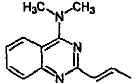 | 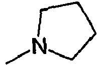 | 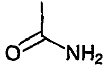 | 2HCl | MS·APCI(MeOH)-2: 390[M+H]+ |
| 1.129 | 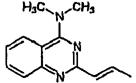 | 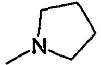 | 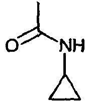 | 2HCl | MS·APCI (10mM-AcONH₄/MeOH)-1: 430[M+H]+ |
| 1.130 | 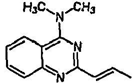 | 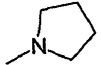 | 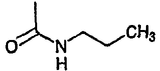 | 2HCl | MS·APCI (10mM-AcONH₄/MeOH)-1: 432[M+H]+ |
| 1.131 | 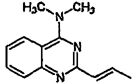 | 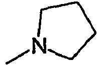 | 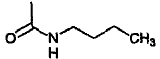 | 2HCl | MS · APCI (10mM-AcONH₄/MeOH)-1: 446 [M+H] + |
| 1.132 | 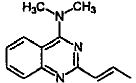 | 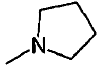 | 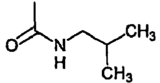 | 2HCl | MS · APCI(MeOH)-1: 446[M+H]+ |
| 1.133 | 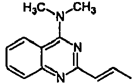 | 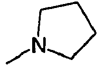 | 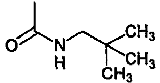 | 2HCl | MS·APCI(MeOH)-1: 460[M+H]+ |
| 1.134 | 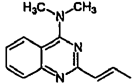 | 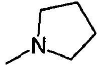 | 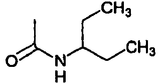 | 2HCl | MS · APCI(MeOH)-1: 460[M+H]+ |
| 1.135 | 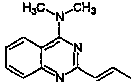 | 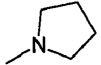 | 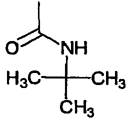 | 2HCl | MS · APCI (10mM-AcONH₄/MeOH)-1: 446[M+NH4]+ |
| 1.136 | 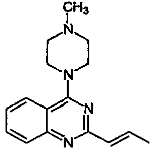 | 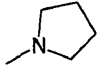 | 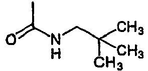 | 3HCl | MS·APCI(MeOH)-1: 515[M+H]+ |
| 1.137 | 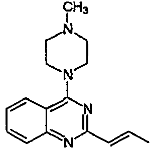 | 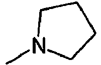 | 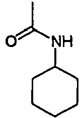 | 3HCl | MS·APCI(MeOH)-1: 527[M+H]+ |
| 1.138 | 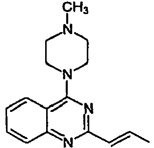 | 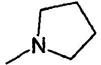 | 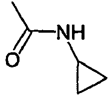 | 3HCl | MS · APCI(MeOH)-1: 485 [M+H]+ |
| 1.139 | 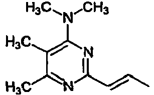 | 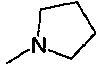 | 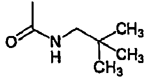 | 2HCl | MS · APCI(MeOH)-1: 438 [M+H]+ |
| 1.140 | 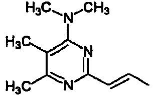 | 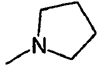 | 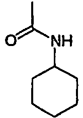 | 2HCl | MS·APCI(MeOH)-1: 450 [M+H]+ |
| 1.141 | 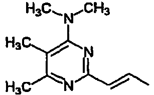 | 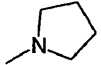 | 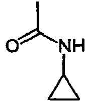 | 2HCl | MS·APCI(MeOH)-1: 408 [M+H]+ |
| 1.142 | 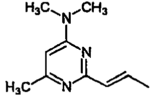 | 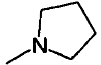 | 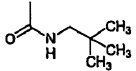 | 2HCl | MS·APCI(MeOH)-1: 424[M+H]+ |
| 1.143 | 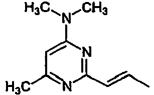 | 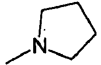 | 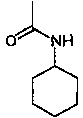 | 2HCl | MS·APCI(MeOH)-1: 436 [M+H]+ |
| 1.144 | 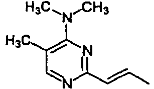 | 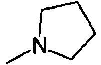 | 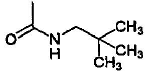 | 2HCl | MS·APCI(MeOH)-1: 424[M+H]+ |
| 1.145 | 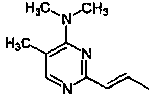 | 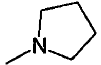 | 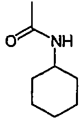 | 2HCl | MS·APCI(MeOH)-1: 436[M+H]+ |
| 1.146 | 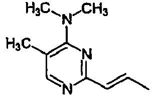 | 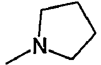 | 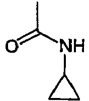 | 2HCl | MS · APCI(MeOH)-1: 394[M+H]+ |
| 1.147 | 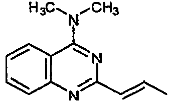 | 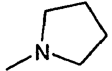 | 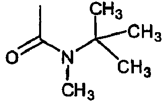 | 2HCl | MS·APCI:460 (M+H) |
| 1.148 | 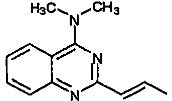 | 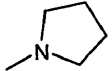 | 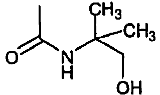 | 2HCl | MS·APCI:462 (M+H) |
| 1.149 | 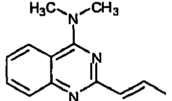 | 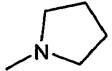 | 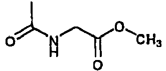 | 2HCl | MS·APCI:462 (M+H) |
| 1.150 | 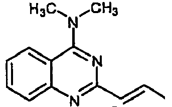 | 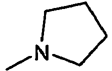 | 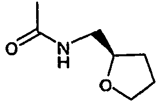 | 2HCl | MS·APCI:474 (M+H) |
| 1.151 | 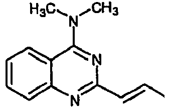 | 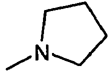 | 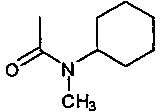 | 2HCl | MS·APCI:480 (M+H) |
| 1.152 | 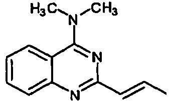 | 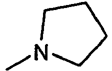 | 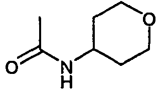 | 2HCl | MS · APCI : 474 (M+H) |
| 1.153 | 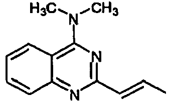 | 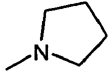 | 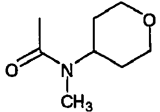 | 2HCl | MS·APCI:488 (M+H) |
| 1.154 | 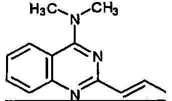 | 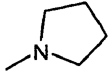 | 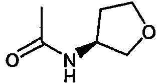 | 2HCl | MS·APCI:460 (M+H) |
| 1.155 | 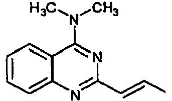 | 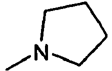 | 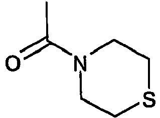 | 2HCl | MS·APCI:476 (M+H) |
| 1.156 | 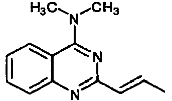 | 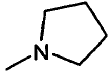 | 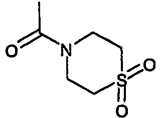 | 2HCl | MS·APCI:508 (M+H) |
| 1.157 | 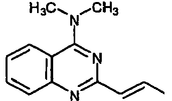 | 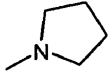 | 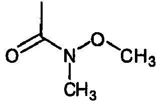 | 2HCl | MS·APCI:434 (M+H) |
| 1.158 | 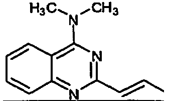 | 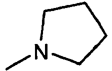 | 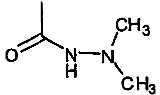 | 3HCl | MS·APCI:433 (M+H) |
| 1.159 | 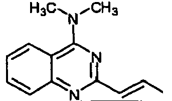 | 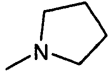 | 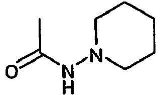 | 3HCl | MS·APCI:473 (M+H) |
| 1.160 | 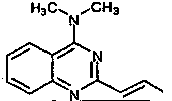 | 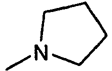 | 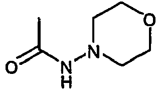 | 2HCl | MS·APCI:475 (M+H) |
| 1.161 | 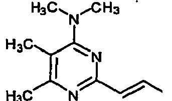 | 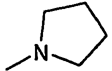 | 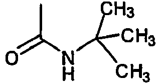 | 2HCl | MS·APCI:424 (M+H) |
| 1.162 | 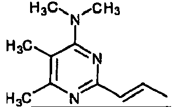 | 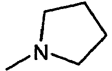 | 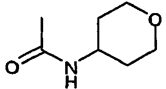 | 2HCl | MS·APCI:452 (M+H) |
| 1.163 | 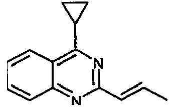 | 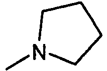 | 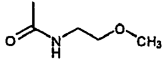 | 2HCl | MS·APCI:445 (M+H) |
| 1.164 | 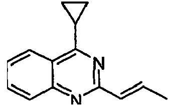 | 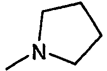 | 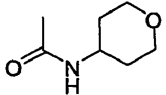 | 2HCl | MS·APCI:471 (M+H) |
| 1.165 | 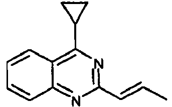 | 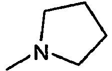 | 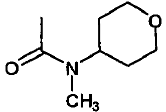 | 2HCl | MS·APCI:485 (M+H) |
| 1.166 | 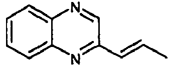 | 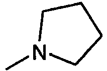 | 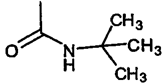 | HCl | MS·APCI:403 (M+H) |
| 1.167 | 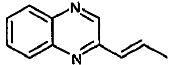 | 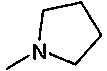 | 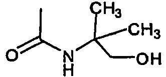 | HCl | MS·APCI:419 (M+H) |
| 1.168 | 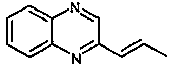 | 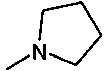 | 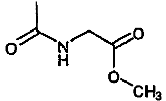 | HCl | MS·APCI:419 (M+H) |
| 1.169 | 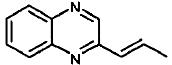 | 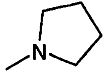 | 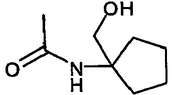 | free form | MS·APCI:445 (M+H) |
| 1.170 | 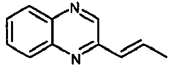 | 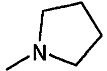 | 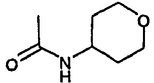 | free form | MS·APCI:431 (M+H) |
| 1.171 | 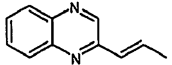 | 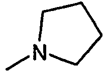 | 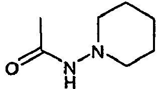 | free form | MS·APCI:430 (M+H) |
| 1.172 | 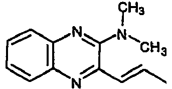 | 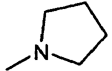 | 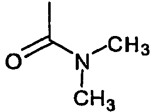 | 2HCl | MS·APCI:418 (M+H) |
| 1.173 | 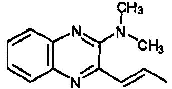 | 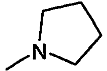 | 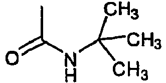 | 2HCl | MS·APCI:446 (M+H) |
| 1.174 | 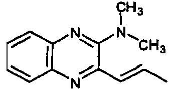 | 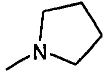 | 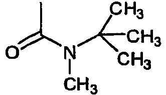 | 2HCl | MS·APCI:460 (M+H) |
| 1.175 | 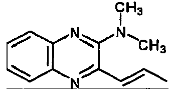 | 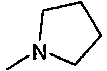 | 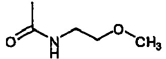 | 2HCl | MS·APCI:448 (M+H) |
| 1.176 | 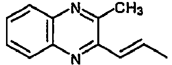 | 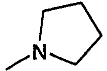 | 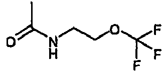 | 3/2HCl | MS·APCI:473 (M+H) |
| 1.177 | 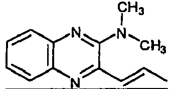 | 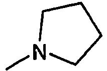 | 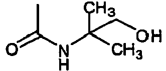 | 2HCl | MS·APCI:462 (M+H) |
| 1.178 | 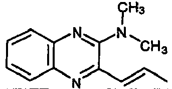 | 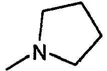 | 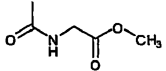 | 2HCl | MS·APCI:462 (M+H) |
| 1.179 | 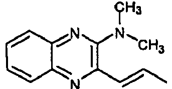 | 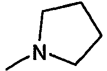 | 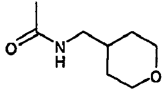 | 2HCl | MS·APCI:488 (M+H) |
| 1.180 | 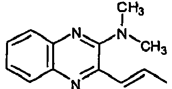 | 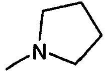 | 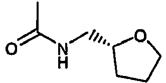 | 2HCl | MS·APCI:474 (M+H) |
| 1.181 | 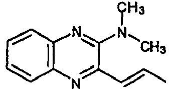 | 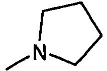 | 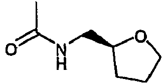 | 2HCl | MS·APCI:474 (M+H) |
| 1.182 | 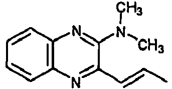 | 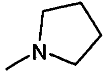 | 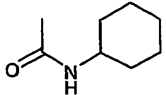 | 2HCl | MS·APCI:472 (M+H) |
| 1.183 | 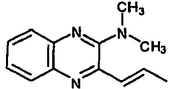 | 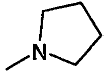 | 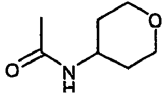 | 2HCl | MS·APCI: 474 (M+H) |
| 1.184 | 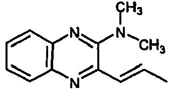 | 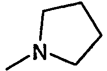 | 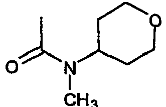 | 2HCl | MS·APCI:488 (M+H) |
| 1.185 | 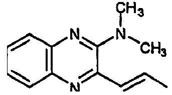 | 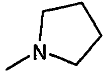 | 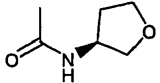 | 2HCl | MS·APCI:460 (M+H) |
| 1.186 | 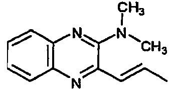 | 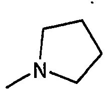 | 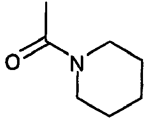 | 2HCl | MS·APCI:458 (M+H) |
| 1.187 | 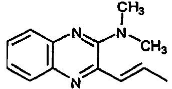 | 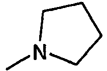 | 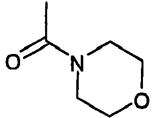 | 2HCl | MS·APCI:460 (M+H) |
| 1.188 | 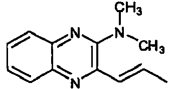 | 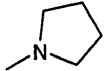 | 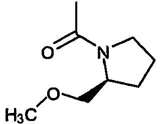 | 2HCl | MS·APCI:488 (M+H) |
| 1.189 | 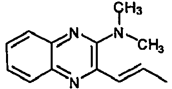 | 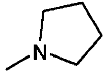 | 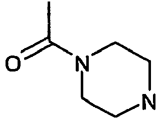 | 3HCl | MS·APCI:405 (M+H) |
| 1.190 | 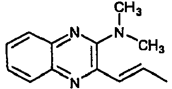 | 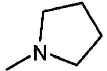 | 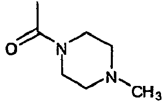 | 3HCl | MS·APCI:488 (M+H) |
| 1.191 | 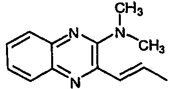 | 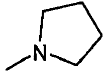 | 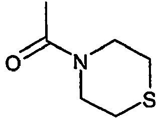 | 2HCl | MS·APCI:476 (M+H) |
| 1.192 | 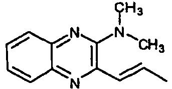 | 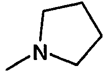 | 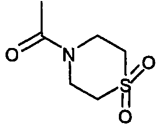 | 2HCl | MS·APCI:508 (M+H) |
| 1.193 | 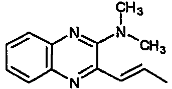 | 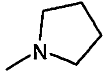 | 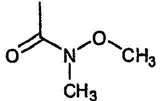 | 2HCl | MS·APCI:434 (M+H) |
| 1.194 | 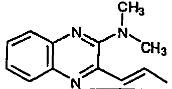 | 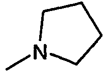 | 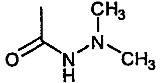 | 3HCl | MS·APCI:433 (M+H) |
| 1.195 | 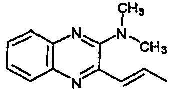 | 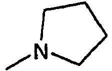 | 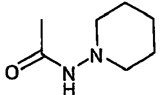 | 3HCl | MS·APCI:473 (M+H) |
| 1.196 | 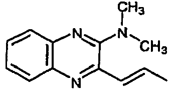 | 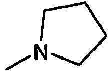 | 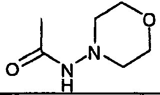 | 3HCl | MS·APCI:475 (M+H) |
| 1.197 | 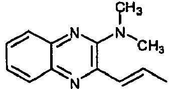 | 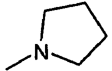 | 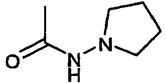 | 3HCl | MS·APCI:459 (M+H) |
| 1.198 | 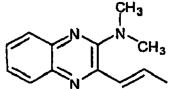 | 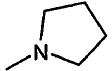 | 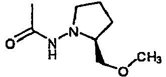 | 3HCl | MS·APCI:503 (M+H) |
| 1.199 | 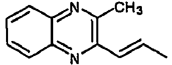 | 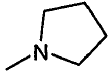 | 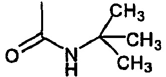 | 3/2HCl | MS·APCI:417 (M+H) |
| 1.200 | 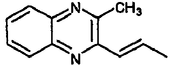 | 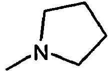 | 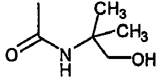 | 2HCl | MS·APCI:433 (M+H) |
| 1.201 | 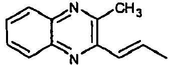 | 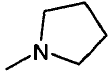 | 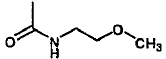 | 2HCl | MS·APCI:419 (M+H) |
| 1.202 | 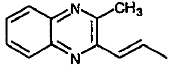 | 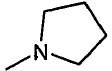 | 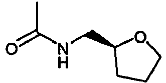 | 2HCl | MS·APCI:445 (M+H) |
| 1.203 | 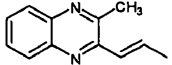 | 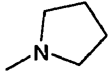 | 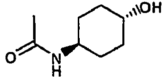 | 2HCl | MS·APCI:459 (M+H) |
| 1.204 | 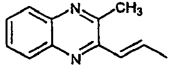 | 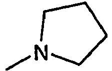 | 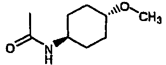 | HCl | MS·APCI:473 (M+H) |
| 1.205 | 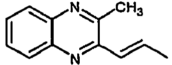 | 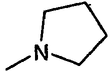 | 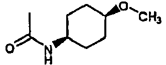 | 2HCl | MS·APCI:473 (M+H) |
| 1.206 | 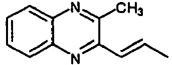 | 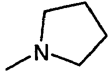 | 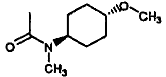 | 2HCl | MS·APCI: 487 (M+H) |
| 1.207 | 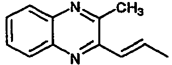 | 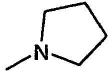 | 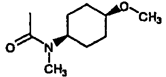 | 2HCl | MS·APCI:487(M+H) |
| 1.208 | 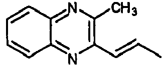 | 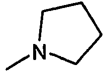 | 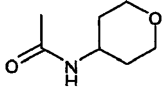 | 2HCl, HCl | MS·APCI:445 (M+H) |
| 1.209 | 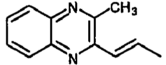 | 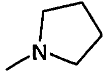 | 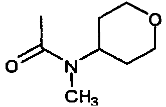 | 2HCl | MS·APCI:459 (M+H) |
| 1.210 | 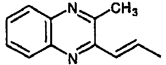 | 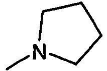 | 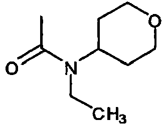 | 3/2HCl | MS·APCI:473 (M+H) |
| 1.211 | 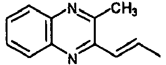 | 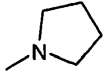 | 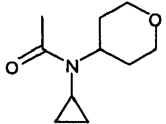 | 2HCl | MS·APCI:485 (M+H) |
| 1.212 | 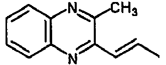 | 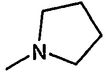 | 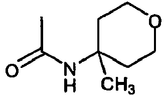 | 2HCl | MS·APCI:459 (M+H) |
| 1.213 | 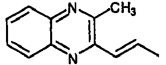 | 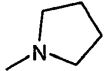 | 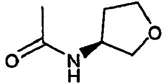 | 2HCl | MS·APCI:431 (M+H) |
| 1.214 | 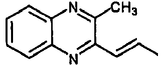 | 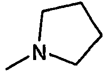 | 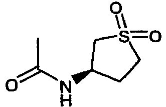 | 2HCl | MS·APCI:479 (M+H) |
| 1.215 | 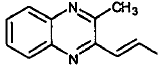 | 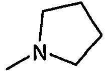 | 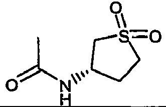 | 2HCl | MS·APCI:479 (M+H) |
| 1.216 | 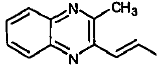 | 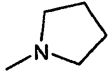 | 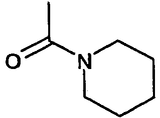 | 2HCl | MS·APCI:429 (M+H) |
| 1.217 | 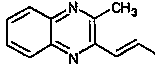 | 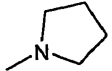 | 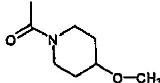 | 2HCl | MS·APCI:459 (M+H) |
| 1.218 | 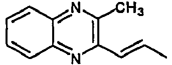 | 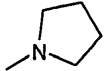 | 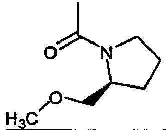 | 2HCl | MS·APCI:459 (M+H) |
| 1.219 | 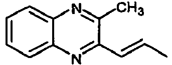 | 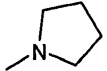 | 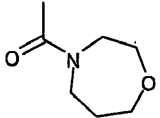 | 2HCl | MS·APCI:445 (M+H) |
| 1.220 | 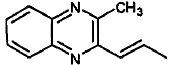 | 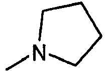 | 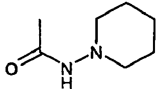 | 2HCl | MS·APCI:444 (M+H) |
| 1.221 | 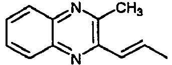 | 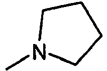 | 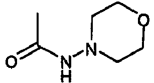 | HCl | MS·APCI:446 (M+H) |
| 1.222 | 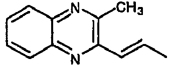 | 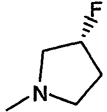 | 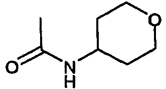 | 3/2HCl | MS·APCI: 463 (M+H) |
| 1.223 | 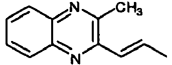 | 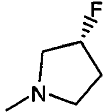 | 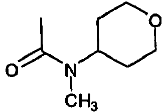 | 2HCl | MS·APCI:477 (M+H) |
| 1.224 | 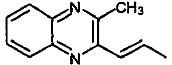 | 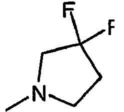 | 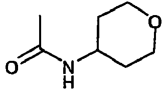 | 2HCl | MS·APCI:480 (M+H) |
| 1.225 | 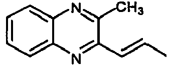 | 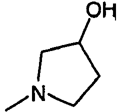 | 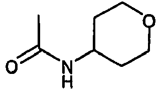 | 3/2HCl | MS·APCI:461 (M+H) |
| 1.226 | 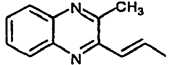 | 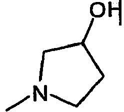 | 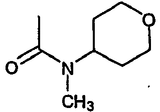 | 3/2HCl | MS·APCI:475 (M+H) |
| 1.227 | 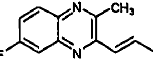 | 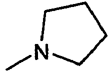 | 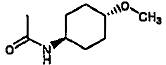 | HCl | MS·APCI: 491 (M+H) |
| 1.228 | 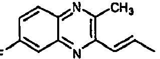 | 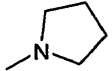 | 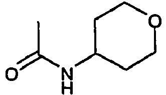 | 3/2HCl | MS·APCI: 463 (M+H) |
| 1.229 | 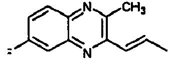 | 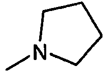 | 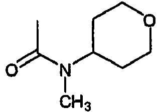 | 2HCl | MS·APCI:477 (M+H) |
| 1.230 | 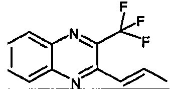 | 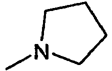 | 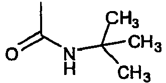 | HCl | MS·APCI:471 (M+H) |
| 1.231 | 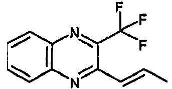 | 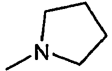 | 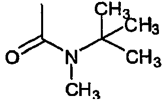 | HCl | MS·APCI:485 (M+H) |
| 1.232 | 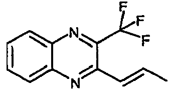 | 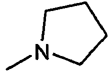 | 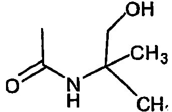 | HCl | MS·APCI:487 (M+H) |
| 1.233 | 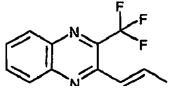 | 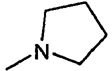 | 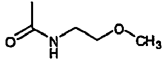 | HCl | MS·APCI:473 (M+H) |
| 1.234 | 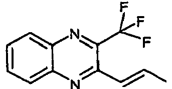 | 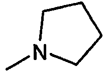 | 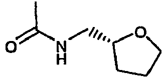 | HCl | MS·APCI:499 (M+H) |
| 1.235 | 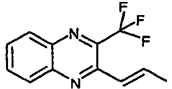 | 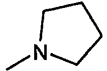 | 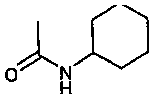 | HCl | MS·APCI:497 (M+H) |
| 1.236 | 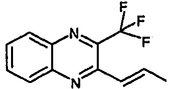 | 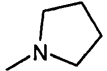 | 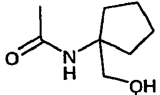 | HCl | MS·APCI:513 (M+H) |
| 1.237 | 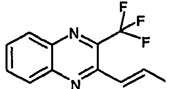 | 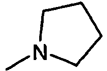 | 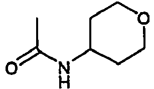 | HCl | MS·APCI:499 (M+H) |
| 1.238 | 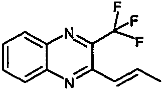 | 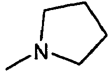 | 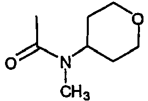 | HCl | MS·APCI:513 (M+H) |
| 1.239 | 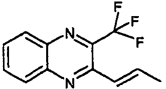 | 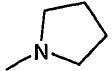 | 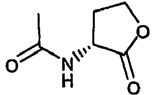 | HCl | MS·APCI:499 (M+H) |
| 1.240 | 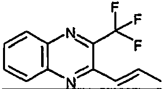 | 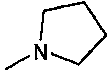 | 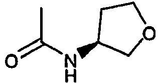 | HCl | MS·APCI:485 (M+H) |
| 1.241 | 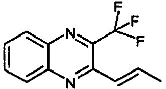 | 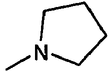 | 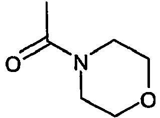 | HCl | MS·APCI:485 (M+H) |
| 1.242 | 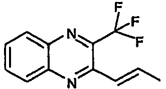 | 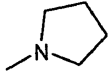 | 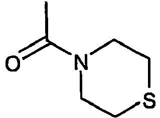 | HCl | MS·APCI:501 (M+H) |
| 1.243 | 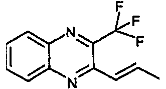 | 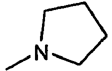 | 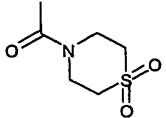 | 2HCl | MS·APCI:533 (M+H) |
| 1.244 | 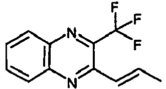 | 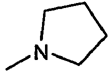 | 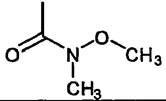 | HCl | MS·APCI:459 (M+H) |
| 1.245 | 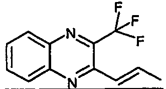 | 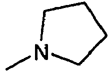 | 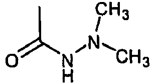 | 2HCl | MS·APCI:458 (M+H) |
| 1.246 | 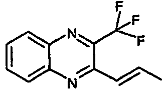 | 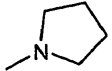 | 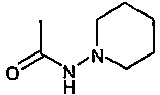 | 2HCl | MS·APCI:498 (M+H) |
| 1.247 | 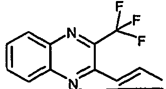 | 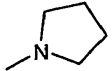 | 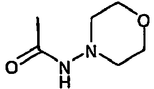 | 2HCl | MS·APCI:500 (M+H) |
| 1.248 | 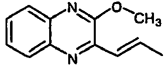 | 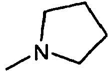 | 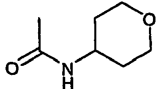 | HCl | MS·APCI:461 (M+H) |
| 1.249 | 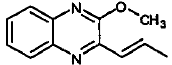 | 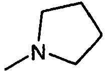 | 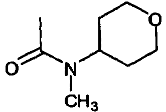 | HCl | MS·APCI:475 (M+H) |
| 1.250 | 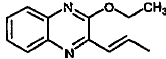 | 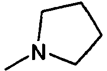 | 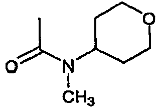 | HCl | MS·APCI:489 (M+H) |
| 1.251 | 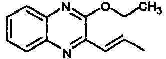 | 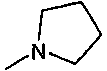 | 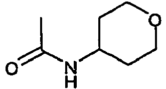 | HCl | MS·APCI:475 (M+H) |
| 1.252 | 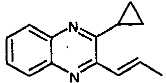 | 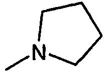 | 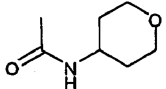 | HCl | MS·APCI:471 (M+H) |
| 1.253 | 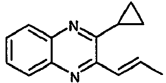 | 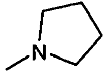 | 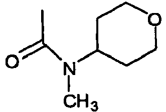 | HCl | MS·APCI:485 (M+H) |
| 1.254 | 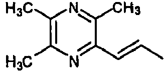 | 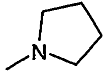 | 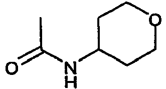 | 2HCl | MS·APCI:423 (M+H) |
| 1.255 | 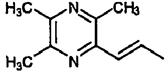 | 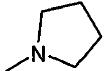 | 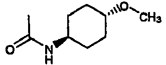 | 2HCl | MS·APCI:451 (M+H) |
| 1.256 | 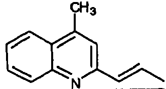 | 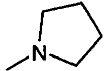 | 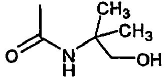 | 2HCl | MS·APCI:432 (M+H) |
| 1.257 | 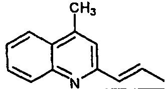 | 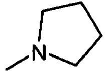 | 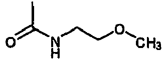 | 2HCl | MS·APCI:418 (M+H) |
| 1.258 | 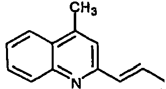 | 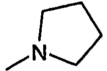 | 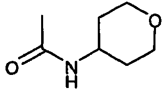 | 2HCl | MS·APCI:444 (M+H) |
| 1.259 | 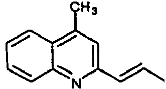 | 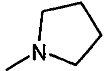 | 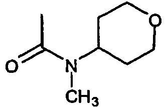 | 2HCl | MS·APCI:458 (M+H) |
| 1.260 | 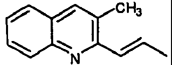 | 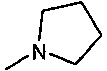 | 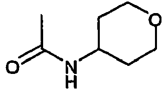 | 2HCl | MS·APCI:444(M+H) |
| 1.261 | 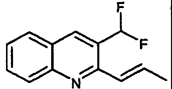 | 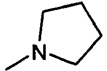 | 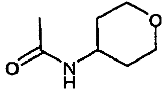 | HCl | MS·APCI:480(M+H) |
| 1.262 | 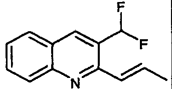 | 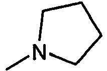 | 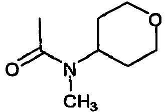 | HCl | MS·APCI:494 (M+H) |
| 1.263 | 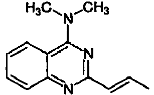 | 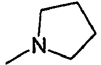 | 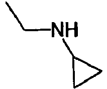 | 3HCl | MS · APCI(MeOH)-1: 416[M+H]+ |
| 1.264 | 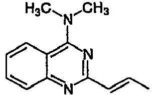 | 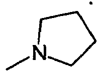 | 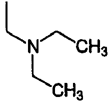 | 3HCl | MS · APCI(MeOH)-1: 432[M+H]+ |
| 1.265 | 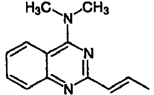 | 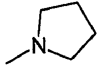 | 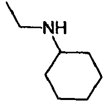 | 3HCl | MS · APCI(MeOH)-1: 458[M+H]+ |
| 1.266 | 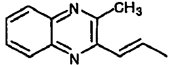 | 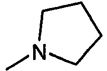 | 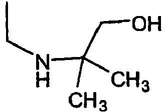 | 2HCl | MS·APCI:419 (M+H) |
| 1.267 | 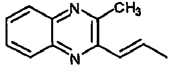 | 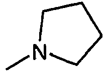 | 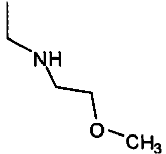 | 5/2HCl | MS·APCI: 405 (M+H) |
| 1.268 | 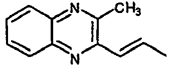 | 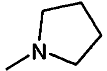 | 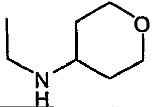 | 2HCl | MS·APCI:431 (M+H) |
| 1.269 | 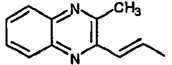 | 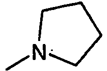 | 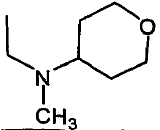 | 2HCl | MS·APCI:445 (M+H) |
| 1.270 | 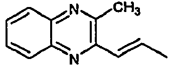 | 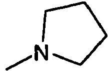 | 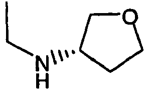 | 5/2HCl | MS·APCI:417 (M+H) |
| 1.271 | 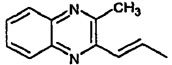 | 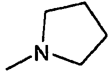 | 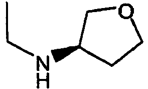 | 5/2HCl | MS·APCI:417 (M+H) |
| 1.272 | 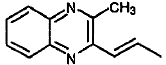 | 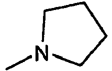 | 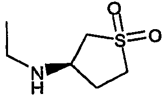 | 5/2HCl | MS·APCI: 465 (M+H) |
| 1.273 | 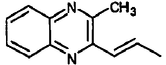 | 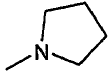 | 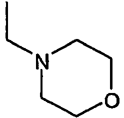 | 5/2HCl | MS·APCI:417 (M+H) |

**Table 4**

| | | | | | |
|---|---|---|---|---|---|
| | 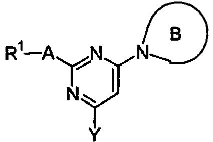 | | | | |

| Example No. | R¹-A- | 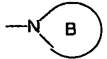 | -Y | Salt | Physical properties, etc. |
|---|---|---|---|---|---|
| 2.001 | 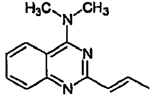 | 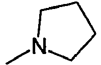 | 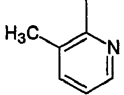 | 2HCl | MS · APCI(MeOH)-1: 438[M+H]+ |
| 2.002 | 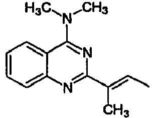 | 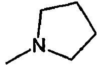 | 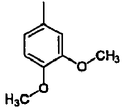 | 2HCl | MS · APCI(MeOH)-1: 497[M+H]+ |
| 2.003 | 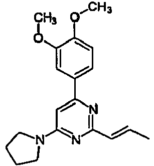 | 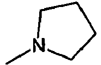 | 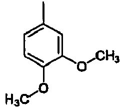 | 2HCl | MS · APCI(MeOH)-1: 595 [M+H]+ |
| 2.004 | 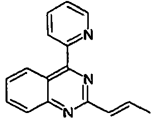 | 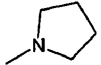 | 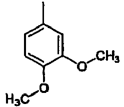 | 2HCl | MS·APCI(MeOH)-1: 517[M+H]+ |
| 2.005 | 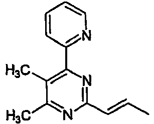 | 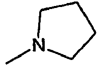 | 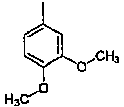 | 3HCl | MS · APCI(MeOH)-1: 495[M+H]+ |
| 2.006 | 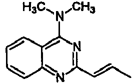 | 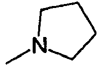 | 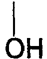 | 2HCl | MS·APCI(MeOH)-1: 363[M+H]+ |
| 2.007 | 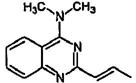 | 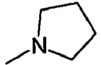 | 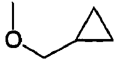 | 2HCl | MS·APCI(MeOH)-2: 417[M+H]+ |
| 2.008 | 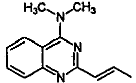 | 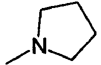 | 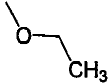 | 2HCl | MS·APCI(MeOH)-1: 391[M+H]+ |
| 2.009 | 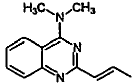 | 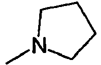 | 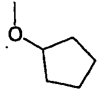 | 2HCl | MS · APCI(MeOH)-2 : 431[M+H]+ |
| 2.010 | 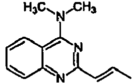 | 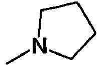 | 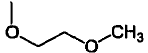 | 2HCl | MS·APCI(MeOH)-1: 421[M+H]+ |
| 2.011 | 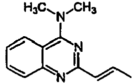 | 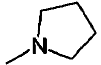 | 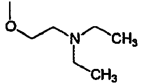 | 2HCl | MS · APCI(MeOH)-2 : 462[M+H]+ |
| 2.012 | 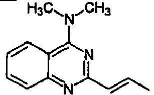 | 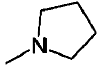 | 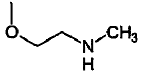 | 2HCl | MS · APCI(MeOH)-1: 419[M+H]+ |
| 2.013 | 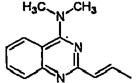 | 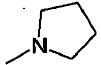 | 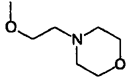 | 2HCl | MS·APCI(MeOH)-1: 476[M+H]+ |
| 2.014 | 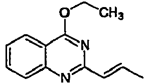 | 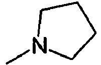 | 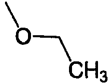 | 2HCl | MS·APCI(MeOH)-1: 392[M+H]+ |
| 2.015 | 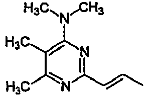 | 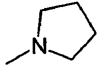 | 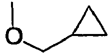 | 2HCl | MS · APCI(MeOH)-1: 395[M+H]+ |
| 2.016 | 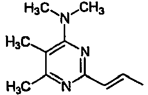 | 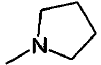 | 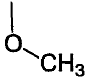 | 2HCl | MS · APCI(MeOH)-1: 355[M+H]+ |
| 2.017 | 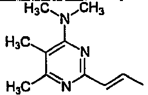 | 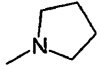 | 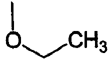 | 2HCl | MS · APCI(MeOH)-1: 369[M+H]+ |
| 2.018 | 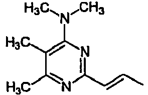 | 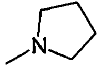 | 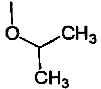 | 2HCl | MS · APCI(MeOH)-1: 383 [M+H]+ |
| 2.019 | 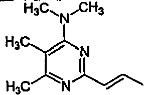 | 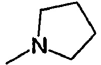 | 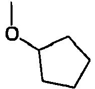 | 2HCl | MS · APCI(MeOH)-1: 409[M+H]+ |
| 2.020 | 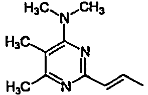 | 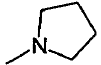 | 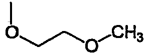 | 2HCl | MS·APCI(MeOH)-1: 399 [M+H]+ |
| 2.021 | 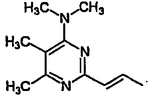 | 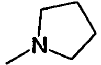 | 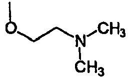 | 3HCl | MS·APCI(MeOH)-1: 412[M+H]+ |
| 2.022 | 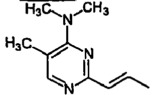 | 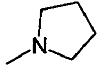 | 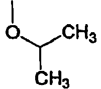 | 2HCl | MS · APCI(MeOH)-1: 369[M+H]+ |
| 2.023 | 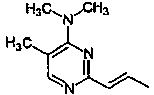 | 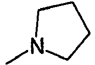 | 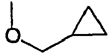 | 2HCl | MS · APCI(MeOH)-1: 381 [M+H]+ |
| 2.024 | 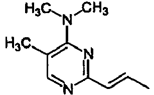 | 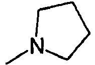 | 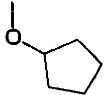 | 2HCl | MS · APCI(MeOH)-1: 395 [M+H]+ |
| 2.025 | 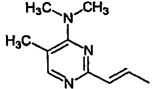 | 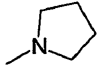 | 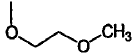 | 2HCl | MS · APCI(MeOH)-1: 385[M+H]+ |
| 2.026 | 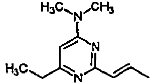 | 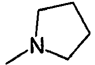 | 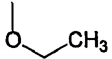 | 2HCl | MS·APCI(MeOH)-1: 369[M+H]+ |
| 2.027 | 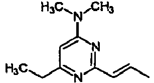 | 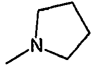 | 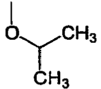 | 2HCl | MS·APCI(MeOH)-1: 383 [M+H]+ |
| 2.028 | 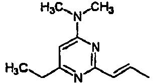 | 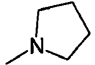 | 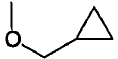 | 2HCl | MS·APCI(MeOH)-1: 395[M+H]+ |
| 2.029 | 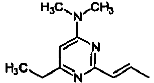 | 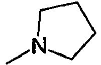 | 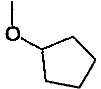 | 2HCl | MS · APCI(MeOH)-1: 409 [M+H]+ |
| 2.030 | 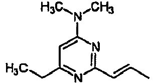 | 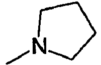 | 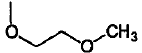 | 2HCl | MS · APCI(MeOH)-1: 399[M+H]+ |
| 2.031 | 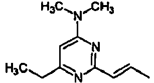 | 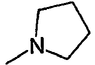 | 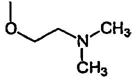 | 3HCl | MS · APCI(MeOH)-1: 412[M+H]+ |
| 2.032 | 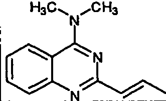 | 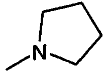 | 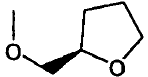 | 2HCl | MS·APCI : 447 (M+H) |
| 2.033 | 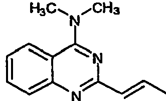 | 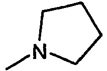 | 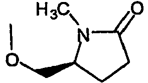 | 2HCl | MS·APCI:472 (M+H) |
| 2.034 | 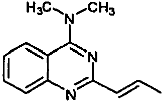 | 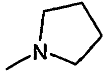 | 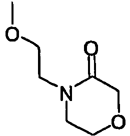 | 2HCl | MS·APCI:490 (M+H) |
| 2.035 | 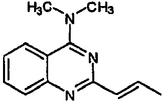 | 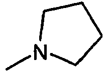 | 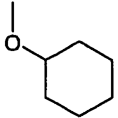 | 2HCl | MS·APCI:445 (M+H) |
| 2.036 | 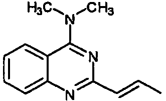 | 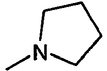 | 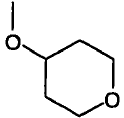 | 2HCl | MS·APCI:447 (M+H) |
| 2.037 | 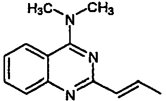 | 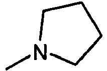 | 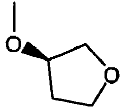 | 2HCl | MS·APCI:433 (M+H) |
| 2.038 | 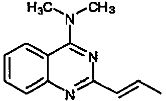 | 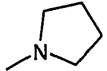 | 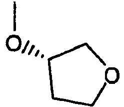 | 2HCl | MS·APCI:433 (M+H) |
| 2.039 | 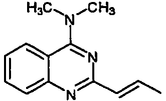 | 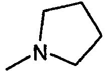 | 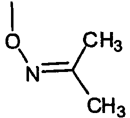 | 2HCl | MS·APCI:418 (M+H) |
| 2.040 | 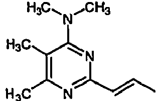 | 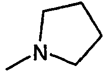 | 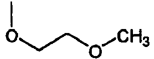 | HCl | MS·APCI:399 (M+H) |
| 2.041 | 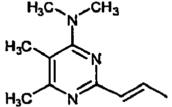 | 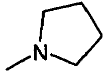 | 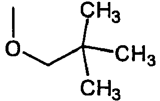 | 2HCl | MS·APCI:411 (M+H) |
| 2.042 | 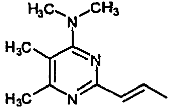 | 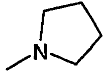 | 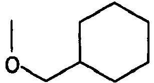 | 2HCl | MS·APCI:437 (M+H) |
| 2.043 | 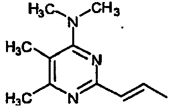 | 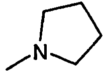 | 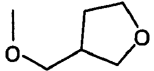 | 2HCl | MS·APCI:425 (M+H) |
| 2.044 | 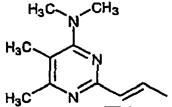 | 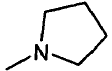 | 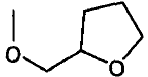 | 2HCl | MS•APCI: 425 (M+H) |
| 2.045 | 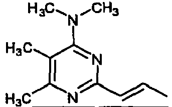 | 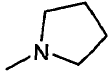 | 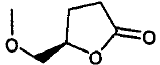 | HCl | MS•APCI: 411 (M+H) |
| 2.046 | 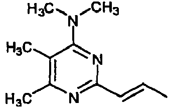 | 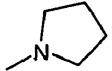 | 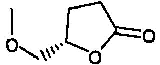 | 2HCl | MS•APCI: 439 (M+H) |
| 2.047 | 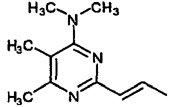 | 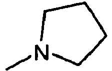 | 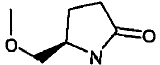 | HCl | MS•APCI: 438 (M+H) |
| 2.048 | 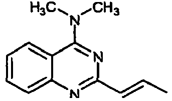 | 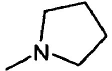 | 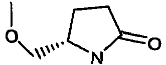 | 2HCl | MS •APCI: 460 (M+H) |
| 2.049 | 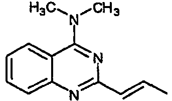 | 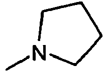 | 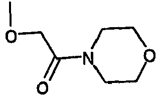 | 2HCl | MS•APCI: 490 (M+H) |
| 2.050 | 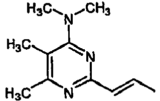 | 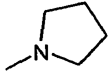 | 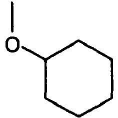 | 2HCl | MS•APCI: 423 (M+H) |
| 2.051 | 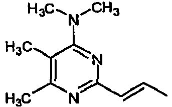 | 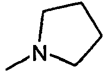 | 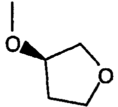 | 2HCl | MS•APCI: 411 (M+H) |
| 2.052 | 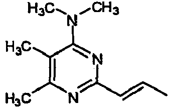 | 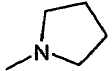 | 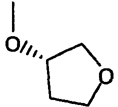 | 2HCl | MS•APCI: 411 (M+H) |
| 2.053 | 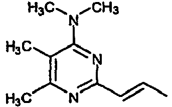 | 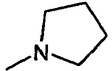 | 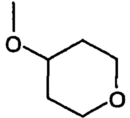 | 2HCl | MS•APCI: 425 (M+H) |
| 2.054 | 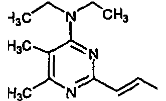 | 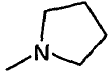 | 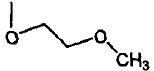 | 2HCl | MS•APCI: 427 (M+H) |
| 2.055 | 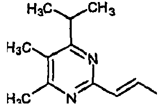 | 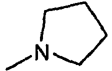 | 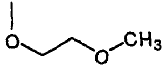 | 2HCl | MS•APCI: 398 (M+H) |
| 2.056 | 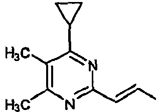 | 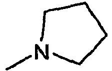 | 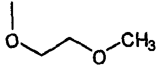 | 2HCl | MS•APCI: 396(M+H) |
| 2.057 | 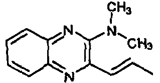 | 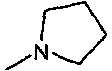 | 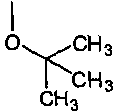 | free form | MS•APCI: 419 (M+H) |
| 2.058 | 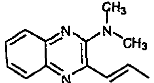 | 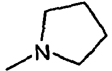 | 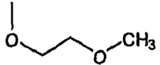 | 2HCl | MS•APCI: 421 (M+H) |
| 2.059 | 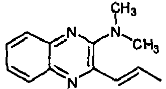 | 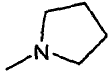 | 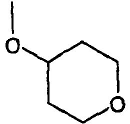 | 2HCl | MS•APCI: 447 (M+H) |
| 2.060 | 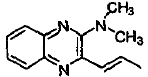 | 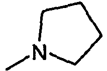 | 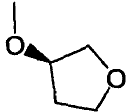 | 2HCl | MS•APCI: 433 (M+H) |
| 2.061 | 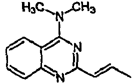 | 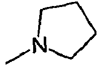 | 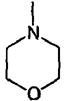 | 2HCl | MS•APCI(MeOH)-1: 432[M+H]+ |
| 2.062 | 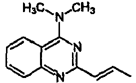 | 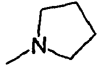 | 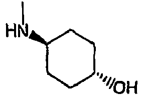 | 2HCl | MS•APCI(MeOH)-2: 460[M+H]+ |
| 2.063 | 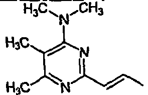 | 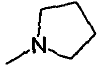 | 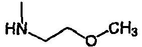 | 2HCl | MS•APCI(MeOH)-1: 398[M+H]+ |

**Table 5**

| | | | | | |
|---|---|---|---|---|---|
| | 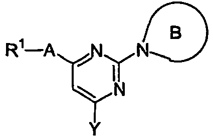 | | | | |

| Example No. | R¹-A- | 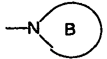 | -Y | Salt | Physical properties, etc. |
|---|---|---|---|---|---|
| 3.001 | 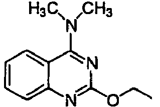 | 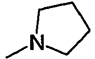 | 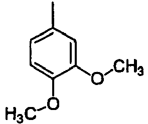 | 2HCl | MS•APCI(MeOH)-2 : 487[M+H]+ |
| 3.002 | 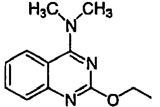 | 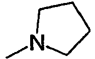 | 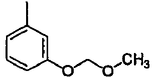 | free form | MS•APCI(MeOH) : 487[M+H]+ |
| 3.003 | 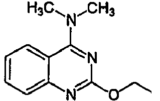 | 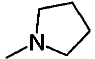 | 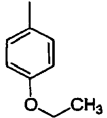 | 2HCl | MS•APCI(MeOH)-1: 471 [M+H]+ |
| 3.004 | 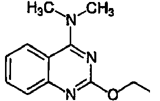 | 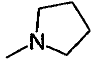 | 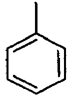 | 2HCl | MS•APCI(MeOH)-1: 427[M+H]+ |
| 3.005 | 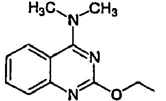 | 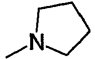 | 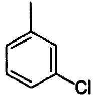 | 2HCl | MS•APCI(MeOH)-1: 461/463 [M+H]+ |
| 3.006 | 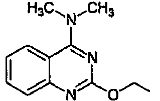 | 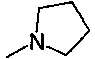 | 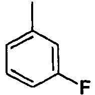 | 2HCl | MS•APCI (10mM-AcONH₄/MeOH)-1: 445[M+H]+ |
| 3.007 | 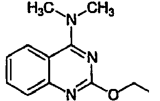 | 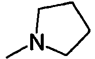 | 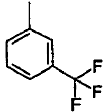 | 2HCl | MS•APCI: (10mM-AcONH₄/MeOH)-1: 495[M+H]+ |
| 3.008 | 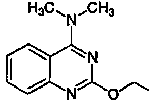 | 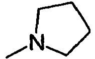 | 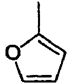 | 2HCl | MS•APCI(MeOH)-1: 417[M+H]+ |
| 3.009 | 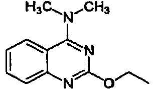 | 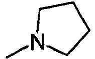 | 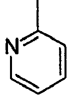 | 2HCl | MS•APCI(MeOH)-1: 428[M+H]+ |
| 3.010 | 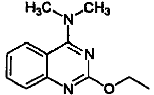 | 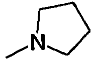 | 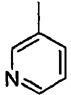 | 2HCl | MS•APCI(MeOH)-1: 428[M+H]+ |
| 3.011 | 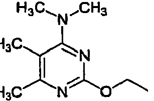 | 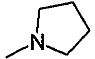 | 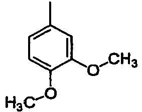 | 2HCl | MS•APCI(MeOH)-2: 465[M+H]+ |
| 3.012 | 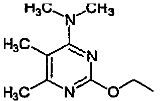 | 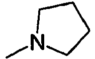 | 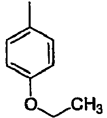 | 2HCl | MS•APCI(MeOH)-1: 449[M+H]+ |
| 3.013 | 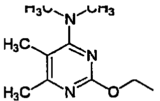 | 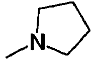 | 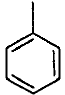 | 2HCl | MS•APCI(MeOH)-1: 405[M+H]+ |
| 3.014 | 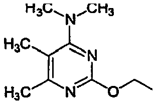 | 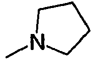 | 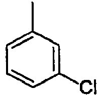 | 2HCl | MS•APCI(MeOH)-1: 439/441 [M+H]+ |
| 3.015 | 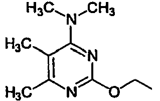 | 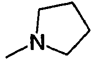 | 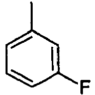 | 2HCl | MS•APCI (10mM-AcONH₄/MeOH)-1: 423[M+H]+ |
| 3.016 | 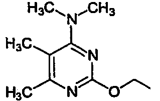 | 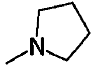 | 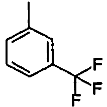 | 2HCl | MS•APCI (10mM-AcONH₄/MeOH)-1: 473[M+H]+ |
| 3.017 | 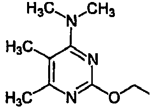 | 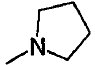 | 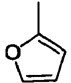 | 2HCl | MS•APCI(MeOH)-1: 395[M+H]+ |
| 3.018 | 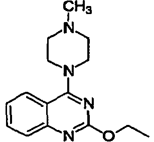 | 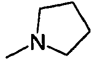 | 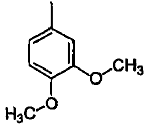 | 3HCl | MS•APCI(MeOH)-2 : 542[M+H]+ |
| 3.019 | 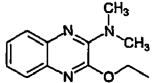 | 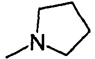 | 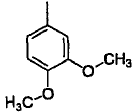 | 2HCl | MS•APCI: (10mM-AcONH₄/MeOH)-1: 487 [M+H] + |
| 3.020 | 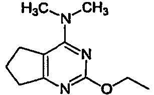 | 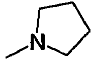 | 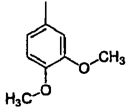 | 2HCl | MS•APCI(MeOH)-1: 477[M+H]+ |
| 3.021 | 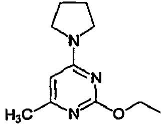 | 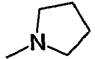 | 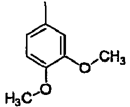 | 2HCl | MS•APCI(MeOH)-1: 477[M+H]+ |
| 3.022 | 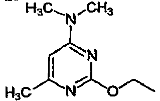 | 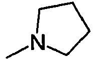 | 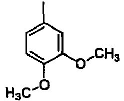 | 2HCl | MS•APCI(MeOH)-2: 451 [M+H]+ |
| 3.023 | 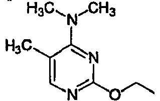 | 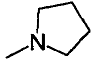 | 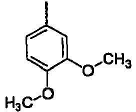 | 2HCl | MS•APCI(MeOH)-2: 451[M+H]+ |
| 3.024 | 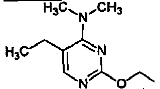 | 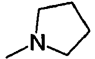 | 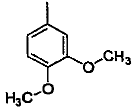 | 2HCl | MS•APCI(MeOH)-1: 465[M+H]+ |
| 3.025 | 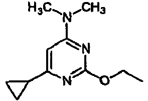 | 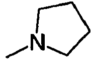 | 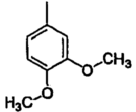 | 2HCl | MS•APCI (10mM-AcONH₄/MeOH)-1: 477[M+H]+ |
| 3.026 | 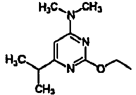 | 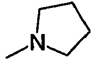 | 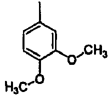 | 2HCl | MS•APCI (10mM-AcONH₄/MeOH)-1: 479[M+H]+ |
| 3.027 | 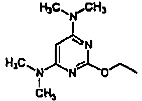 | 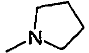 | 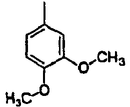 | 2HCl | MS•APCI: (10mM-AcONH₄/MeOH)-1: 480[M+H]+ |
| 3.028 | 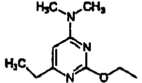 | 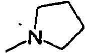 | 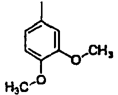 | 2HCl | MS•APCI (10mM-AcONH₄/MeOH)-1: 465[M+H]+ |
| 3.029 | 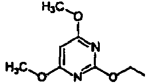 | 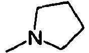 | 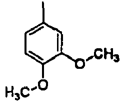 | 2HCl | MS•APCI(MeOH)-2: 454[M+H]+ |
| 3.030 | 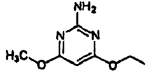 | 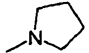 | 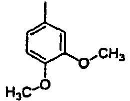 | 2HCl | MS•APCI(MeOH)-2: 439[M+H]+ |
| 3.031 | 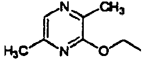 | 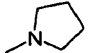 | 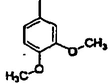 | 2HCl | MS•APCI(MeOH)-1: 422 [M+H] + |

**Table 6**

| | | | | | |
|---|---|---|---|---|---|
| | 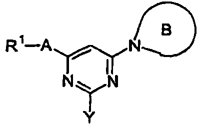 | | | | |

| Example No. | R¹-A- | 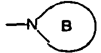 | -Y | Salt | Physical properties, etc. |
|---|---|---|---|---|---|
| 3.032 | 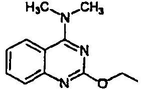 | 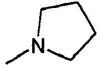 | 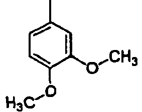 | 2HCl | MS•APCI(MeOH)-1: 487[M+H]+ |
| 3.033 | 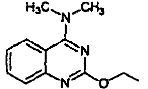 | 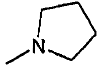 | 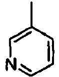 | 2HCl | MS•APCI(MeOH)-1: 428[M+H]+ |
| 3.034 | 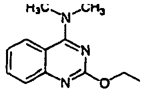 | 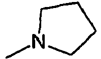 | 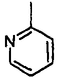 | 3HCl | MS•APCI(MeOH)-1: 428[M+H]+ |
| 3.035 | 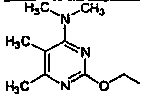 | 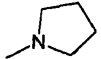 | 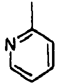 | 3HCl | MS•APCI(MeOH)-1: 406[M+H]+ |

**Table 7**

| | | | | | |
|---|---|---|---|---|---|
| | 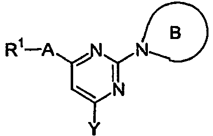 | | | | |

| Example No. | R¹-A- | 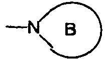 | -Y | Salt | Physical properties, etc. |
|---|---|---|---|---|---|
| 4.001 | 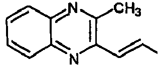 | 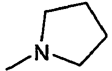 | 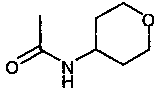 | HCl | MS•APCI: 445 (M+H) |
| 4.002 | 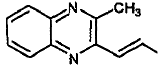 | 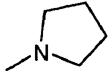 | 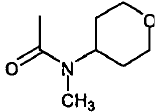 | HCl | MS•APCI: 459 (M+H) |

**Table 8**

| | | | | | |
|---|---|---|---|---|---|
| | 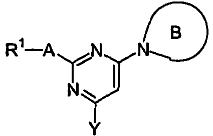 | | | | |

| Example No. | R¹-A- | 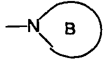 | -Y | Salt | Physical properties, etc. |
|---|---|---|---|---|---|
| 5.001 | 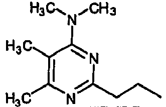 | 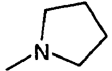 | 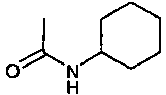 | 2HCl | MS•APCI: 452 (M+H) |
| 5.002 | 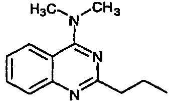 | 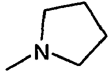 | 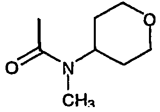 | 2HCl | MS•APCI: 490 (M+H) |
| 5.003 | 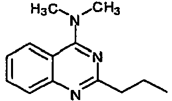 | 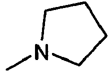 | 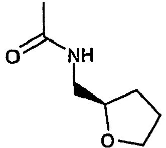 | 2HCl | MS•APCI: 476 (M+H) |
| 5.004 | 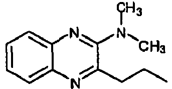 | 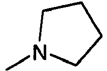 | 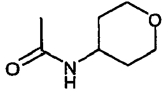 | free form | MS•APCI: 476 (M+H) |
| 5.005 | 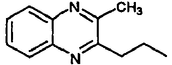 | 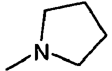 | 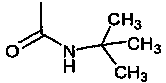 | free form | MS•APCI: 419 (M+H) |
| 5.006 | 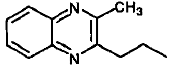 | 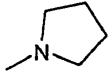 | 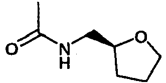 | free form | MS•APCI: 447 (M+H) |
| 5.007 | 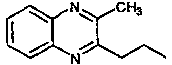 | 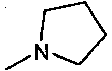 | 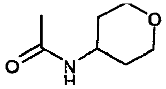 | 2HCl | MS•APCI: 447 (M+H) |
| 5.008 | 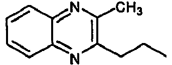 | 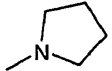 | 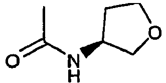 | free form | MS•APCI: 433 (M+H) |
| 5.009 | 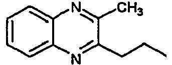 | 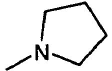 | 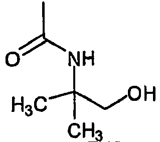 | free form | MS•APCI: 435 (M+H) |
| 5.010 | 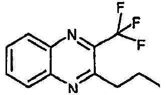 | 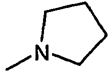 | 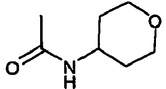 | free form | MS•APCI: 501 (M+H) |
| 5.011 | 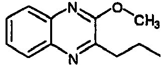 | 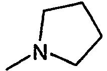 | 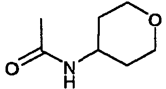 | HCl | MS•APCI: 463 (M+H) |
| 5.012 | 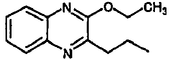 | 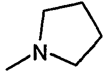 | 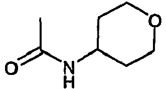 | free form | MS•APCI: 477(M+H) |
| 5.013 | 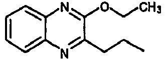 | 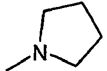 | 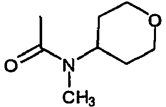 | free form | MS•APCI: 491(M+H) |

**Table of Reference Example**

| Reference Example No. | Structural formula | Salt | Physical properties, etc. |
|---|---|---|---|
| 1 | 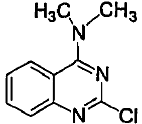 | free form | MS•APCI: 208/210 [M+H]+ |
| 2 | 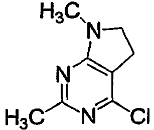 | free form | MS•APCI: 184 [M+H]+ |
| 3 | 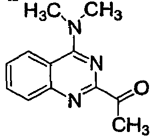 | free form | MS•APCI: 216 [M+H] + |
| 4 | 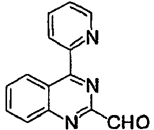 | free form | MS•APCI: 236 [M+H]+ |
| 5 | 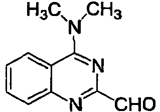 | free form | MS•APCI: 202 [M+H] + |
| 6 | 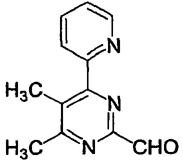 | free form | MS•APCI: 214 [M+H]+ |
| 7 | 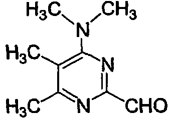 | free form | MS•APCI: 180 [M+H]+ |
| 8 | 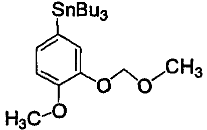 | free form | ¹H-NMR (CDCl₃) δ 7.22 (d, 1H, J=1.3 Hz), 7.06 (dd, 1H, J=7.9, 1.1 Hz), 6.91 (d, 1H, J=7.9 Hz), 5.22 (s, 2H), 3.86 (s, 3H), 3.52 (s, 3H), 1.59-0.86 (m, 27H) |
| 9 | 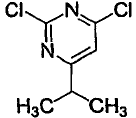 | free form | ¹H-NMR (CDCl₃) δ 7.16 (1H, s), 3.02 (1H, dt, J = 7.0, 7.0 Hz), 1.31 (6H, d, J = 7.0 Hz) |
| 10 | 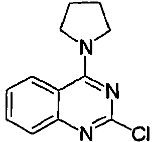 | free form | MS•APCI: 234/236 [M+H]+ |
| 11 | 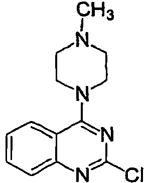 | free form | MS•APCI: 263/265 [M+H]+ |
| 12 | 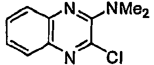 | free form | MS (APCI): 208/210 (M+H). |
| 13 | 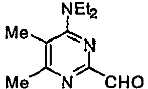 | free form, | MS (APCI): 208 (M+H) |
| 14 | 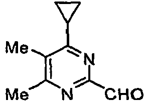 | free form | MS (APCI): m/z 177 (M+H) |
| 15 | 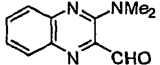 | free form | mp 111-112 °C from hexane-diethyl ether. (APCI): m/z 204 (M+H) |
| 16 | 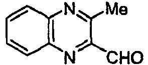 | free form | MS (APCI): m/z 173 (M+H) |
| 17 | 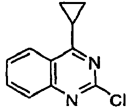 | free form | MS (APCI): m/z 205/207 (M+H) |
| 18 | 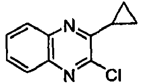 | free form | MS (APCI): m/z 205/207 (M+H) |
| 19 | 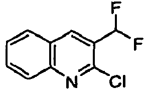 | free form | MS (APCI): m/z 214/216 (M+H) |
| 20 | 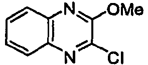 | free form | MS (APCI): m/z 195 (M+H). |
| 21 | 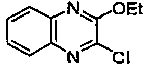 | free form | MS (APCI): m/z 09/211 (M+H) |
| 22 | 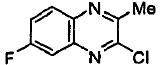 | free form | MS (APCI): m/z 197/199 (M+H). |
| 23 | 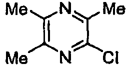 | free form | MS (APCI): m/z 157/159 (M+H). |
| 24 | 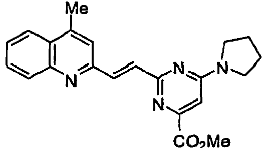 | free form | MS (APCI): m/z 375 (M+H). |
| 25 | 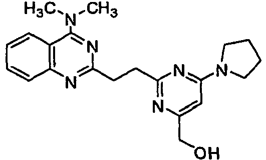 | 2HCl | MS•APCI (10mM-AcONH₄/MeOH)-1 379[M+H]+ |
| 26 | 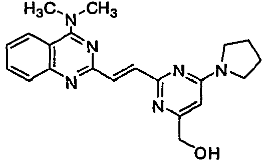 | 2HCl | MS•APCI(MeOH)-1: 377[M+H]+ |

## Claims

1. An aromatic nitrogen-containing 6-membered ring compound represented by general formula [I]: 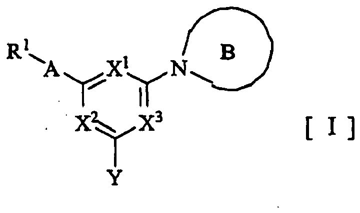 wherein:
X¹, X² and X³ each independently N or CH, and at least two of X¹, X² and X³ are N;
A is *-CH=CH-, *-C(Alk)=CH-, *-CH₂-CH₂-or *-O-CH₂-(* is a bond with R1) ;
Alk is a C₁₋₆ alkyl group;
Ring B is an optionally substituted nitrogen-containing aliphatic heterocyclic group;
R¹ is an optionally substituted nitrogen-containing heterocyclic group, wherein the nitrogen-containing heterocyclic group is selected from the group consisting of quinoxalinyl, quinolyl, isoquinolyl, quinazolinyl, pyrazinyl, pyrimidinyl and the nitrogen-containing heterocyclic group may further be fused with a 5 to 6-membered aliphatic ring;
Y is a group selected from the group consisting of the following (1) to (3):
(1) an optionally substituted phenyl or an optionally substituted aromatic monocyclic 5 to 6-membered heterocyclic group;
(2) an optionally substituted aminocarbonyl;
(3) an optionally substituted amino C₁₋₆ alkyl; or a pharmaceutically acceptable salt thereof.

2. The compound of claim 1, wherein when A is *-CH=CH- or *-C(Alk)=CH-, the double bond in A is E isomeric form.

3. The compound of claim 1, wherein the nitrogen-containing heterocyclic moiety of the optionally substituted nitrogen-containing heterocyclic group represented by R¹ is selected from the group consisting of 2-quinoxalinyl, 2-quinolyl and 2-quinazolinyl.

4. The compound of any one of claims 1 to 3, wherein Y is an optionally substituted aminocarbonyl.

5. The compound of any one of claims 1 to 4, wherein A is *-CH=CH-, *-C(Alk)=CH- or *-CH₂-CH₂-.

6. The compound of any one of claims 1 to 4, wherein A is *-CH=CH-.

7. The compound of any one of claims 1 to 4, wherein X¹ and X² is independently N, X³ is CH, and A is *-CH=CH-.

8. The compound of any one of claims 1 to 4, wherein A is *-O-CH₂-.

9. A compound selected from
2-{(E)-2-[4-(5,6-dihydroimidazo[1,2-a]pyrazin-7(8H)-yl)-6-(3,4-dimethoxyphenyl)pyrimidin-2-yl]vinyl}-N,N-dimethylquinazolin-4-amine;
2-{(E)-2-[4-(dimethylamino)quinazolin-2-yl]vinyl}-N-piperidin-1-yl-6-pyrrolidin-1-ylpyrimidine-4-carboxamide;
2-[(E)-2-(3-methylquinoxalin-2-yl)vinyl]-6-pyrrolidin-1-yl-N-(tetrahydro-2H-pyran-4-yl)pyrimidine-4-carboxamide;
N-cyclopropyl-2-[(E)-2-(3-methylquinoxalin-2-yl)vinyl]-6-pyrrolidin-1-yl-N-(tetrahydro-2H-pyran-4-yl)pyrimidine-4-carboxamide;
2-[(E)-2-(3-methylquinoxalin-2-yl)vinyl]-N-piperidin-1-yl-6-pyrrolidin-1-ylpyrimidine-4-carboxamide;
N-({2-[(E)-2-(3-methylquinoxalin-2-yl)vinyl]-6-pyrrolidin-1-ylpyrimidin-4-yl}methyl)tetrahydro-2H-pyran-4-amine;
2-{(E)-2-[4-(cyclohexyloxy)-6-pyrrolidin-1-ylpyrimidin-2-yl]vinyl}-N,N-dimethylquinazolin-4-amine; acetone
O-(2-{(E)-2-[4-(dimethylamino)quinazolin-2-yl]vinyl}-6-pyrrolidin-1-ylpyrimidin-4-yl)oxime;
(5R)-5-{[(2-{(E)-2-[4-(dimethylamino)quinazolin-2-yl]vinyl}-6-pyrrolidin-1-ylpyrimidin-4-yl)oxy]methyl}pyrrolidin-2-one;
N,N,5,6-tetramethyl-2-{(E)-2-[4-pyrrolidin-1-yl-6-(tetrahydro-2H-pyran-4-yloxy)pyrimidin-2-yl]vinyl}pyrimidin-4-amine; and
6-[(E)-2-(3-methylquinoxalin-2-yl)vinyl]-2-pyrrolidin-1-yl-N-(tetrahydro-2H-pyran-4-yl)pyrimidine-4-carboxamide;
or a pharmaceutically acceptable salt thereof.

10. An aromatic nitrogen-containing 6-membered ring compound represented by formula [I⁰] : 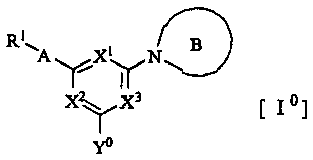 wherein:
X¹, X² and X³ each independently are N or CH, and at least two of X¹, X² and X³ are N;
A is *-CH=CH-, *-C(Alk)=CH-, *-CH₂-CH₂- or *-O-CH₂-(* is a bond with R¹) ;
Alk is a C₁₋₆ alkyl group;
Ring B is an optionally substituted nitrogen-containing aliphatic heterocyclic group;
R¹ is an optionally substituted nitrogen-containing heterocyclic group, wherein the nitrogen-containing heterocyclic group is selected from the group consisting of quinoxalinyl, quinolyl, isoquinolyl, quinazolinyl, pyrazinyl, pyrimidinyl and the nitrogen-containing heterocyclic group may further be fused with a 5 to 6-membered aliphatic ring;
Y⁰ is a group selected from the group consisting of the following (1) to (5):
(1) an optionally substituted phenyl or an optionally substituted aromatic monocyclic 5 to 6-membered heterocyclic group;
(2) an optionally substituted aminocarbonyl;
(3) an optionally substituted amino C₁₋₆ alkyl;
(4) -O-R²
wherein R² is hydrogen, an optionally substituted C₁₋₆ alkyl, C₃₋₈ cycloalkyl, aliphatic monocyclic 5 to 6-membered heterocyclic group, or 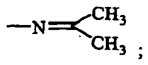
(5) mono- or di-substituted amino;
provided that, when Y⁰ is mono- or di-substituted amino, the nitrogen-containing heterocyclic moiety of R¹ is not quinoxalinyl or quinolyl, or a pharmaceutically acceptable salt thereof for treating or preventing a disease or condition selected from the group consisting of schizophrenia, anxiety disorder, drug addiction, a disease comprising as a symptom a deficiency in cognition, mood disorder and mood episode.

11. The compound of claim 10, wherein the treatment or prevention occurs by inhibition of phosphodiesterase 10 activity.

12. Use of the aromatic nitrogen-containing 6-membered ring compound represented by formula [I⁰] as set forth in claim 10 or a pharmaceutically acceptable salt thereof for the manufacture of a medicament for treating or preventing a disease or condition selected from the group consisting of schizophrenia, anxiety disorder, drug addiction, a disease comprising as a symptom a deficiency in cognition, mood disorder and mood episode.

13. A pharmaceutical composition for treating or preventing a disease or condition selected from the group consisting of schizophrenia, anxiety disorder, drug addiction, a disease comprising as a symptom a deficiency in cognition, mood disorder and mood episode, comprising the aromatic nitrogen-containing 6-membered ring compound represented by formula [I⁰] as set forth in claim 10 or a pharmaceutically acceptable salt thereof as an active ingredient.

## Patentansprüche

1. Aromatische, stickstoffhaltige, 6-gliedrige Ringverbindung, dargestellt durch die allgemeine Formel [I] : 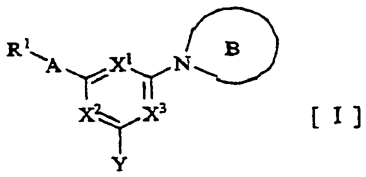 wobei gilt:
X¹, X² und X³ sind jeweils unabhängig voneinander N oder CH, und wenigstens zwei Gruppen von X¹, X² und X³ sind N;
A ist *-CH=CH-, *-CH(Alk)=CH-, *-CH₂-CH₂- oder *-O-CH₂-(* stellt eine Bindung mit R1 dar);
Alk ist eine C₁₋₆-Alkylgruppe;
Ring B ist eine gegebenenfalls substituierte, stickstoffhaltige, aliphatische, heterocyclische Gruppe;
R¹ ist eine gegebenenfalls substituierte, stickstoffhaltige, heterocyclische Gruppe, wobei die stickstoffhaltige, heterocyclische Gruppe ausgewählt ist aus der Gruppe bestehend aus Chinoxalinyl, Chinolyl, Isochinolyl, Chinazolinyl, Pyrazinyl, Pyrimidinyl und die stickstoffhaltige, heterocyclische Gruppe ferner mit einem 5- oder 6-gliedrigen, aliphatischen Ring kondensiert sein kann;
Y ist eine Gruppe ausgewählt aus der Gruppe bestehend aus dem folgenden (1) bis (3):
(1) eine gegebenenfalls substituierte Phenylgruppe oder eine gegebenenfalls substituierte, aromatische, monocyclische, 5- bis 6-gliedrige, heterocyclische Gruppe;
(2) eine gegebenenfalls substituierte Aminocarbonylgruppe;
(3) eine gegebenenfalls substituierte Amino-C₁₋₆-Alkylgruppe; oder eines seiner pharmazeutisch akzeptablen Salze.

2. Verbindung gemäß Anspruch 1, wobei die Doppelbindung in A eine E-isomerische Form besitzt, wenn A *-CH=CH- oder *-C(Alk)=CH- darstellt.

3. Verbindung gemäß Anspruch 1, wobei der stickstoffhaltige, heterocyclische Rest der gegebenenfalls substituierten, stickstoffhaltigen, heterocyclischen Gruppe, dargestellt durch R¹, ausgewählt ist aus der Gruppe bestehend aus 2-Chinoxalinyl, 2-Chinolyl und 2-Chinazolinyl.

4. Verbindung gemäß einem der Ansprüche 1 bis 3, wobei Y eine gegebenenfalls substituierte Aminocarbonylgruppe darstellt.

5. Verbindung gemäß einem der Ansprüche 1 bis 4, wobei A *-CH=CH- oder *-C(Alk)=CH- oder *-CH₂-CH₂- darstellt.

6. Verbindung gemäß einem der Ansprüche 1 bis 4, wobei A *-CH=CH- darstellt.

7. Verbindung gemäß einem der Ansprüche 1 bis 4, wobei X¹ und X² unabhängig voneinander N sind, X³ CH ist und A *-CH=CH- ist.

8. Verbindung gemäß einem der Ansprüche 1 bis 4, wobei A *-O-CH₂- darstellt.

9. Verbindung ausgewählt aus
2-{(E)-2-[4-(5,6-Dihydroimidazo[1,2-a]pyrazin-7(8H)-yl)-6-(3,4-dimethoxyphenyl)pyrimidin-2-yl]vinyl}-N,N-dimethylchinazolin-4-amin;
2-{(E)-2-[4-(Dimethylamino)chinazolin-2-yl]vinyl}-N-piperidin-1-yl-6-pyrrolidin-1-ylpyrimidin-4-carboxamid;
2-[(E)-2-(3-Methylchinoxalin-2-yl)vinyl]-6-pyrrolidin-1-yl-N-(tetrahydro-2H-pyran-4-yl)pyrimidin-4-carboxamid;
N-Cyclopropyl-2-[(E)-2-(3-methylchinoxalin-2-yl)vinyl]-6-pyrrolidin-1-yl-N-(tetrahydro-2H-pyran-4-yl)pyrimidin-4-carboxamid;
2-[(E)-2-(3-Methylchinoxalin-2-yl)vinyl]-N-piperidin-1-yl-6-pyrrolidin-1-ylpyrimidin-4-carboxamid;
N-({2-[(E)-2-(3-Methylchinoxalin-2-yl)vinyl]-6-pyrrolidin-1-ylpyrimidin-4-yl}methyl)tetrahydro-2H-pyran-4-amin;
2-{(E)-2-[4-(Cyclohexyloxy)-6-pyrrolidin-1-ylpyrimidin-2-yl]vinyl}-N,N-dimethylchinazolin-4-amin;
0-(2-{(E)-2-[4-(Dimethylamino)chinazolin-2-yl]vinyl}-6-pyrrolidin-1-ylpyrimidin-4-yl)oxim;
(5R)-5-{[2-{(E)-2-[4-(Dimethylamino)chinazolin-2-yl]vinyl}-6-pyrrolidin-1-ylpyrimidin-4-yl)oxy]methyl}pyrrolidin-2-on;
N,N,5,6-Tetramethyl-2-{(E)-2-[4-(pyrrolidin-1-yl-6-(tetrahydro-2H-pyran-4-yloxy)pyrimidin-2-yl]vinyl}pyrimidin-4-amin; und
6-[(E)-2-(3-Methylchinoxalin-2-yl)vinyl]-2-pyrrolidin-1-yl-N-(tetrahydro-2H-pyran-4-yl)pyrimidin-4-carboxamid;
oder eines seiner pharmazeutisch akzeptablen Salze.

10. Aromatische, stickstoffhaltige, 6-gliedrige Ringverbindung, dargestellt durch Formel [I⁰]: 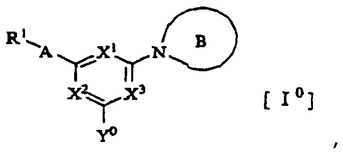 wobei gilt:
X¹, X² und X³ sind jeweils unabhängig voneinander N oder CH, und wenigstens zwei Gruppen von X¹, X² und X³ sind N;
A ist *-CH=CH-, *-CH(Alk)=CH-, *-CH₂-CH₂- oder *-O-CH₂-(* stellt eine Bindung mit R1 dar);
Alk ist eine C₁₋₆-Alkylgruppe;
Ring B ist eine gegebenenfalls substituierte, stickstoffhaltige, aliphatische, heterocyclische Gruppe;
R¹ ist eine gegebenenfalls substituierte, stickstoffhaltige, heterocyclische Gruppe, wobei die stickstoffhaltige, heterocyclische Gruppe ausgewählt ist aus der Gruppe bestehend aus Chinoxalinyl, Chinolyl, Isochinolyl, Chinazolinyl, Pyrazinyl, Pyrimidinyl und die stickstoffhaltige, heterocyclische Gruppe ferner mit einem 5- oder 6-gliedrigen aliphatischen Ring kondensiert sein kann;
Y⁰ ist eine Gruppe ausgewählt aus der Gruppe bestehend aus dem folgenden (1) bis (5):
(1) eine gegebenenfalls substituierte Phenylgruppe oder eine gegebenenfalls substituierte, aromatische, monocyclische, 5- bis 6-gliedrige, heterocyclische Gruppe;
(2) eine gegebenenfalls substituierte Aminocarbonylgruppe;
(3) eine gegebenenfalls substituierte Amino-C₁₋₆-Alkylgruppe;
(4) -O-R², wobei R² Wasserstoff, eine gegebenenfalls substituierte C₁₋₆-Alkylgruppe, eine gegebenenfalls substituierte C₃₋₈-Cycloalkylgruppe, eine gegebenenfalls substituierte, aliphatische, monocyclische, 5- bis 6-gliedrige, heterocyclische Gruppe oder 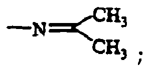 darstellt;
(5) mono- oder di-substituierte Aminogruppe;
mit der Maßgabe, dass, wenn Y⁰ eine mono- oder di-substituierte Aminogruppe ist, der stickstoffhaltige, heterocyclische Rest von R¹ nicht Chinoxalinyl oder Chinolyl ist, oder eines seiner pharmazeutisch akzeptablen Salze zur Behandlung oder Prävention einer Erkrankung oder eines Zustandes ausgewählt aus der Gruppe bestehend aus Schizophrenie, Angststörung, Drogenabhängigkeit, einer Erkrankung, umfassend als ein Symptom einen Mangel an Wahrnehmung, einer Gemütserkrankung und einer Stimmungsepisode.

11. Verbindung gemäß Anspruch 10, wobei die Behandlung oder Prävention durch Inhibition der Phosphodiesterase 10-Aktivität erfolgt.

12. Anwendung der aromatischen, stickstoffhaltigen, 6-gliedrigen Ringverbindung, dargestellt durch Formel [I⁰], wie in Anspruch 10 angeführt, oder eines seiner pharmazeutisch akzeptablen Salze zur Herstellung eines Medikamentes zur Behandlung oder Prävention einer Erkrankung oder eines Zustandes ausgewählt aus der Gruppe bestehend aus Schizophrenie, Angststörung, Drogenabhängigkeit, einer Erkrankung, umfassend als ein Symptom einen Mangel an Wahrnehmung, einer Gemütserkrankung und einer Stimmungsepisode.

13. Pharmazeutische Zusammensetzung zur Behandlung oder Prävention einer Erkrankung oder eines Zustandes ausgewählt aus der Gruppe bestehend aus Schizophrenie, Angststörung, Drogenabhängigkeit, einer Erkrankung, umfassend als ein Symptom einen Mangel an Wahrnehmung, einer Gemütserkrankung und einer Stimmungsepisode, umfassend die aromatische, stickstoffhaltige, 6-gliedrige Ringverbindung, dargestellt durch Formel [I⁰], wie in Anspruch 10 angeführt, oder eines seiner pharmazeutisch akzeptablen Salze als einen Wirkstoff.

## Revendications

1. Composé aromatique cyclique à 6 éléments contenant de l'azote représenté par la formule générale (I) : 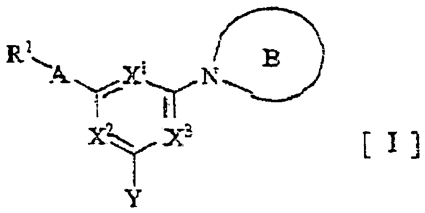 dans laquelle :
X¹, X² et X³ sont chacun indépendamment N ou CH, et au moins deux parmi X¹, X² et X³ sont N ;
A est *-CH=CH-, *-C(Alk)=CH-, *-CH₂-CH₂- ou *-O-CH₂- (* est une liaison avec R¹) ;
Alk est un groupe alkyle en C₁-C₆ ;
le cycle B est un groupe hétérocyclique aliphatique contenant de l'azote facultativement substitué ;
R¹ est un groupe hétérocyclique contenant de l'azote facultativement substitué, dans lequel le groupe hétérocyclique contenant de l'azote est choisi parmi le groupe consistant en un quinoxalinyle, un quinolyle, un isoquinolyle, un quinazolinyle, un pyrazinyle, un pyrimidinyle et le groupe hétérocyclique contenant de l'azote peut en outre être fusionné avec un cycle aliphatique à 5 à 6 éléments ;
Y est un groupe choisi parmi le groupe consistant en les (1) à (3) suivants :
(1) un phényle facultativement substitué ou un groupe hétérocyclique monocyclique aromatique à 5 à 6 éléments facultativement substitué ;
(2) un aminocarbonyle facultativement substitué ;
(3) un aminoalkyle en C₁-C₆ facultativement substitué ; ou
sel pharmaceutiquement acceptable de celui-ci.

2. Composé selon la revendication 1, dans lequel A est *-CH=CH- ou *-C(Alk)=CH-, la double liaison dans A est une forme isomérique E.

3. Composé selon la revendication 1, dans lequel le groupe caractéristique hétérocyclique contenant de l'azote du groupe hétérocyclique contenant de l'azote facultativement substitué représenté par R¹ est choisi parmi le groupe consistant en le 2-quinoxalinyle, le 2-quinolyle et le 2-quinazolinyle.

4. Composé selon l'une quelconque des revendications 1 à 3, dans lequel Y est un aminocarbonyle facultativement substitué.

5. Composé selon l'une quelconque des revendications 1 à 4, dans lequel A est *-CH=CH-, *-C(Alk)=CH- ou *-CH₂-CH₂-.

6. Composé selon l'une quelconque des revendications 1 à 4, dans lequel A est *-CH=CH-.

7. Composé selon l'une quelconque des revendications 1 à 4, dans lequel X¹ et X² sont indépendamment N, X³ est CH, et A est *-CH=CH-.

8. Composé selon l'une quelconque des revendications 1 à 4, dans lequel A est *-O-CH₂-.

9. Composé choisi parmi
la 2-{(E)-2-[4-(5,6-dihydroimidazo[l,2-a]pyrazin-7(8H)-yl)-6-(3,4-diméthoxyphényl)pyrimidin-2-yl]vinyl}-N,N-diméthylquinazolin-4-amine ;
le 2-{(E)-2-[4-(diméthylamino)quinazolin-2-yl]vinyl}-N-pipéridin-1-yl-6-pyrrolidin-1-ylpyrimidine-4-carboxamide ;
le 2-[(E)-2-(3-méthylquinoxalin-2-yl)vinyl]-6-pyrrolidin-1-yl-N-(tétrahydro-2H-pyran-4-yl)pyrimidine-4-carboxamide ;
le N-cyclopropyl-2-[(E)-2-(3-méthylquinoxalin-2-yl)vinyl]-6-pyrrolidin-1-yl-N-(tétrahydro-2H-pyran-4-yl)pyrimidine-4-carboxamide ;
le 2-[(E)-2-(3-méthylquinoxalin-2-yl)vinyl]-N-pipéridin-1-yl-6-pyrrolidin-1-ylpyrimidine-4-carboxamide ;
la N-({2-[(E)-2-(3-méthylquinoxalin-2-yl)vinyl]-6-pyrrolidin-1-ylpyrimidin-4-yl}méthyl)tétrahydro-2H-pyran-4-amine ;
la 2-{(E)-2-[4-(cyclohexyloxy)-6-pyrrolidin-1-ylpyrimidin-2-yl]vinyl}-N,N-diméthylquinazolin-4-amine ; acétone
la O-(2-{(E)-2-[4-(diméthylamino)quinazolin-2-yllvinyl}-6-pyrrolidin-1-ylpyrimidin-4-yl)oxime ;
la (5R)-5-{[(2-{(E)-2-[4-(diméthylamino)quinazolin-2-yl]vinyl}-6-pyrrolidin-1-ylpyrimidin-4-yl)oxy]méthyl}pyrrolidin-2-one ;
la N,N,5,6-tétrarnéthyl-2-{(E)-2-[4-pyrrolidin-1-yl-6-(tétrahydro-2H-pyran-4-yloxy)pyrimidin-2-yl]vinyl}pyrimidin-4-amine ; et
le 6-[(E)-2-(3-méthylquinoxalin-2-yl)vinyl]-2-pyrrolidin-1-yl-N-(tétrahydro-2H-pyran-4-yl)pyrimidine-4-carboxamide ;
ou sel pharmaceutiquement acceptable de ceux-ci.

10. Composé aromatique cyclique à 6 éléments contenant de l'azote représenté par la formule générale (I⁰) : 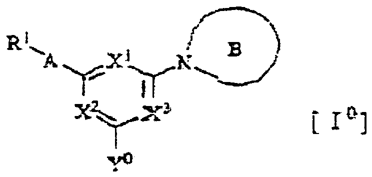 dans laquelle :
X¹, X² et X³ sont chacun indépendamment N ou CH, et au moins deux parmi X¹, X² et X³ sont N ;
A est *-CH=CH-, *-C(Alk)=CH-, *-CH₂-CH₂- ou *-O-CH₂- (* est une liaison avec R¹) ;
Alk est un groupe alkyle en C₁-C₆ ;
le cycle B est un groupe hétérocyclique aliphatique contenant de l'azote facultativement substitué ;
R¹ est un groupe hétérocyclique contenant de l'azote facultativement substitué, dans lequel le groupe hétérocyclique contenant de l'azote est choisi parmi le groupe consistant en un quinoxalinyle, un quinolyle, un isoquinolyle, un quinazolinyle, un pyrazinyle, un pyrimidinyle et le groupe hétérocyclique contenant de l'azote peut en outre être fusionné avec un cycle aliphatique à 5 à 6 éléments ;
Y⁰ est un groupe choisi parmi le groupe consistant en les (1) à (5) suivantes :
(1) un phényle facultativement substitué ou un groupe hétérocyclique monocyclique aromatique à 5 à 6 éléments facultativement substitué ;
(2) un aminocarbonyle facultativement substitué ;
(3) un aminoalkyle en C₁-C₆ facultativement substitué ;
(4) -O-R²
dans laquelle R² est un hydrogène, un alkyle en C₁-C₆, un cycloalkyle en C₃-C₈, un groupe hétérocyclique monocyclique aliphatique à 5 à 6 éléments facultativement substitué, ou 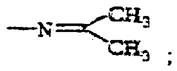
(5) un amino mono- ou di-substitué ;
sous réserve que, lorsque Y⁰ est un amino mono- ou di-substitué, le groupe caractéristique hétérocyclique contenant de l'azote de R¹ n'est pas un quinoxalinyle ni un quinolyle, ou sel pharmaceutiquement acceptable de celui-ci pour traiter ou prévenir une maladie ou un état choisi(e) parmi le groupe consistant en une schizophrénie, un trouble anxieux, une pharmacodépendance, une maladie comprenant comme un symptôme un déficit cognitif, un trouble de l'humeur et un épisode d'humeur.

11. Composé selon la revendication 10, dans lequel le traitement ou la prévention fonctionne par inhibition de l'activité phosphodiestérase de type 10.

12. Utilisation du composé aromatique cyclique à 6 éléments contenant de l'azote représenté par la formule (I⁰) selon la revendication 10 ou d'un sel pharmaceutiquement acceptable de celui-ci pour la fabrication d'un médicament pour traiter ou prévenir une maladie ou un état choisi(e) parmi le groupe consistant en une schizophrénie, un trouble anxieux, une pharmacodépendance, une maladie comprenant comme un symptôme un déficit cognitif, un trouble de l'humeur et un épisode d'humeur.

13. Composition pharmaceutique pour traiter ou prévenir une maladie ou un état choisi(e) parmi le groupe consistant en une schizophrénie, un trouble anxieux, une pharmacodépendance, une maladie comprenant comme un symptôme un déficit cognitif, un trouble de l'humeur et un épisode d'humeur, comprenant le composé aromatique cyclique à 6 éléments contenant de l'azote représenté par la formule (I⁰) selon la revendication 10 ou un sel pharmaceutiquement acceptable de celui-ci comme un ingrédient actif.
